# EUROPEAN PATENT APPLICATION

(11) **EP 2 199 283 A1**
(43) Date of publication of application: **23.06.2010**
(21) Application number: 08834303.3
(22) Date of filing: 29.09.2008
(51) Int. Cl.: C07D 215/22, A61K 31/47, A61K 31/4706, A61K 31/4709, A61K 31/496, A61K 31/5377, A61P 7/06, A61P 43/00, C07D 215/44, C07D 215/48, C07D 413/04

(54) **PROPHYLACTIC AND/OR THERAPEUTIC AGENT FOR ANEMIA, COMPRISING TETRAHYDROQUINOLINE COMPOUND AS ACTIVE INGREDIENT**

(30) Priority: 27.09.2007 JP 2007252727
(71) Applicant: Kowa Company, Ltd., Nagoya-shi, Aichi 460-8625 (JP)
(72) Inventor: ASHIKAWA, Masanori, Higashimurayama-shi Tokyo 189-0022 (JP); TAGASHIRA, Junya, Higashimurayama-shi Tokyo 189-0022 (JP); KOKETSU, Akiyasu, Higashimurayama-shi Tokyo 189-0022 (JP); MORIMOTO, Toshiharu, Higashimurayama-shi Tokyo 189-0022 (JP); NISHIYAMA, Tatsuaki, Higashimurayama-shi Tokyo 189-0022 (JP); GODA, Satoshi, Higashimurayama-shi Tokyo 189-0022 (JP); YAMABI, Masaki, Higashimurayama-shi Tokyo 189-0022 (JP); DOI, Takeshi, Higashimurayama-shi Tokyo 189-0022 (JP); ISHIWATA, Hiroyuki, Higashimurayama-shi Tokyo 189-0022 (JP)
(74) Representative: Albrecht, Thomas
(86) International application number: PCT/JP2008/002712
(87) International publication number: WO 2009/041072

(57) **Abstract**

Disclosed is a low-molecular-weight compound having an EPO production-promoting activity and/or a hemoglobin expression-enhancing activity. Specifically disclosed is an EPO production promoter and/or a hemoglobin expression enhancer comprising a 1-acyl-4-(phenoxy, benzyloxy or phenylamino)-1,2,3,4-tetrahydroquinoline derivative, more specifically a tetrahydroquinoline compound represented by the general formula (1); 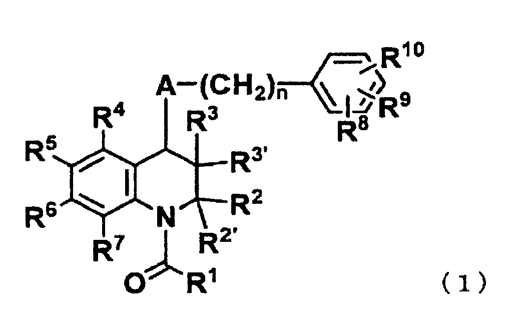 [wherein R¹, R², R^{2'}, R³ and R^{3'} independently represent a hydrogen atom, a C₁₋₆ alkyl group, or the like; R⁴, R⁵, R⁶, R⁷, R⁸, R⁹ and R¹⁰ independently represent a hydrogen atom, a halogen atom, a C₁₋₆ alkyl group, or the like; A represents N-R¹¹ or an oxygen atom; R¹¹ represents a hydrogen atom, a C₁₋₆ alkyl group, or the like; and n represents an integer of 0 or 1], a salt of the tetrahydroquinoline compound, or a solvate of the tetrahydroquinoline compound or the salt.

## Description

### Technical Field

The present invention relates to novel erythropoietin production accelerators and/or hemoglobin expression potentiators. More specifically, the present invention relates to a preventive and/or therapeutic agent for disorders caused by reduced production of erythropoietin, such as anemia.

### Background Art

Erythropoietin (EPO) is a glycoprotein hormone, which participates in maturation and differentiation of an erythroid progenitor cell to a matured red blood cell. It is a 165-amino-acid polypeptide found in nature in the form of a monomer (Non-Patent Document 1).
Human erythropoietin plays an essential role in proliferation and differentiation of red blood cells. Therefore, the hormone is useful for treatment of blood disorders which are characterized by reduced production of red blood cells. Clinically, EPO is used in treatment of anemia associated with chronic renal failure (CRF), anemia associated with autologous blood transfusion or prematurity (Non-Patent Documents 2 to 5), or treatment of an AIDS patient or a cancer patient under chemotherapy (Non-Patent Document 6). Further, EPO has been found to be effective in treatment of chronic anemia.
In adults, EPO is mainly produced by kidney, but also produced by astrocytes and neurons of a central nervous system. EPO and EPO receptor are expressed in capillary blood vessels present in border region between brain and peripheral system. Further, it has been reported that the systemically administered EPO passes blood-brain barrier and reduces loss of neuron cells, responding to ischemia and mechanical trauma of brain and spinal cord , epilepsy, excitotoxin and neuronal inflammation (Non-Patent Documents 7 to 11).
Meanwhile, a therapy using a protein such as EPO is problematic in that the protein has a short plasma half-life, as being susceptible to degradation by protease (Non-Patent Documents 12 and 13), and therefore intravenous injection must be performed several times in order to maintain the effective therapeutic blood level of the protein in circulation. In this regard, subcutaneous injection may be an alternative administration route. However, when administered subcutaneously, the agent is absorbed slowly from the administration site. Thus, plasma level of the protein is significantly low as compared with the case of intravenous administration, although effect of sustained release may be appreciable. Therefore, in order to obtain a therapeutic effect of similar level, subcutaneous injection must be performed several times as in the case of intravenous administration, and this can be a burden to a patient. Further, since human plasma EPO is a broadly and diversely glycosylated protein which is not reproduced in any recombinant human EPO, there is also a problem like heterogeneous size, etc.
Thus, for the treatment of disorders caused by reduced production of erythropoietin including anemia as described above, instead of EPO having poor bioavailability, demand exists for a method and a compound for potentiating intrinsic EPO in the corresponding technology field.

Meanwhile, it has been known that expression amount of EPO is controlled by oxygen concentration via hypoxia inducible factor (HIF), which is a transcription factor. Specifically, in normoxia, the HIF subunit (HIF-1α) in which the proline residue is hydroxylated by 2-oxoglutarate dioxygenase enzyme is degraded by ubiquitin-proteasome system, and EPO production is not potentiated. However, in hypoxia, hydroxylation of the proline residue in HIF-1α by 2-oxoglutarate dioxygenase enzyme is inhibited so that the stabilized HIF-1α translocates from the cytoplasm to the nucleus to form a dimer with HIF-1β. As a result, it binds to hypoxia responsible element (HRE) sequence of EPO gene to promote the transcription and to potentiate the production of EPO (Non-Patent Document 14).
Based on this mechanism of producing EPO, an enzyme inhibitor for HIF prolylhydroxylase containing 2-oxoglutarate dioxygenase enzyme, etc. has been suggested as an agent for potentiating EPO production (Patent Documents 1 to 4).

However, genes of which expression is controlled by HIF are not only EPO but also vascular endothelial growth factor (VEGF) and the like. It has been reported that VEGF has an activity of promoting angiogenesis, and based on this activity, it may aggravate malignant tumor (Non-Patent Documents 15 and 16). Further, anemia can also be caused by chemotherapy for cancer, and since there might be a case in which a therapeutic agent for anemia is administered to a cancer patient undergoing chemotherapy (Non-Patent Document 6), a compound having an activity of inhibiting HIF prolylhydroxylase, which may also potentiate the expression of VEGF and the like that aggravate cancer, also bears risk as described above.

It is reported that the expression of EPO is controlled by a promoter and an enhancer, which locate at 5' side and 3' side of the EPO gene, respectively, and HIF binds to HRE sequence in the enhancer to potentiate the expression of EPO. In addition to HIF, GATA-2, NFκB and the like are also known to have an activity of controlling EPO production (Non-Patent Documents 17 and 18). It is further believed that, according to the mechanism other than the inhibition of HIF prolylhydroxylase enzyme activity, potentiation of EPO production can be achieved. For such reasons, it is believed that a compound having an activity of potentiating EPO production without depending on inhibition of HIF prolylhydroxylase enzyme activity will be useful for the treatment of anemia.
Further, as described in detail in the above, EPO can promote proliferation and maturation of erythroid precursor cells. However, a compound which has an activity of promoting maturation and differentiation of erythroid precursor cells without being involved with EPO production is also useful as a therapeutic agent for anemia. A compound having an activity of potentiating hemocyte proliferation acceleration, which is a property of EPO, or a compound having an inhibitory effect on the phosphatase of hematopoietic cell, which catalyzes dephosphorylation as one of the important control mechanisms in EPO induced signal transduction , has been reported (Patent Documents 5 to 7). However, it cannot be said that their activity is fully satisfying. Further, although a synthetic peptide and Hematide which act on EPO receptor have been reported (Non-Patent Document 19), they are problematic in that high dose administration is required for obtaining the activity similar to EPO and they cannot be orally administered.
Therefore, a low molecular weight therapeutic agent for anemia, which has both activities of promoting EPO production and promoting hemoglobin production and can be orally administered, is considered to be useful for future therapeutics for anemia.

Meanwhile, as for a pharmaceutical agent having the tetrahydroquinoline skeleton related to the present invention, CRTH2 inhibitor that is useful for inflammatory diseases (Patent Documents 8 to 11), an inhibitor for eosinophil infiltration that is useful for inflammatory diseases (Patent Document 12), a potentiator for expression of ecdysone steroid hormone receptor involved with growth, molt and development of an insect (Patent Document 13), an agent of promoting secretion of β-amyloid precursor protein that is effective for neurodegenerative disorders like Alzheimer's disease, Parkinson's disease and the like (Patent Document 14), an inhibitor for activation of STAT6 that is effective for atopic dermatitis, etc. (Patent Document 15), and an agent of promoting production of Apolipo protein A-I that is effective for hyperlipidemia (Patent Document 16) are disclosed. However, there is no description or suggestion regarding the compound's activity of promoting EPO production or potentiating moglobin expression. Further, a compound which has the tetrahydroquinoline skeleton and is useful as a preventive and/or therapeutic agent for anemia has never been known.

Patent Document 1: Japanese Patent Application Laid-Open (JP-A) No. 2006-137763
Patent Document 2: WO2003/53997
Patent Document 3: W02005/11696
Patent Document 4: WO2007/38571
Patent Document 5: Japanese Patent Application National Publication (Laid-Open) No. 2000-536365
Patent Document 6: Japanese Patent Application Laid-Open (JP-A) No. 11-171774
Patent Document 7: Japanese Patent Application Laid-Open (JP-A) No. 2002-275159
Patent Document 8: WO2004/32848
Patent Document 9: WO2004/35543
Patent Document 10: WO2005/7094
Patent Document 11: WO2005/100321
Patent Document 12: WO2004/52863
Patent Document 13: WO2003/105849
Patent Document 14: WO2001/76629
Patent Document 15: WO2002/79165
Patent Document 16: Japanese Patent Application Laid-Open (JP-A) No. 2002-53557
Non-Patent Document 1: Lin F-K et al., Proc. Natl. Acad. Sci. USA, 82: 7580-7584 (1985)
Non-Patent Document 2: Eschbach JW, Egrie JC, Downing MR et al., NEJM, 316: 73-78(1987)
Non-Patent Document 3: Eschbach JW, Abdulhadi MH, Browne JK et al., Ann. Intern. Med., 111: 992 (1989)
Non-Patent Document 4: Egrie JC, Eschbach JW, McGuire T, Adamson JW, Kidney Intl., 33: 262 (1988)
Non-Patent Document 5: Lim VS, Degowin RL, Zavala D et al., Ann. Intern. Med., 110: 108-114 (1989)
Non-Patent Document 6: Danna RP, Rudnick SA, Abels RI, Gamick MB, Erythropoietin in Clinical Applications-An International Perspective, New York: Marcel Dekker; p301-324 (1990)
Non-Patent Document 7: Sakanaka et al, Proc. Natl. Acad. Sci. USA., 95, 4635-4640 (1998)
Non-Patent Document 8: Celik et al, Proc. Natl. Acad. Sci. USA., 99, 2258-2263 (2002)
Non-Patent Document 9: Brines et al, Proc. Natl. Acad. Sci. USA., 97, 10526-10531 (2000)
Non-Patent Document 10: Calapai et al, Eur. J. Pharmacol., 401,349-356 (2000)
Non-Patent Document 11: Siren et al, Proc. Natl. Acad. Sci. USA., 98, 4044-404 (2001)
Non-Patent Document 12: Spivack JL and Hogans BB, Blood, 73: 90 (1989)
Non-Patent Document 13: McMahon FG et al., Blood, 76: 1718 (1990)
Non-Patent Document 14: Wolfgang Jelkman, Internal Medicine 43, 649-659 (2004)
Non-Patent Document 15: Maxwell PH et al, Proc. Natl. Acad. Sci. USA. 94, 15, 8104-9 (1997)
Non-Patent Document 16: Cancer Res., 61,15, Fang J et al, 5731-5 (2001)
Non-Patent Document 17: Imagawa S et al, Blood 89, 1430-1439 (1997)
Non-Patent Document 18: La Ferla K et al, FASEB J, 16, 1811-1813 (2002)
Non-Patent Document 19: Stead RB, et al., Blood 108: 1830-1834 (2006)

### Disclosure of the Invention

### Problems to be Solved by the Invention

An object of the present invention is to provide a low molecular weight compound having an activity of promoting EPO production and/or an activity of potentiating hemoglobin expression. More specifically, it is to provide a pharmaceutical agent useful for the prophylaxis and/or treatment of anemia.

### Means for Solving the Problems

Under the circumstances described above, the present inventors extensively studied to find out a compound which has an activity of promoting EPO production or an activity of potentiating hemoglobin expression. As a result, it was found that 1,2,3,4-tetrahydroquinoline derivatives, preferably 1-acyl-4-(phenoxy, benzyloxy, or phenylamino)-1,2,3,4-tetrahydroquinoline derivatives, more specifically the tetrahydroquinoline compound represented by the following Formula (1) can promote the EPO production in a test using HepG2 cells derived from human liver cancer and can potentiate the hemoglobin expression in a test using human proerythroblast K562 cells, and therefore the present invention was completed.
To be specific, the present invention relates to an agent for promoting EPO production comprising as an effective component 1-acyl-4-(phenoxy, benzyloxy or phenylamino)-1,2,3,4-tetrahydroquinoline derivatives, a salt of the tetrahydroquinoline derivatives, or a solvate of the tetrahydroquinoline derivatives or the salt.
More specifically, the present invention is to provide an EPO production accelerator comprising as an effective component the tetrahydroquinoline compound represented by the following Formula (1):

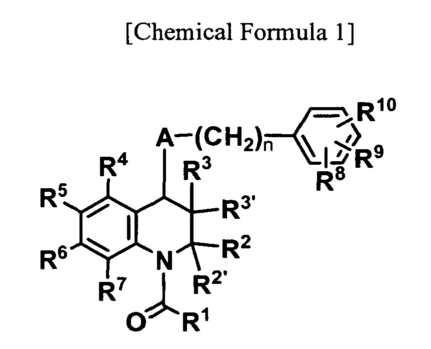

[in the formula, R¹, R², R^{2'}, R³ and R^{3'} independently represent a hydrogen atom, a C₁₋₆ alkyl group, a C₂₋₆ alkenyl group, a C₂₋₆ alkynyl group, a C₁₋₆ alkylamino group, a di C₁₋₆ alkylamino group or a C₃₋₆ cycloalkyl group,
R⁴, R⁵, R⁶, R⁷, R⁸, R⁹ and R¹⁰ independently represent a hydrogen atom, a halogen atom, a C₁₋₆ alkyl group which may be substituted, a C₂₋₆ alkenyl group which may be substituted, a C₂₋₆ alkynyl group which may be substituted, a C₃₋₆ cycloalkyl group which may be substituted, a C₆₋₁₄ aryl group which may be substituted, a 5- to 10-membered heterocyclic group which may be substituted group, a C₁₋₆ alkoxy group which may be substituted, a C₆₋₁₄ aryloxy group which may be substituted, an amino group, a C₁₋₆ alkylcarbonylamino group, a C₁₋₆ alkoxycarbonylamino group, a C₁₋₆ alkylamino group which may be substituted, a di C₁₋₆ alkylamino group which may have a substituent group, a C₆₋₁₄ arylamino group which may be substituted, a C₁₋₆ alkylthio group which may be substituted, a C₁₋₆ alkylsulfonyl group which may be substituted, a C₁₋₆ alkylsulfonamide group which may be substituted, a C₁₋₆ alkylsulfinyl group which may be substituted, a C₁₋₆ alkoxycarbonyl group which may be substituted, a C₁₋₆ alkanoyl group which may be substituted, a 5- to 7-membered saturated heterocyclic carbonyl group which may be substituted, a hydroxy group, a nitro group, a carboxy group, a carbamoyl group which may be substituted or a cyano group, wherein R⁹ and R₁₀ together may form a carbon ring or a heterocycle,
A represents N-R¹¹ or an oxygen atom, wherein R¹¹ represents a hydrogen atom or a C₁₋₆ alkyl group which may be substituted, and
n represents an integer of 0 or 1.],
a salt of the tetrahydroquinoline derivatives, or a solvate of the tetrahydroquinoline derivatives or the salt.

Further, the present invention is to provide a hemoglobin expression potentiator which comprises as an effective component the 1-acyl-4-(phenoxy, benzyloxy, or phenylamino)-1,2,3,4-tetrahydroquinoline derivatives, more specifically the tetrahydroquinoline compound represented by the Formula (1) above, a salt of the tetrahydroquinoline derivatives, or a solvate of the tetrahydroquinoline derivatives or the salt.
Further, the present invention is to provide an agent for the prophylaxis and/or treatment of anemia which comprises as an effective component the 1-acyl-4-(phenoxy, benzyloxy, or phenylamino)-1,2,3,4-tetrahydroquinoline derivatives, more specifically the tetrahydroquinoline compound represented by the Formula (1) above, a salt of the tetrahydroquinoline derivatives, or a solvate of the tetrahydroquinoline derivatives or the salt.

Further, the present invention relates to the use of the 1-acyl-4-(phenoxy, benzyloxy, or phenylamino)-1,2,3,4-tetrahydroquinoline derivatives, more specifically the tetrahydroquinoline compound represented by the Formula (1) above, a salt of the tetrahydroquinoline derivatives, or a solvate of the tetrahydroquinoline derivatives or the salt for the production of a preparation which is used for promoting EPO production.
Further, the present invention relates to the use of the 1-acyl-4-(phenoxy, benzyloxy, or phenylamino)-1,2,3,4-tetrahydroquinoline derivatives, more specifically the tetrahydroquinoline compound represented by the Formula (1) above, a salt of the tetrahydroquinoline derivatives, or a solvate of the tetrahydroquinoline derivatives or the salt for the production of a preparation which is used for potentiating hemoglobin expression.
Further, the present invention relates to the use of the 1-acyl-4-(phenoxy, benzyloxy, or phenylamino)-1,2,3,4-tetrahydroquinoline derivatives, more specifically the tetrahydroquinoline compound represented by the Formula (1) above, a salt of the tetrahydroquinoline derivatives, or a solvate of the tetrahydroquinoline derivatives or the salt for the production of a preparation which is used for the prophylaxis and/or treatment of anemia.

Further, the present invention relates to a method of promoting EPO production, which is **characterized in that** an effective amount of the 1-acyl-4-(phenoxy, benzyloxy, or phenylamino)-1,2,3,4-tetrahydroquinoline derivatives, more specifically the tetrahydroquinoline compound represented by the Formula (1) above, a salt of the tetrahydroquinoline derivatives, or a solvate of the tetrahydroquinoline derivatives or the salt is administered to a subject who is in need of promoted EPO production.
Further, the present invention relates to a method of potentiating hemoglobin expression, which is **characterized in that** an effective amount of the 1-acyl-4-(phenoxy, benzyloxy, or phenylamino)-1,2,3,4-tetrahydroquinoline derivatives, more specifically the tetrahydroquinoline compound represented by the Formula (1) above, a salt of the tetrahydroquinoline derivatives, or a solvate of the tetrahydroquinoline derivatives or the salt is administered to a subject who is in need of potentiated hemoglobin expression.
Further, the present invention relates to a method for the prophylaxis and/or treatment of anemia, which is **characterized in that** an effective amount of the 1-acyl-4-(phenoxy, benzyloxy, or phenylamino)-1,2,3,4-tetrahydroquinoline derivatives, more specifically the tetrahydroquinoline compound represented by the Formula (1) above, a salt of the tetrahydroquinoline derivatives, or a solvate of the tetrahydroquinoline derivatives or the salt is administered to a subject who suffers from anemia.

Further, the present invention relates to a method of promoting EPO production in cells wherein an effective amount of the 1-acyl-4-(phenoxy, benzyloxy, or phenylamino)-1,2,3,4-tetrahydroquinoline derivatives, more specifically the tetrahydroquinoline compound represented by the Formula (1) above, a salt of the tetrahydroquinoline derivatives, or a solvate of the tetrahydroquinoline derivatives or the salt is brought into contact with the cells.

Further, the present invention relates to a pharmaceutical composition for promoting EPO production comprising the 1-acyl-4-(phenoxy, benzyloxy, or phenylamino)-1,2,3,4-tetrahydroquinoline derivatives, more specifically the tetrahydroquinoline compound represented by the Formula (1) above, a salt of the tetrahydroquinoline derivatives, or a solvate of the tetrahydroquinoline derivatives or the salt, and a pharmaceutically acceptable carrier.
Further, the present invention relates to a pharmaceutical composition for potentiating hemoglobin expression comprising the 1-acyl-4-(phenoxy, benzyloxy, or phenylamino)-1,2,3,4-tetrahydroquinoline derivatives, more specifically the tetrahydroquinoline compound represented by the Formula (1) above, a salt of the tetrahydroquinoline derivatives, or a solvate of the tetrahydroquinoline derivatives or the salt, and a pharmaceutically acceptable carrier.
Further, the present invention relates to a pharmaceutical composition for the prophylaxis and/or treatment of anemia comprising the 1-acyl-4-(phenoxy, benzyloxy, or phenylamino)-1,2,3,4-tetrahydroquinoline derivatives, more specifically the tetrahydroquinoline compound represented by the Formula (1) above, a salt of the tetrahydroquinoline derivatives, or a solvate of the tetrahydroquinoline derivatives or the salt, and a pharmaceutically acceptable carrier.

Further, the present invention relates to the tetrahydroquinoline compounds selected from the group of compounds described below, a salt of the tetrahydroquinoline compounds, or a solvate of the tetrahydroquinoline compounds or the salt.
A group of compounds consisting of:
1-acetyl-8-fluoro-4-[(2-fluorophenyl)amino]-2-methyl-1,2,3,4-tetrahydroquinoline [Compound (4)];
1-cyclohexanecarbonyl-6-fluoro-4-[(4-fluorophenyl)amino]-2-methyl-1,2,3,4-tetrahydroquinoline [Compound (6)];
1-acetyl-7-cyano-2-methyl-4-phenylamino-1,2,3,4-tetrahydroquinoline [Compound (7)];
1-acetyl-6-cyano-2-methyl-4-phenylamino-1,2,3,4-tetrahydroquinoline [Compound (14)];
1-acetyl-4-[(4-isopropoxyphenyl)amino]-2-methyl-1,2,3,4-tetrahydroquinoline [Compound (18)];
1-acetyl-2-methyl-4-[(4-morpholinophenyl)amino]-1,2,3,4-tetrahydroquinoline [Compound (19)];
1-acetyl-4-[(4-N,N-dimethylaminophenyl)amino]-2-methyl-1,2,3,4-tetrahydroquinoline [Compound (20)];
1-acetyl-7-bromo-2-methyl-4-phenylamino-1,2,3,4-tetrahydroquinoline [Compound (23)];
1-acetyl-4-[(4-hydroxyphenyl)amino]-2-methyl-1,2,3,4-tetrahydroquinoline [Compound (24)];
1-acetyl-2-methyl-4-[(1,1'-biphenyl-4-yl)amino]-1,2,3,4-tetrahydroquinoline [Compound (25)];
1-acetyl-2-methyl-4-phenoxy-1,2,3,4-tetrahydroquinoline [Compound (26)];
1-acetyl-4-(4-fluorophenoxy)-2-methyl-1,2,3,4-tetrahydroquinoline [Compound (27)];
1-acetyl-4-(3-fluorophenoxy)-2-methyl-1,2,3,4-tetrahydroquinoline [Compound (28)];
1-acetyl-4-(2-fluorophenoxy)-2-methyl-1,2,3,4-tetrahydroquinoline [Compound (29)];
1-acetyl-4-(2,4-difluorophenoxy)-2-methyl-1,2,3,4-tetrahydroquinoline [Compound (30)];
1-acetyl-4-(3,4-difluorophenoxy)-2-methyl-1,2,3,4-tetrahydroquinoline [Compound (31)];
1-acetyl-7-fluoro-2-methyl-4-phenoxy-1,2,3,4-tetrahydroquinoline [Compound (32)];
1-acetyl-8-fluoro-2-methyl-4-phenoxy-1,2,3,4-tetrahydroquinoline [Compound (33)];
1 -acetyl-4-(4-fluorophenoxy)-6-fluoro-2-methyl-1,2,3,4-tetrahydroquinoline [Compound (34)];
1-acetyl-6-fluoro-2-methyl-4-phenoxy-1,2,3,4-tetrahydroquinoline [Compound (35)];
1-acetyl-2-methyl-4-benzyloxy-1,2,3,4-tetrahydroquinoline [Compound (36)];
1-acetyl-4-[(4-fluorophenyl)amino]-2-methyl-6-methoxy-1,2,3,4-tetrahydroquinoline [Compound (37)];
1-acetyl-4-[(4-hydroxymethylphenyl)amino]-2-methyl-1,2,3,4-tetrahydroquinoline [Compound (38)];
1-acetyl-4-[(4-methanesulfonylamidephenyl)amino]-2-methyl-1,2,3,4-tetrahydroquinoline [Compound (39)];
1-acetyl-4-[(4-chlorophenyl)amino]-2-methyl-6-morpholino-1,2,3,4-tetrahydroquinoline [Compound (40)];
ethyl 1-acetyl-4-[(4-chlorophenyl)amino]-2-methyl-1,2,3,4-tetrahydroquinoline-6-carboxylate [Compound (41)];
1-acetyl-4-[(4-chlorophenyl)amino]-2-methyl-1,2,3,4-tetrahydroquinoline-6-carboxylic acid [Compound (42)];
1-acetyl-4-[(4-chlorophenyl)amino]-2-methyl-1,2,3,4-tetrahydroquinoline-6-carboxamide [Compound (43)];
1-acetyl-4-[(4-chlorophenyl)amino]-2-methyl-7-morpholino-1,2,3,4-tetrahydroquinoline [Compound (44)];
1-acetyl-4-[(4-chlorophenyl)amino]-2-methyl-6-methanesulfonylamino-1,2,3,4-tetrahydroquinoline [Compound (45)];
ethyl 1-acetyl-4-(4-fluorophenoxy)-2-methyl-1,2,3,4-tetrahydroquinoline-6-carboxylate [Compound (53)];
1-acetyl-4-(4-fluorophenoxy)-2-methyl-1,2,3,4-tetrahydroquinoline-6-carboxamide [Compound (54)];
1-acetyl-4-(4-morpholinophenoxy)-2-methyl-1,2,3,4-tetrahydroquinoline [Compound (55)];
1 -acetyl-7-fluoro-4-(4-fluorophenoxy)-2-methyl-1,2,3,4-tetrahydroquinoline [Compound (56)];
1-acetyl-4-(4-hydroxyphenoxy)-2-methyl-1,2,3,4-tetrahydroquinoline [Compound (57)];
1-acetyl-7-fluoro-4-[(3-fluorophenyl)amino]-2-methyl-1,2,3,4-tetrahydroquinoline [Compound (58)];
1-acetyl-2-ethyl-4-phenylamino-1,2,3,4-tetrahydroquinoline [Compound (59)];
1-acetyl-3,3-dimethyl-4-phenylamino-1,2,3,4-tetrahydroquinoline [Compound (60)];
1-acetyl-3,3-dimethyl-4-phenylamino-1,2,3,4-tetrahydroquinoline [Compound (61)];
1-acetyl-4-(3,5-difluorophenoxy)-2-methyl-1,2,3,4-tetrahydroquinoline [Compound (62)];
1-acetyl-8-bromo-4-phenylamino-2-methyl-1,2,3,4-tetrahydroquinoline [Compound (63)];
1-acetyl-4-(4-benzyloxyphenoxy)-2-methyl-1,2,3,4-tetrahydroquinoline [Compound (64)];
6-fluoro-4-[(4-fluorophenyl)amino]-2-methyl-1-N-methylcarbamoyl-1,2,3,4-tetrahydroquinoline [Compound (65)]; 1-cyclopentanecarbonyl-6-fluoro-2-methyl-4-[(4-fluorophenyl)amino]-1,2,3,4-tetrahydroquinoline [Compound (66)];
1-acetyl-2-methyl-4-(4-nitrophenoxy)-1,2,3,4-tetrahydroquinoline [Compound (67)];
1-acetyl-4-(4-aminophenoxy)-2-methyl-1,2,3,4-tetrahydroquinoline [Compound (68)];
1-acetyl-4-[(4-methoxymethylphenyl)amino]-2-methyl-1,2,3,4-tetrahydroquinoline [Compound (69)];
1-acetyl-4-[(4-ethoxycarbonylmethylphenyl)amino]-2-methyl-1,2,3,4-tetrahydroquinoline [Compound (70)];
1-acetyl-4-[(4-carboxymethylphenyl)amino]-2-methyl-1,2,3,4-tetrahydroquinoline [Compound (71)];
1-acetyl-2-methyl-4-[(2-morpholinophenyl)amino]-1,2,3,4-tetrahydroquinoline [Compound (72)];
1-acetyl-4-[(4-fluoro-3-morpholinophenyl)amino]-2-methyl-1,2,3,4-tetrahydroquinoline [Compound (73)];
1-acetyl-6-bromo-4-[(4-chlorophenyl)amino]-2-methyl-1,2,3,4-tetrahydroquinoline [Compound (74)];
1-acetyl-2-methyl-4-[(4-piperazinylphenyl)amino]-1,2,3,4-tetrahydroquinoline [Compound (76)];
cis-1-acetyl-4-{[4-(4-acetylpiperazinyl)phenyl]amino}-2-methyl-1,2,3,4-tetrahydroquinoline [Compound (77)];
cis-1-acetyl-4-{[4-(4-methanesulfonylpiperazinyl)phenyl]amino}-2-methyl-1,2,3,4-tetrahydroquinoline [Compound (78)];
cis-1-acetyl-6-[(4-acetyl)piperazino]-4-[(4-chlorophenyl)amino]-2-methyl-1,2,3,4-tetrahydroquinoline [Compound (79)];
1-acetyl-6-[(4-methanesulfonyl)piperazino]-4-[(4-chlorophenyl)amino]-2-methyl-1,2,3,4-tetrahydroquinoline [Compound (80)];
1-acetyl-4-[(4-chlorophenyl)amino]-6-[(4-isopropyl)piperazino]-2-methyl-1,2,3,4-tetrahydroquinoline [Compound (81)];
1-acetyl-4-[(4-chlorophenyl)amino]-6-[(2-hydroxy)ethylamino]-2-methyl-1,2,3,4-tetrahydroquinoline [Compound (82)];
1-acetyl-4-[(4-chlorophenyl)amino]-6-[(cis-3,5-dimethyl)morpholino]-2-methyl-1,2,3,4-tetrahydroquinoline [Compound (83)];
1-acetyl-4-[(4-chlorophenyl)amino]-6-[(4-isopropylcarbonyl)piperazino]-2-methyl-1,2,3,4-tetrahydroquinoline [Compound (84)];
1-acetyl-4-[(4-chlorophenyl)amino]-6-[(4-cyclohexylcarbonyl)piperazino]-2-methyl-1,2,3,4-tetrahydroquinoline [Compound (85)];
1-acetyl-6-[(4-benzoyl)piperazino]-2-methyl-4-[(4-chlorophenyl)amino]-1,2,3,4-tetrahydroquinoline [Compound (86)];
1-acetyl-4-[(4-chlorophenyl)amino]-6-[4-(N,N-diethylaminocarbonyl)piperazino]-2-methyl-1,2,3,4-tetrahydroquinoline [Compound (87)];
1-acetyl-4-[(4-chlorophenyl)amino]-6-[4-(isopropylaminocarbonyl)piperazino]-2-methyl-1,2,3,4-tetrahydroquinoline [Compound (88)];
1-acetyl-4-[(4-carboxymethylphenyl)amino]-6-morpholino-2-methyl-1,2,3,4-tetrahydroquinoline [Compound (89)];
1-acetyl-4-[(4-carbamoylmethylphenyl)amino]-2-methyl-6-morpholino-1,2,3,4-tetrahydroquinoline [Compound (90)];
1-acetyl-6-(4-acetylpiperazinyl)-4-[(4-carboxymethylphenyl)amino]-2-methyl-1,2,3,4-tetrahydroquinoline [Compound (91)];
1-acetyl-2-methyl-4-[(4-morpholinophenyl)amino]-1,2,3,4-tetrahydroquinoline-6-carboxamide [Compound (92)];
1-acetyl-4-[(4-chlorophenyl)amino]-6-[(1-morpholino)carbonyl]-2-methyl-1,2,3,4-tetrahydroquinoline [Compound (93)];
cis-1-acetyl-6-[(4-acetyl)piperazino]-2-methyl-4-[(4-morpholinophenyl)amino]-1,2,3,4-tetrahydroquinoline [Compound (94)];
1-acetyl-6-amino-4-[(4-chlorophenyl)amino]-2-methyl-1,2,3,4-tetrahydroquinoline [Compound (95)];
1-acetyl-6-acetylamino-4-[(4-chlorophenyl)amino]-2-methyl-1,2,3,4-tetrahydroquinoline [Compound (96)];
1-acetyl-4-[(4-chlorophenyl)amino]-2-methyl-1,2,3,4-tetrahydroquinoline-6-ethylcarbamate [Compound (97)];
1-acetyl-6-methanesulfonylamino-2-methyl-4-[(4-morpholinophenyl)amino]-1,2,3,4-tetrahydroquinoline [Compound (98)];
1-acetyl-6-methanesulfonylamino-2-methyl-4-[(4-ethoxycarbonylmethylphenyl)amino]-1,2,3,4-tetrahydroquinoline [Compound (99)];
1-acetyl-4-[(4-fluorophenyl)amino]-2-methyl-1,2,3,4-tetrahydroquinoline-6-carboxylic acid [Compound (100)];
ethyl 1-acetyl-4-[(4-methanesulfonylaminophenyl)amino]-2-methyl-1,2,3,4-tetrahydroquinoline-6-carboxylate [Compound (101)];
cis-1-acetyl-4-[(4-methanesulfonylaminophenyl)amino]-2-methyl-1,2,3,4-tetrahydroquinoline-6-carboxamide [Compound (102)];
ethyl 1-acetyl-4-[(4-cyanomethylphenyl)amino]-2-methyl-1,2,3,4-tetrahydroquinoline-6-carboxylate [Compound (103)];
1-acetyl-4-[(4-cyanomethylphenyl)amino]-2-methyl-1,2,3,4-tetrahydroquinoline-6-carboxamide [Compound (104)];
cis-1-acetyl-4-[(4-carboxymethylphenyl)amino]-2-methyl-1,2,3,4-tetrahydroquinoline-6-carboxamide [Compound (105)];
cis-1-acetyl-4-[(4-carbamoylmethylphenyl)amino]-2-methyl-1,2,3,4-tetrahydroquinoline-6-carboxamide [Compound (106)];
1-acetyl-4-[(4-chlorophenyl)amino]-7-methanesulfonylamino-2-methyl-1,2,3,4-tetrahydroquinoline [Compound (107)];
1-acetyl-4-[(4-hydroxy-3-methoxycarbonylphenyl)amino]-2-methyl-1,2,3,4-tetrahydroquinoline [Compound (108)];
cis-1-acetyl-4-[(2-carboxyphenyl)amino]-2-methyl-1,2,3,4-tetrahydroquinoline [Compound (109)];
1-acetyl-6-[cis-2,6-dimethylmorpholin-4-yl]-4-[(4-methanesulfonylaminophenyl)amino]-2-methyl-1,2,3,4-tetrahydroquinoline [Compound (110)];
1-acetyl-6-[(4-isopropylcarbonyl)piperazino]-4-[(4-methanesulfonylaminophenyl)amino]-2-methyl-1,2,3,4-tetrahydroquinoline [Compound (111)];
cis-1-acetyl-4-[(4-benzylphenyl)amino]-2-methyl-1,2,3,4-tetrahydroquinoline [Compound (112)];
1-acetyl-4-[(4-chlorophenyl)amino]-N,N,2-trimethyl-1,2,3,4-tetrahydroquinoline-6-carboxamide [Compound (113)];
1-acetyl-4-[(4-chlorophenyl)amino]-N,2-dimethyl-1,2,3,4-tetrahydroquinoline-6-carboxamide [Compound (114)];
1-acetyl-4-[(4-chlorophenyl)amino]-2-methyl-1,2,3,4-tetrahydroquinoline-6-carbohydrazide [Compound (115)];
1-acetyl-4-[(4-chlorophenyl)amino]-6-cyano-2-methyl-1,2,3,4-tetrahydroquinoline [Compound (117)];
1-acetyl-4-[(4-chlorophenyl)amino]-N-methoxy-2-methyl-1,2,3,4-tetrahydroquinoline-6-carboxamide [Compound (118)];
1-acetyl-4-[(4-chlorophenyl)amino]-2-methyl-6-(1H-tetrazol-5-yl)-1,2,3,4-tetrahydroquinoline [Compound (119)];
1-acetyl-4-[(4-chlorophenyl)amino]-2-methyl-6-[1,2,4-oxadiazol-5(2H)-on-3-yl]-1,2,3,4-tetrahydroquinoline [Compound (120)];
cis-1-acetyl-4-[(4-chlorophenyl)amino]-6-hydroxymethyl-2-methyl-1,2,3,4-tetrahydroquinoline [Compound (121)];
1-acetyl-4-[(4-ethoxycarbonylmethylphenyl)amino]-6-fluoro-2-methyl-1,2,3,4-tetrahydroquinoline [Compound (122)];
1-acetyl-4-[(4-carboxymethylphenyl)amino]-6-fluoro-2-methyl-1,2,3,4-tetrahydroquinoline [Compound (123)];
1-acetyl-4-[(4-carbamoylmethylphenyl)amino]-6-fluoro-2-methyl-1,2,3,4-tetrahydroquinoline [Compound (124)]; and
1-acetyl-4-[4-(N,N-dimethylaminocarbonylmethyl)phenylamino]-6-fluoro-2-methyl-1,2,3,4-tetrahydroquinoline [Compound (125)]

Further, the present invention relates to a pharmaceutical composition consisting of one, two or more of the tetrahydroquinoline compounds selected from the group of compounds described above, a salt of the tetrahydroquinoline compounds, or a solvate of the tetrahydroquinoline compounds or the salt, and a pharmaceutically acceptable carrier.
Further, the pharmaceutical composition described above relates to a pharmaceutical composition used for promoting EPO production.
Further, the pharmaceutical composition described above relates to a pharmaceutical composition used for potentiating hemoglobin expression.
Further, the pharmaceutical composition described above relates to a pharmaceutical composition used for the prophylaxis and/or treatment of anemia.

### Effects of the Invention

According to the present invention, it was found that the 1-acyl-4-(phenoxy, benzyloxy, or phenylamino)-1,2,3,4-tetrahydroquinoline derivatives, more specifically the tetrahydroquinoline compound represented by the Formula (1) above, a salt of the tetrahydroquinoline derivatives, or a solvate of the tetrahydroquinoline derivatives or the salt have an excellent activity of promoting EPO production and/or an excellent activity of potentiating hemoglobin expression. As such, they are useful for a pharmaceutical composition for the prophylaxis and/or treatment of disorders of which symptoms are improved by promoting EPO production and/or by potentiating hemoglobin expression (for example, anemia such as anemia in a patient with chronic renal failure, anemia associated with autologous blood trasnfurtion or prematurity, anemia in a patient with AIDS, anemia in a cancer patient having chemotherapy, chronic anemia, iron deficiency anemia, aplastic anemia, hemolytic anemia, megaloblastic anemia and the like). Further, the present invention is to provide an agent for the prophylaxis and/or treatment of anemia comprising a low molecular weight compound as an effective component, wherein the compound has an activity of promoting EPO production and/or an activity of potentiating hemoglobin expression and can be orally administered.

### Best Mode for Carrying Out the Invention

Hereinafter, the present invention will be described in detail.
Definitions of the terms used in the present invention are as follows.
In the present invention, the "1-acyl-4-(phenoxy, benzyloxy, or phenylamino)-1,2,3,4-tetrahydroquinoline derivatives" indicates 1-acyl-4-(phenoxy, benzyloxy, or phenylamino)-1,2,3,4-tetrahydroquinoline in which the nitrogen atom at 1-position is acylated and a phenoxy group, a benzyloxy group, or a phenylamino group is bonded at 4-position, or derivatives thereof. As for the acyl group which is bonded at 1-position, an alkylcarbonyl group, a cycloalkylcarbonyl group, or an aminocarbonyl group and the like can be mentioned. Thus, more specifically, the "1-acyl-4-(phenoxy, benzyloxy, or phenylamino)-1,2,3,4-tetrahydroquinoline" of the present invention can be "1-(alkylcarbonyl, cycloalkylcarbonyl, or aminocarbonyl)-4-(phenoxy, benzyloxy, or phenylamino)-1,2,3,4-tetrahydroquinoline".
The derivatives of the present invention correspond to a general name of 1-acyl-4-(phenoxy, benzyloxy, or phenylamino)-1,2,3,4-tetrahydroquinoline in which two hydrogen atoms at 2-position, two hydrogen atoms at 3-position, the hydrogen atom of a phenoxy group, a benzyloxy group, or a phenyl group or an amino group in an aminophenyl group at 4-position, and the hydrogen atom at 5-position, 6-position, 7-position and 8-position is replaced with an atom other than hydrogen atom or with a substituent (atomic group). As for such atom and substituent, a halogen atom, a C₁₋₆ alkyl group, a C₂₋₆ alkenyl group, a C₂₋₆ alkynyl group, a C₃₋₆ cycloalkyl group, a C₆₋₁₄ aryl group, a C₇₋₁₈ arylalkyl group, a 5- to 10-membered heterocyclic group, a C₁₋₆ alkoxy group, a C₆₋₁₄ aryloxy group, a C₇₋₁₈ arylalkoxy group, an amino group, a C₁₋₆ alkylcarbonylamino group, a C₁₋₆ alkoxycarbonylamino group, a C₁₋₆ alkylamino group, a di C₁₋₆ alkylamino group, a C₆₋₁₄ arylamino group, a C₇₋₁₈ arylalkylamino group, a C₁₋₆ alkylthio group, a C₁₋₆ alkylsulfonyl group, a C₁₋₆ alkylsulfonamide group, a C₁₋₆ alkylsulfinyl group, a C₁₋₆ alkanoyl group, a 5- to 7-membered saturated heterocyclic carbonyl group, a hydroxy group, a nitro group, a nitrile group, a carboxy group, a carbamoyl group and the like can be mentioned, for example.
As for the 1,2,3,4-tetrahydroquinoline derivatives preferred in the present invention, 1-acyl-2-alkyl-4-(phenoxy, benzyloxy, or phenylamino)-1,2,3,4-tetrahydroquinoline in which an alkyl group, preferably an alkyl group having 1 to 6 carbon atoms, more preferably an alkyl group having 1 to 4 carbon atoms, is bonded to the carbon atom at 2-position, preferably in cis configuration, or derivatives thereof can be mentioned. There can be one or two substituents for the carbon atom at 2-position. Preferably, there is one substituent. In addition, as for the substituent which is preferred for the carbon atom at 3-position, an alkyl group can be mentioned.

The "halogen atom" of the present invention indicates a halogeno group.
Specifically, it is a fluorine atom, a chlorine atom, a bromine atom or an iodine atom.
The "alkyl group" of the present invention can be a linear or a branched group. Therefore, as a specific example of the "C₁₋₆ alkyl group", a linear or branched alkyl group having 1 to 6 carbon atoms such as a methyl group, an ethyl group, an n-propyl group, an isopropyl group, an n-butyl group, an isobutyl group, a sec-butyl group, a tert-butyl group, an n-pentyl group, an isopentyl group, a neopentyl group, a 4-methylbutyl group, a 1-ethylpropyl group, an n-hexyl group, an isohexyl group, a 4-methylpentyl group, a 3-methylpentyl group, a 2-methylpentyl group, a 1-methylpentyl group, a 3,3-dimethylbutyl group, a 2,2-dimethylbutyl group, a 1,1-dimethylbutyl group, a 1,2-dimethylbutyl group, a 1,3-dimethylbutyl group, a 2,3-dimethylbutyl group, a 1-ethylbutyl group, a 2-ethylbutyl group and the like can be mentioned. More preferably, it is a "C₁₋₄ alkyl group".
As for the specific example of the "C₁₋₄ alkyl group" of the present invention, a linear or branched alkyl group having 1 to 4 carbon atoms such as a methyl group, an ethyl group, an n-propyl group, an isopropyl group, an n-butyl group, an isobutyl group, a sec-butyl group, a tert-butyl group and the like can be mentioned.
In addition, as for the alkyl group in the alkylcarbonyl group of the present invention, the alkyl group described above is preferable.

The "alkenyl group" of the present invention can be a linear or a branched group. Therefore, as a specific example of the "C₂₋₆ alkenyl group", a linear or branched alkenyl group having 2 to 6 carbon atoms such as a vinyl group, a prop-1-en-1-yl group, an allyl group, an isopropenyl group, a but-1-en-1-yl group, a but-2-en-1-yl group, a but-3-en-1-yl group, a 2-methylprop-2-en-1-yl group, a 1-methylprop-2-en-1-yl group, a pent-1-en-1-yl group, a pent-2-en-1-yl group, a pent-3-en-1-yl group, a pent-4-en-1-yl group, a 3-methylbut-2-en-1-yl group, a 3-methylbut-3-en-1-yl group, a hexa-1-en-1-yl group, a hexa-2-en-1-yl group, a hexa-3-en-1-yl group, a hexa-4-en-1-yl group, a hexa-5-en-1-yl group, a 4-methylpent-3-en-1-yl group and the like can be mentioned. More preferably, it is a "C₂₋₄ alkenyl group".
As for the "C₂₋₄ alkenyl group" of the present invention, specifically, a linear or branched alkenyl group having 2 to 4 carbon atoms such as a vinyl group, a prop-1-en-1-yl group, an allyl group, an isopropenyl group, a but-1-en-1-yl group, a but-2-en-1-yl group, a but-3-en-1-yl group, a 2-methylprop-2-en-1-yl group, a 1-methylprop-2-en-1-yl group and the like can be mentioned.

The "alkynyl group" of the present invention can be a linear or a branched group. Therefore, as a specific example of the "C₂₋₆ alkynyl group", a linear or branched alkynyl group having 2 to 6 carbon atoms such as an ethynyl group, a prop-1-yn-1-yl group, a prop-2-yn-1-yl group, a but-1-yn-1-yl group, a but-3-yn-1-yl group, a 1-methylprop-2-yn-1-yl group, a pent-1-yn-1-yl group, a pent-4-yn-1-yl group, a hex-1-yn-1-yl group, a hex-5-yn-1-yl group and the like can be mentioned. More preferably, it is a "C₂₋₄ alkynyl group".
As for the "C₂₋₄ alkynyl group" of the present invention, specifically, a linear or branched alkynyl group having 2 to 4 carbon atoms such as an ethynyl group, a prop-1-yn-1-yl group, a prop-2-yn-1-yl group, a but-1-yn-1-yl group, a but-3-yn-1-yl group, a 1-methylprop-2-yn-1-yl group and the like can be mentioned.

The "C₃₋₆ cycloalkyl group" of the present invention is a monocyclic cycloalkyl group having 3 to 6 carbon atoms such as a cyclopropyl group, a cyclobutyl group, a cyclopentyl group, a cyclohexyl group and the like.
Further, the cycloalkyl group in the "cycloalkylcarbonyl" of the present invention is preferably the "C₃₋₆ cycloalkyl group" described above.
The "aryl group" of the present invention is a monocyclic, polycyclic, or condensed aromatic hydrocarbon group having 6 to 14 carbon atoms. Therefore, as a specific example of the "C₆₋₁₄ aryl group", a monocyclic, polycyclic, or condensed aromatic hydrocarbon group having 6 to 14 carbon atoms such as a phenyl group, a naphthyl group, an azulenyl group, an anthryl group, an indenyl group, a fluorenyl group, a phenanthryl group and the like can be mentioned. More preferably, it is a "C₆₋₁₀ aryl group".
The "C₆₋₁₀ aryl group" of the present invention is a monocyclic, polycyclic, or condensed aromatic hydrocarbon group having 6 to 10 carbon atoms. As a specific example, a phenyl group, a naphthyl group, an azulenyl group and the like can be mentioned.

The "heterocyclic group" of the present invention means a 5- to 10-membered saturated or unsaturated monocyclic, polycyclic, or condensed heterocyclic group having 1 to 4 hetero atoms selected from an oxygen atom, a sulfur atom and a nitrogen atom. Thus, as a specific example of the "5- to 10-membered heterocyclic group", a pyrrolidinyl group, an imidazolidinyl group, an imidazolinyl group, a pyrazolidinyl group, a pyrazolinyl group, a piperidyl group, a piperazinyl group, a morpholinyl group, a diazepan-1-yl group, a furyl group, a thienyl group, a pyrrolyl group, an oxazolyl group, an isoxazolyl group, a dihydroisoxazolyl group, a thiazolyl group, an isothiazolyl group, an imidazolyl group, a pyrazolyl group, an oxadiazolyl group, a thiadiazolyl group, a triazolyl group, a tetrazolyl group, a pyridyl group, an azepinyl group, an oxazepinyl group, a benzofuranyl group, an isobenzofuranyl group, a benzothienyl group, an indolyl group, an isoindolyl group, an indazolyl group, a benzimidazolyl group, a benzoxazolyl group, a benzisoxazolyl group, a benzothiazolyl group, a benzisothiazolyl group, a benzoxadiazolyl group, a benzothiadiazolyl group, a benzotriazolyl group, a quinolyl group, an isoquinolyl group, a cinnolinyl group, a quinazolinyl group, a quinoxalinyl group, a phthalazinyl group, a naphthylidinyl group, a purinyl group, a pteridinyl group, a carbazolyl group, a carbolinyl group, an acridinyl group, a phenoxazinyl group, a phenothiazinyl group, a phenazinyl group and the like can be mentioned. More preferably, it is a "5- to 7-membered heterocyclic group".
As a specific example of the "5- to 7-membered heterocyclic group" of the present invention, a pyrrolidinyl group, an imidazolidinyl group, a pyrazolidinyl group, a piperidyl group, a piperazinyl group, a morpholinyl group, a furyl group, a thienyl group, a pyrrolyl group, an oxazolyl group, an isoxazolyl group, a dihydroisoxazolyl group, a thiazolyl group, an isothiazolyl group, an imidazolyl group, a pyrazolyl group, an oxadiazolyl group, a thiadiazolyl group, a triazolyl group, a tetrazolyl group, and a pyridyl group can be mentioned. More specifically, the "5- to 7-membered monocyclic heterocyclic group" having 1 to 4 hetero atoms selected from an oxygen atom or a nitrogen atom such as a piperidyl group, a piperazinyl group, a morpholinyl group, a tetrazolyl group, an oxadiazolyl group and the like can be mentioned.

The "alkoxy group" of the present invention can be a linear or a branched group. Therefore, as a specific example of the "C₁₋₆ alkoxy group", a linear or branched alkoxy group having 1 to 6 carbon atoms such as a methyloxy group, an ethyloxy group, an n-propyloxy group, an isopropyloxy group, an n-butyloxy group, an isobutyloxy group, a sec-butyloxy group, a tert-butyloxy group, an n-pentyloxy group, an isopentyloxy group, a neopentyloxy group, a 4-methylbutyloxy group, a 1-ethylpropyloxy group, an n-hexyloxy group, an isohexyloxy group, a 4-methylpentyloxy group, a 3-methylpentyloxy group, a 2-methylpentyloxy group, a 1-methylpentyloxy group, a 3,3-dimethylbutyloxy group, a 2,2-dimethylbutyloxy group, a 1,1-dimethylbutyloxy group, a 1,2-dimethylbutyloxy group, a 1,3-dimethylbutyloxy group, a 2,3-dimethylbutyloxy group, a 1-ethylbutyloxy group, a 2-ethylbutyloxy group and the like can be mentioned. More preferably, it is a "C₁₋₄ alkoxy group".
As a specific example of the "C₁₋₄ alkoxy group" of the present invention, a methyloxy group, an ethyloxy group, an n-propyloxy group, an isopropyloxy group, an n-butyloxy group, an isobutyloxy group, a sec-butyloxy group, a tert-butyloxy group and the like can be mentioned.

The "alkylcarbonylamino group" of the present invention means a group in which one alkanoyl group, that is described below, is bonded to the nitrogen atom of an amino group. Therefore, a specific example of the "C₁₋₆ alkylcarbonylamino group" is a formylamino group, an acetylamino group, a propionylamino group, a butyrylamino group, an isobutyrylamino group, a valerylamino group, an isovalerylamino group, a pyvaloylamino group and the like. More preferably, it is a "C₁₋₄ alkylcarbonylamino group".

The "alkoxycarbonylamino group" of the present invention means a group in which one alkoxycarbonyl group, that is described below, is bonded to the nitrogen atom of an amino group. Therefore, as a specific example of the "C₁₋₆ alkoxycarbonylamino group", a methyloxycarbonylamino group, an ethyloxycarbonylamino group, an n-propyloxycarbonylamino group, an isopropyloxycarbonylamino group, an n-butyloxycarbonylamino group, an isobutyloxycarbonylamino group, a sec-butyloxycarbonylamino group, a tert-butyloxycarbonylamino group, an n-pentyloxycarbonylamino group, an isopentyloxycarbonylamino group, a neopentyloxycarbonylamino group, a 4-methylbutyloxycarbonylamino group, a 1-ethylpropyloxycarbonylamino group, an n-hexyloxycarbonylamino group, an isohexyloxycarbonylamino group, a 4-methylpentyloxycarbonylamino group, a 3-methylpentyloxycarbonylamino group, a 2-methylpentyloxycarbonylamino group, a 1-methylpentyloxycarbonylamino group, a 3,3-dimethylbutyloxycarbonylamino group, a 2,2-dimethylbutyloxycarbonylamino group, a 1,1-dimethylbutyloxycarbonylamino group, a 1,2-dimethylbutyloxycarbonylamino group, a 1,3-dimethylbutyloxycarbonylamino group, a 2,3-dimethylbutyloxycarbonylamino group, a 1-ethylbutyloxycarbonylamino group, a 2-ethylbutyloxycarbonylamino group and the like can be mentioned. More preferably, it is a "C₁₋₄ alkoxycarbonylamino group".

The "C₆₋₁₄ aryloxy group" of the present invention means a group in which an oxygen atom is bonded to the "C₆₋₁₄ aryl group" described above. Therefore, as a specific example of the "C₆₋₁₄ aryloxy group", a phenyloxy group, a naphthyloxy group, an azulenyloxy group, an anthryloxy group, an indenyloxy group, a fluorenyloxy group, a phenanthryloxy group and the like can be mentioned. More preferably, it is a "C₆₋₁₀ aryloxy group".
As a specific example of the "C₆₋₁₀ aryloxy group" of the present invention, a phenyloxy group, a naphthyloxy group, an azulenyloxy group and the like can be mentioned.

The "alkylamino group" of the present invention means a group in which one alkyl group described above is bonded to the nitrogen atom of an amino group. Therefore, as a specific example of the "C₁₋₆ alkylamino group", a methylamino group, an ethylamino group, an n-propylamino group, an isopropylamino group, an n-butylamino group, a sec-butylamino group, a tert-butylamino group, an n-pentylamino group, an isopentylamino group, a neopentylamino group, a 4-methylbutylamino group, a 1-ethylpropylamino group, an n-hexylamino group, an isohexylamino group, a 4-methylpentylamino group, a 3-methylpentylamino group, a 2-methylpentylamino group, a 1-methylpentylamino group, a 3,3-dimethylbutylamino group, a 2,2-dimethylbutylamino group, a 1,1-dimethylbutylamino group, a 1,2-dimethylbutylamino group, a 1,3-dimethylbutylamino group, a 2,3-dimethylbutylamino group, a 1-ethylbutylamino group, a 2-ethylbutylamino group and the like can be mentioned.
More preferably, it is a "C₁₋₄ alkylamino group".
As a specific example of the "C₁₋₄ alkylamino group", a methylamino group, an ethylamino group, an n-propylamino group, an isopropylamino group, an n-butylamino group, a sec-butylamino group, a tert-butylamino group and the like can be mentioned.

The "dialkylamino group" of the present invention means a group in which two alkyl groups described above, which are the same or different from each other, are bonded to the nitrogen atom of an amino group. Therefore, as a specific example of the "di C₁₋₆ alkylamino group", a dimethylamino group, a methylethylamino group, a diethylamino group, a methyl-n-propylamino group, an ethyl-n-propylamino group, a di-n-propylamino group, a methylisopropylamino group, an ethylisopropylamino group, a diisopropylamino group, a methyl-n-butylamino group, an ethyl-n-butylamino group, an n-propyl-n-butylamino group, a di-n-butylamino group, a di-sec-butylamino group, a di-tert-butylamino group, a dipentylamino group, a dihexylamino group and the like can be mentioned. More preferably, it is a "di C₁₋₄ alkylamino group".
As a specific example of the "di C₁₋₄ alkylamino group" of the present invention, a dimethylamino group, a methylethylamino group, a diethylamino group, a methyl-n-propylamino group, an ethyl-n-propylamino group, a di-n-propylamino group, a methylisopropylamino group, an ethylisopropylamino group, a diisopropylamino group, a methyl-n-butylamino group, an ethyl-n-butylamino group, an n-propyl-n-butylamino group, a di-n-butylamino group, a di-sec-butylamino group, a di-tert-butylamino group and the like can be mentioned.

The "C₆₋₁₄ arylamino group" of the present invention means a group in which one "C₆₋₁₄ aryl group" described above is bonded to the nitrogen atom of an amino group. Therefore, as a specific example of the "C₆₋₁₄ arylamino group", a phenylamino group, a naphthylamino group, an azulenylamino group, an anthrylamino group, an indenylamino group, a fluorenylamino group, a phenanthrylamino group and the like can be mentioned. More preferably, it is a "C₆₋₁₀ arylamino group".
As a specific example of the "C₆₋₁₀ arylamino group", a phenylamino group, a naphthylamino group, an azulenylamino group and the like can be mentioned.
Further, although the amino group comprised in the aminocarbonyl group of the present invention can be a free amino group, the "alkylamino group", "dialkylamino group", "C₆₋₁₄ arylamino group" described above and the like can be exemplified as a preferred example.

The "alkylthio group" of the present invention means a group in which one alkyl group described above is bonded to a sulfur atom. Therefore, as a specific example of the "C₁₋₆ alkylthio group", a methylthio group, an ethylthio group, an n-propylthio group, an isopropylthio group, an n-butylthio group, an isobutylthio group, a sec-butylthio group, a tert-butylthio group, an n-pentylthio group, an isopentylthio group, a neopentylthio group, a 4-methylbutylthio group, a 1-ethylpropylthio group, an n-hexylthio group, an isohexylthio group, a 4-methylpentylthio group, a 3-methylpentylthio group, a 2-methylpentylthio group, a 1-methylpentylthio group, a 3,3-dimethylbutylthio group, a 2,2-dimethylbutylthio group, a 1,1-dimethylbutylthio group, a 1,2-dimethylbutylthio group, a 1,3-dimethylbutylthio group, a 2,3-dimethylbutylthio group, a 1-ethylbutylthio group, a 2-ethylbutylthio group and the like can be mentioned. More preferably, it is a "C₁₋₄ alkylthio group".
As a specific example of the "C₁₋₄ alkylthio group" of the present invention, a methylthio group, an ethylthio group, an n-propylthio group, an isopropylthio group, an n-butylthio group, an isobutylthio group, a sec-butylthio group, a tert-butylthio group and the like can be mentioned.

The "alkylsulfonyl group" of the present invention means a sulfonyl (SO₂) group which is substituted with the alkyl group described above. Therefore, as a specific example of the "C₁₋₆ alkylsulfonyl group", a methylsulfonyl group, an ethylsulfonyl group, an n-propylsulfonyl group, an isopropylsulfonyl group, an n-butylsulfonyl group, an isobutylsulfonyl group, a sec-butylsulfonyl group, a tert-butylsulfonyl group, an n-pentylsulfonyl group, an isopentylsulfonyl group, a neopentylsulfonyl group, a 4-methylbutylsulfonyl group, a 1-ethylpropylsulfonyl group, an n-hexylsulfonyl group, an isohexylsulfonyl group, a 4-methylpentylsulfonyl group, a 3-methylpentylsulfonyl group, a 2-methylpentylsulfonyl group, a 1-methylpentylsulfonyl group, a 3,3-dimethylbutylsulfonyl group, a 2,2-dimethylbutylsulfonyl group, a 1,1-dimethylbutylsulfonyl group, a 1,2-dimethylbutylsulfonyl group, a 1,3-dimethylbutylsulfonyl group, a 2,3-dimethylbutylsulfonyl group, a 1-ethylbutylsulfonyl group, a 2-ethylbutylsulfonyl group and the like can be mentioned. More preferably, it is a "C₁₋₄ alkylsulfonyl group".
As a specific example of the "C₁₋₄ alkylsulfonyl group" of the present invention, a methylsulfonyl group, an ethylsulfonyl group, an n-propylsulfonyl group, an isopropylsulfonyl group, an n-butylsulfonyl group, an isobutylsulfonyl group, a sec-butylsulfonyl group, a tert-butylsulfonyl group and the like can be mentioned.

The "alkylsulfonamide group" of the present invention means a group in which one "alkylsulfonyl group", that is described above, is bonded to the nitrogen atom of an amino group. Therefore, as a specific example of the "C₁₋₆ alkylsulfonamide group", a methanesulfonamide group, an ethanesulfonamide group, a propanesulfonamide group, an isopropanesulfonamide group, an n-butanesulfonamide group, a 2-methylpropanesulfonamide group, a 1-methylpropanesulfonamide group, a 1,1-dimethylethylsulfonamide group, a pentanesulfonyl group, a 3-methylbutanesulfonamide group, a 2-methylbutanesulfonamide group, a 1-methylbutanesulfonamide group, a 1,1-dimethylpropanesulfonamide group, a 1,2-dimethylpropanesulfonamide group, a 2,2-dimethylpropanesulfonamide group, a 1-ethylpropanesulfonamide group, a hexanesulfonamide group, a 1-methylpentanesulfonamide group, a 2-methylpentanesulfonamide group, a 3-methylpentanesulfonamide group, a 4-methylpentanesulfonamide group, a 1,1-dimethylbutanesulfonamide group, a 1,2-dimethylbutanesulfonamide group, a 1,3-dimethylbutanesulfonamide group, a 2,2-dimethylbutanesulfonamide group, a 2,3-dimethylbutanesulfonamide group, a 3,3-dimethylbutanesulfonamide group, a 1-ethylbutanesulfonamide group, a 2-ethylbutanesulfonamide group, a 3-ethylbutanesulfonamide group and the like can be mentioned. More preferably, it is a "C₁₋₄ alkylsulfonamide group".
As a specific example of the "C₁₋₄ alkylsulfonamide group" of the present invention, a methanesulfonamide group, an ethanesulfonamide group, a propanesulfonamide group, an isopropanesulfonamide group, an n-butanesulfonamide group, a 2-methylpropanesulfonamide group, a 1-methylpropanesulfonamide group, a 1,1-dimethylethylsulfonamide group and the like can be mentioned.

The "alkylsulfinyl group" of the present invention means a sulfinyl (SO) group which is substituted with the alkyl group described above. Therefore, as a specific example of the "C₁₋₆ alkylsulfinyl group", a methylsulfinyl group, an ethylsulfinyl group, an n-propylsulfinyl group, an isopropylsulfinyl group, an n-butylsulfinyl group, an isobutylsulfinyl group, a sec-butylsulfinyl group, a tert-butylsulfinyl group, an n-pentylsulfinyl group, an isopentylsulfinyl group, a neopentylsulfinyl group, a 4-methylbutylsulfinyl group, a 1-ethylpropylsulfinyl group, an n-hexylsulfinyl group, an isohexylsulfinyl group, a 4-methylpentylsulfinyl group, a 3-methylpentylsulfinyl group, a 2-methylpentylsulfinyl group, a 1-methylpentylsulfinyl group, a 3,3-dimethylbutylsulfinyl group, a 2,2-dimethylbutylsulfinyl group, a 1,1-dimethylbutylsulfinyl group, a 1,2-dimethylbutylsulfinyl group, a 1,3-dimethylbutylsulfinyl group, a 2,3-dimethylbutylsulfinyl group, a 1-ethylbutylsulfinyl group, a 2-ethylbutylsulfinyl group and the like can be mentioned. More preferably, it is a "C₁₋₄ alkylsulfinyl group".
As a specific example of the "C₁₋₄ alkylsulfinyl group" of the present invention, a methylsulfinyl group, an ethylsulfinyl group, an n-propylsulfinyl group, an isopropylsulfinyl group, an n-butylsulfinyl group, an isobutylsulfinyl group, a sec-butylsulfinyl group, a tert-butylsulfinyl group and the like can be mentioned.

The "alkoxycarbonyl group" of the present invention indicates a group in which the "alkoxy group" described above is bonded to a carbonyl group. Therefore, as a specific example of the "C₁₋₆ alkoxycarbonyl group", a methyloxycarbonyl group, an ethyloxycarbonyl group, an n-propyloxycarbonyl group, an isopropyloxycarbonyl group, an n-butyloxycarbonyl group, an isobutyloxycarbonyl group, a sec-butyloxycarbonyl group, a tert-butyloxycarbonyl group, an n-pentyloxycarbonyl group, an isopentyloxycarbonyl group, a neopentyloxycarbonyl group, a 4-methylbutyloxycarbonyl group, a 1-ethylpropyloxycarbonyl group, an n-hexyloxycarbonyl group, an isohexyloxycarbonyl group, a 4-methylpentyloxycarbonyl group, a 3-methylpentyloxycarbonyl group, a 2-methylpentyloxycarbonyl group, a 1-methylpentyloxycarbonyl group, a 3,3-dimethylbutyloxycarbonyl group, a 2,2-dimethylbutyloxycarbonyl group, a 1,1-dimethylbutyloxycarbonyl group, a 1,2-dimethylbutyloxycarbonyl group, a 1,3-dimethylbutyloxycarbonyl group, a 2,3-dimethylbutyloxycarbonyl group, a 1-ethylbutyloxycarbonyl group, a 2-ethylbutyloxycarbonyl group and the like can be mentioned. More preferably, it is a "C₁₋₄ alkoxycarbonyl group".
As a specific example of the "C₁₋₄ alkoxycarbonyl group" of the present invention, a methyloxycarbonyl group, an ethyloxycarbonyl group, an n-propyloxycarbonyl group, an isopropyloxycarbonyl group, an n-butyloxycarbonyl group, an isobutyloxycarbonyl group, a sec-butyloxycarbonyl group, a tert-butyloxycarbonyl group can be mentioned.

The "alkanoyl group" of the present invention indicates a group in which an oxo group is bonded to the hydrogen atom or the "C₁₋₆ alkyl group" described above at 1-position. It can be a linear or branched group. Therefore, as a specific example of the "C₁₋₆ alkanoyl group", a formyl group, an acetyl group, a propionyl group, a butyryl group, an isobutyryl group, a valeryl group, an isovaleryl group, a pyvaloyl group and the like can be mentioned. More preferably, it is a "C₁₋₄ alkanoyl group".
As a specific example of the "C₁₋₄ alkanoyl group" of the present invention, a formyl group, an acetyl group, a propionyl group, a butyryl group, and an isobutyryl group can be mentioned.

The "5- to 7-membered saturated heterocyclic carbonyl group" of the present invention means a group in which the saturated "5- to 7-membered heterocycle" is bonded to a carbonyl group. As a specific example, a pyrrolidinylcarbonyl group, an imidazolidinylcarbonyl group, a pyrazolidinylcarbonyl group, a piperidylcarbonyl group, a piperazinylcarbonyl group, a morpholinylcarbonyl group and the like can be mentioned.

The "acyl group" of the present invention indicates the "alkanoyl group" described above, the "cycloalkylcarbonyl group" in which a cyclic alkyl group is bonded via carbonyl group, the "arylcarbonyl group" in which an aryl group is bonded via carbonyl group, and the "heterocyclic carbonyl group" in which a saturated or unsaturated heterocyclic group is bonded via carbonyl group. As a specific example of the "alkanoyl group", the "C₁₋₆ alkanoyl group" such as a formyl group, an acetyl group, a propionyl group, a butyryl group, an isobutyryl group, a valeryl group, an isovaleryl group, a pyvaloyl group and the like can be mentioned.
As a specific example of the "cycloalkylcarbonyl group", the "C₃₋₆ cycloalkylcarbonyl group" such as a cyclopropylcarbonyl group, a cyclobutylcarbonyl group, a cyclopentylcarbonyl group, a cyclohexylcarbonyl group and the like can be mentioned. As a specific example of the "arylcarbonyl group", the "C₆₋₁₀ arylcarbonyl group" such as a benzoyl group, a naphthylcarbonyl group, an azulenylcarbonyl group and the like can be mentioned. As a specific example of the "heterocyclic carbonyl group", the "5- to 10-membered heterocyclic carbonyl group" such as a pyrrolidinylcarbonyl group, an imidazolidinylcarbonyl group, an oxazolidinylcarbonyl group, a thiazolidinylcarbonyl group, an oxolanylcarbonyl group, a morpholinylcarbonyl group, a piperazinylcarbonyl group, a piperidinylcarbonyl group, a hexahydro-1H-1,4-diazepinylcarbonyl group, a furoyl group, a thenoyl group, a pyrrolylcarbonyl group, a pyridylcarbonyl group, a pyrazinylcarbonyl group, a pyrimidinylcarbonyl group, a pyridazinylcarbonyl group, an imidazolylcarbonyl group, a pyrazolylcarbonyl group, an oxazolylcarbonyl group, an isoxazolylcarbonyl group, a thiadiazolylcarbonyl group, a 1,2,3-triazolylcarbonyl group, a 1,2,4-triazolylcarbonyl group, a tetrazolylcarbonyl group, a benzofuranylcarbonyl group, an isobenzofuranylcarbonyl group, a benzothiophenylcarbonyl group, an indolylcarbonyl group, an indolinylcarbonyl group, an isoindolylcarbonyl group, an indazolylcarbonyl group, a benzimidazolylcarbonyl group, a benzoxazolylcarbonyl group, a benzisooxazolylcarbonyl group, a benzothiazolylcarbonyl group, a benzisothiazolylcarbonyl group, a benzotriazolylcarbonyl group, a chromenylcarbonyl group, a quinolylcarbonyl group, an isoquinolylcarbonyl group, a 1,2,3,4-tetrahydroquinolylcarbonyl group, a 1,2,3,4-tetrahydroisoquinolylcarbonyl group, a cinnolinylcarbonyl group, a quinazolinylcarbonyl group, a quinoxalinylcarbonyl group, a phthalazinylcarbonyl group, a naphthylidinylcarbonyl group and the like can be mentioned.

As for the "substituent" comprised in the "C₁₋₆ alkyl group which may be substituted", the "C₂₋₆ alkenyl group which may be substituted", the "C₂₋₆ alkynyl group which may be substituted", the "C₃₋₆ cycloalkyl group which may be substituted", the "C₆₋₁₄ aryl group which may be substituted", the "5- to 10-membered heterocyclic group which may be substituted", the "C₁₋₆ alkoxy group which may be substituted", the "C₆₋₁₄ aryloxy group which may be substituted", the "C₁₋₆ alkylamino group which may be substituted", the "di C₁₋₆ alkylamino group which may be substituted", the "C₆₋₁₄ arylamino group which may be substituted", the "C₁₋₆ alkylthio group which may be substituted", the "C₁₋₆ alkylsulfonyl group which may be substituted", the "C₁₋₆ alkylsulfonamide group which may be substituted", the "C₁₋₆ alkylsulfinyl group which may be substituted", the "C₁₋₆ alkoxycarbonyl group which may be substituted", the "C₁₋₆ alkanoyl group which may be substituted", the "5- to 7-membered saturated heterocyclic carbonyl group which may be substituted", the "carbamoyl group which may be substituted" of the present invention, a halogen atom, a C₁₋₆ alkyl group, a C₂₋₆ alkenyl group, a C₂₋₆ alkynyl group, a C₃₋₆ cycloalkyl group, a C₆₋₁₄ aryl group, a C₇₋₁₈ arylalkyl group, a 5- to 10-membered heterocyclic group, a C₁₋₆ alkoxy group, a C₆₋₁₄ aryloxy group, a C₇₋₁₈ arylalkoxy group, a C₁₋₆ alkylamino group, a di C₁₋₆ alkylamino group, a C₆₋₁₄ arylamino group, a C₇₋₁₈ arylalkylamino group, a C₁₋₆ alkylthio group, a C₁₋₆ alkylsulfonyl group, a C₁₋₆ alkylsulfonamide group, a C₁₋₆ alkylsulfinyl group, a C₁₋₆ alkanoyl group, a hydroxy group, a nitro group, a nitrile group, a carboxyl group, a carbamoyl group, a C₁₋₆ alkoxycarbonyl group, a C₁₋₆ alkylsulfonamide group, a cyano group, an amino group, a monoalkylaminocarbonyl group such as isopropylaminocarbonyl group and the like, a dialkylaminocarbonyl group such as dimethylaminocarbonyl group, a diethylaminocarbonyl group and the like, an alkylcarbonyl group such as isopropylcarbonyl group and the like, a cycloalkylcarbonyl group such as cyclopropylcarbonyl group and the like, an arylcarbonyl group such as phenylcarbonyl group and the like, a C₁₋₆ alkanoylamino group such as acetylamino group and the like can be mentioned.

Regarding the number of the substituent, if it is not specifically described, it substantially means that "it may be substituted with at least one substituent", more preferably "it may be substituted with 1 to 5 substituents", and still more preferably "it may be substituted with 1 to 3 substituents". Therefore, in the "C₁₋₆ alkyl group which may be substituted", a halo C₁₋₆ alkyl group and the like is included.

As an example of the "halo C₁₋₆ alkyl group" of the present invention, a trifluoromethyl group, a 2-fluoroethyl group, a 2-chloroethyl group, a 2-bromoethyl group, a 3-fluoropropyl group, a 3-chloropropyl group, a 4-fluorobutyl group, a 4-chlorobutyl group, a 2,2,2-trifluoroethyl group, a 3,3,3-trifluoropropyl group, a pentafluoroethyl group, a 2,2,2-trifluoro-1-trifluoromethylethyl group and the like can be mentioned. More preferably, it is a "halo C₁₄ alkyl group".

The "carbon ring" included in the phrase "R⁹ and R¹⁰ together may form a carbon ring or a heterocycle" means a saturated or partially saturated 3- to 7-membered carbon ring formed with adjacent carbon atoms. As an example thereof, a cyclopropene ring, a cyclobutene ring, a cyclopentene ring, a cyclohexene ring, a cycloheptene ring, a benzene ring and the like can be mentioned.
The "heterocycle" included in the phrase "R⁹ and R¹⁰ together may form a carbon ring or a heterocycle" means a saturated or partially saturated 3- to 7-membered heterocycle formed with adjacent carbon atoms and comprises 1 to 4 hetero atoms selected from an oxygen atom, a sulfur atom and a nitrogen atom. As an example thereof, a 1,3-dioxol ring, a 1,4-dioxene ring, a 1,4-dihydropyridine ring, a 1,2,3,4-tetrahydropyrazine ring, a 6,7-dihydro-5H-1,4-dioxepine ring, a furan ring, a thiophene ring, a pyrrole ring, an oxazole ring, a thiazole ring, an imidazole ring, a pyrazole ring, a 1,2,3-oxadiazole ring, a 1,2,3-thiadiazole ring, a 1,2,3-triazole ring, a pyridine ring, a pyrimidine ring, a pyridazine ring, a 1H-1,4-diazepine ring and the like can be mentioned.

Furthermore, with respect to the group that is not defined herein, it has a definition that is generally acknowledged in the art.

In the Formula (1), as for the "C₁₋₆ alkyl group" of R¹, the "C₁₋₄ alkyl group" is preferable. For example, a methyl group and an ethyl group are more preferable, and a methyl group is particularly preferable.
In the Formula (1), as for the "C₃₋₆ cycloalkyl group" of R¹, a cyclopropyl group, a cyclobutyl group, a cyclopentyl group, and a cyclohexyl group are preferable, for example. A cyclopropyl group is more preferable.
In the Formula (1), as for the "C₁₋₆ alkylamino group" of R¹, the "C₁₋₄ alkylamino group" is preferable. For example, a methylamino group is more preferable.

In the Formula (1), as for R² and R^{2'}, a hydrogen atom and a C₁₋₆ alkyl group which may be substituted are preferable. As for the "C₁₋₆ alkyl group", the "C₁₋₄ alkyl group" is more preferable, and a methyl group is particularly preferable. Further, when R² is a C₁₋₆ alkyl group, R^{2'} is preferably a hydrogen atom.

In the Formula (1), R³ and R^{3'} are preferably a hydrogen atom.

In the Formula (1), as for the "halogen atom" of R⁴, a fluorine atom and a bromine atom are preferable.
In the Formula (1), as for the "halogen atom" of R⁵, a fluorine atom and a bromine atom are preferable.
In the Formula (1), as for the "C₁₋₆ alkyl group which may be substituted" of R⁵, the "C₁₋₄ alkyl group which may be substituted" is preferable. A methyl group and a hydroxymethyl group are more preferable.
In the Formula (1), as for the "C₁₋₆ alkoxy group" of R⁵, the "C₁₋₄ alkoxy group" is preferable. A methyloxy group is more preferable.
In the Formula (1), as for the "5- to 10-membered heterocyclic group" of R⁵, the "5- to 7-membered heterocyclic group" is preferable. For example, a piperidyl group (a piperidino group, a piperidin-2-yl group, a piperidin-3-yl group, a piperidin-4-yl group), a piperazinyl group (a piperazino group, a piperazin-2-yl group), a morpholinyl group (a morpholino group, a morpholin-2-yl group, a morpholin-3-yl group), a tetrazolyl group, and an oxadiazolyl group are more preferable, and a piperidino group, a piperazino group, a morpholino group, a tetrazolyl group and an oxadiazolyl group are particularly preferable.
In the Formula (1), as for the "C₁₋₆ alkoxycarbonyl group" of R⁵, the "C₁₋₄ alkoxycarbonyl group" is preferable. For example, a methyloxycarbonyl group and an ethyloxycarbonyl group are particularly preferable.
In the Formula (1), as for the "C₁₋₆ alkylsulfonamide group" of R⁵, the "C₁₋₄ alkylsulfonamide group" is preferable. For example, a methanesulfonamide group is more preferable.
In the Formula (1), as for the "C₁₋₆ alkylamino group which may be substituted" of R⁵, a hydroxyethylamino group is preferable.
In the Formula (1), as for the "C₁₋₆ alkylcarbonylamino group" of R⁵, the "C₁₋₄ alkylcarbonylamino group" is preferable and an acetylamino group is preferable.
In the Formula (1), as for the "C₁₋₆ alkoxycarbonylamino group" of R⁵, the "C₁₋₄ alkoxycarbonylamino group" is preferable and an ethoxycarbonylamino group is preferable.
In the Formula (1), as for the "5- to 7-membered saturated heterocyclic carbonyl group" of R⁵, a morpholinocarbonyl group is preferable.
In the Formula (1), as for the "carbamoyl group which may be substituted" of R⁵, a mono C₁₋₆ alkylcarbamoyl group, a di C₁₋₆ alkylcarbamoyl group and a C₁₋₆ alkoxycarbamoyl group are preferable. As for the mono C₁₋₆ alkylcarbamoyl group, a methylcarbamoyl group is particularly preferable. As for the di C₁₋₆ alkylcarbamoyl group, a dimethylcarbamoyl group is particularly preferable. As for the C₁₋₆ alkoxycarbamoyl group, a methoxycarbamoyl group is particularly preferable.

In the Formula (1), as for the "halogen atom" of R⁶, a fluorine atom and a bromine atom are preferable.
In the Formula (1), as for the "C₁₋₆ alkoxy group" of R⁶, the "C₁₋₄ alkoxy group" is preferable. A methyloxy group is more preferable, for example.
In the Formula (1), as for the "5- to 10-membered heterocyclic group" of R⁶, the "5- to 7-membered heterocyclic group" is preferable. For example, a piperidyl group (a piperidino group, a piperidin-2-yl group, a piperidin-3-yl group, a piperidin-4-yl group), a piperazinyl group (a piperazino group, a piperazin-2-yl group), and a morpholinyl group (a morpholino group, a morpholin-2-yl group, a morpholin-3-yl group) are more preferable, and a piperidino group, a piperazino group and a morpholino group are particularly preferable.

In the Formula (1), as for the "halogen atom" of R⁷, a fluorine atom is preferable.

In the Formula (1), as for the "halogen atom" of R⁸ to R¹⁰, a fluorine atom and a chlorine atom are preferable.
In the Formula (1), as for the "C₁₋₆ alkyl group" of R⁸ to R¹⁰, the "C₁₋₄ alkyl group" is preferable. For example, a methyl group, an ethyl group and an isopropyl group are more preferable. As for the "substituent" for the "C₁₋₆ alkyl group", it is preferable that one hydroxyl group, one C₁₋₆ alkoxy group, one carboxy group or one C₁₋₆ alkoxycarbonyl group is substituted thereto or one to three halogen atoms (in particular, a fluorine atom) are substituted thereto.
In the Formula (1), as for the "C₁₋₆ alkoxy group" of R⁸ to R¹⁰, the "C₁₋₄ alkoxy group" is preferable. A methyloxy group, an ethyloxy group and an isopropyloxy group are more preferable, for example.
In the Formula (1), as for the "di C₁₋₆ alkylamino group" of R⁸ to R¹⁰, the "di C₁₋₄ alkylamino group" is preferable. A dimethylamino group is more preferable, for example.
In the Formula (1), as for the "C₆₋₁₄ aryl group" of R⁸ to R¹⁰, the "C₆₋₁₀ aryl group" is preferable. A phenyl group is more preferable, for example.
In the Formula (1), as for the "5- to 10-membered heterocyclic group" of R⁸ to R¹⁰, the "5- to 7-membered heterocyclic group" is preferable. For example, a piperidyl group (a piperidino group, a piperidin-2-yl group, a piperidin-3-yl group, a piperidin-4-yl group), a piperazinyl group (a piperazino group, a piperazin-2-yl group), and a morpholinyl group (a morpholino group, a morpholin-2-yl group, a morpholin-3-yl group) are more preferable, and a piperidino group, a piperazino group and a morpholino group are particularly preferable.
In the Formula (1), as for the "C₁₋₆ alkylsulfonamide group" of R⁸ to R¹⁰, the "C₁₋₄ alkylsulfonamide group" is preferable. A methane sulfonamide group is more preferable, for example.

In the Formula (1), as for the "C₁₋₆ alkyl group" of R¹¹, the "C₁₋₄ alkyl group" is preferable. A methyl group is more preferable, for example.

When the 1,2,3,4-tetrahydroquinoline derivatives of the present invention, more specifically the 1,2,3,4-tetrahydroquinoline derivatives represented by the Formula (1), have an asymmetric center at 2-position and 4-position of the quinoline ring, the steric configuration at 2-position and 4-position can be any one of cis configuration and trans configuration. However, cis configuration is more preferable.

The preferred R¹ to R¹¹ groups for the tetrahydroquinoline compound represented by the Formula (1) can be selected by appropriately combining any one of the R¹ to R¹¹ described above.

The 1,2,3,4-tetrahydroquinoline derivatives of the present invention, more specifically the tetrahydroquinoline compound represented by the Formula (1), may have an optical isomer. The present invention encompasses all the optical isomers and a mixture such as racemate, etc.

The 1,2,3,4-tetrahydroquinoline derivatives of the present invention, more specifically the tetrahydroquinoline compound represented by the Formula (1), salts of the compound, or solvates of the derivatives and the salts include not only the 1,2,3,4-tetrahydroquinoline derivatives of the present invention, more specifically the tetrahydroquinoline compound represented by the Formula (1), but also pharmaceutically acceptable salts of the derivatives, various hydrates, solvates, polymorphs of the derivatives and the salts, and a substance which is a prodrug thereof.
As for the pharmaceutically acceptable salts of the 1,2,3,4-tetrahydroquinoline derivatives of the present invention, more specifically the tetrahydroquinoline compound represented by the Formula (1), when the compound is processed as a basic compound, an acid addition salt and the like including a salt with an inorganic acid (for example, hydrochloric acid, hydrobromic acid, hydriodic acid, sulfuric acid, nitric acid, phosphoric acid and the like) or an organic acid (for example, formic acid, acetic acid, propionic acid, oxalic acid, malonic acid, succinic acid, fumaric acid, maleic acid, lactic acid, malic acid, tartaric acid, citric acid, methanesulfonic acid, ethanesulfonic acid, p-toluene sulfonic acid, asparaginic acid, glutamic acid and the like) can be mentioned. When the compound is processed as an acidic compound, an inorganic salt (for example, sodium salt, potassium salt, lithium salt, barium salt, calcium salt, magnesium salt and the like) or an organic salt (for example, pyridinium salt, picolinium salt, triethylammonium salt and the like) can be mentioned.
As for the solvates of the 1,2,3,4-tetrahydroquinoline derivatives of the present invention, more specifically the tetrahydroquinoline compound represented by the Formula (1), and the pharmaceutically acceptable salts thereof, hydrates or various solvates (for example, a solvate with an alcohol such as ethanol and the like) can be mentioned.

As a specific example of the compounds of the present invention, the compounds shown in Table 1 to Table 16, pharmaceutically acceptable salts thereof, or solvates of the compounds and the salts can be mentioned.

**[Table 1]**

| Compound No. | Structural formula | Name |
|---|---|---|
| 1 | 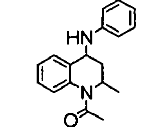 | 1-acetyl-2-methyl-4-phenylamino-1,2,3,4-tetrahydroquinoline |
| 2 | 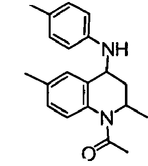 | 1-acetyl-2,6-dimethyl-4-[(4-methylphenyl)amino]-1,2,3,4-tetrahydroquinoline |
| 3 | 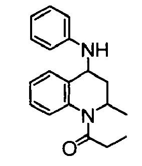 | 2-methyl-4-phenylamino-1-propionyl-1,2,3,4-tetrahydroquinoline |
| 4 | 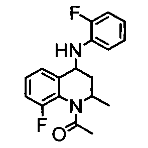 | 1-acetyl-8-fluoro-4-[(2-fluorophenyl)amino]-2-methyl-1,2,3,4-tetrahydroquinoline |
| 5 | 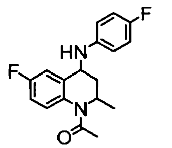 | 1-acetyl-6-fluoro-4-[(4-fluorophenyl)amino]-2-methyl-1,2,3,4-tetrahydroquinoline |
| 6 | 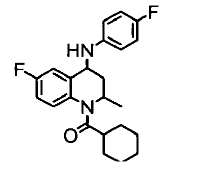 | 1-cyclohexanecarbonyl-6-fluoro-4-[(4-fluorophenyl)amino]-2-methyl-1,2,3,4-tetrahydroquinoline |
| 7 | 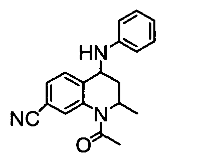 | 1-acetyl-7-cyano-2-methyl-4-phenylamino-1,2,3,4-tetrahydroquinoline |
| 8 | 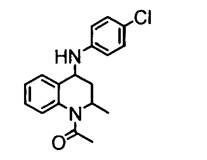 | 1-acetyl-4-[(4-chlorophenyl)amino]-2-methyl-1,2,3,4-tetrahydroquinoline |

**[Table 2]**

| Compound No. | Structural formula | Name |
|---|---|---|
| 9 | 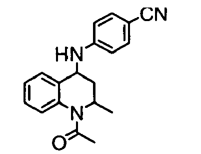 | 1-acetyl-4-[(4-cyanophenyl)amino]-2-methyl-1,2,3,4-tetrahydroquinoline |
| 10 | 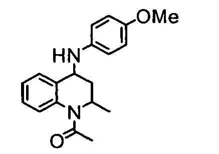 | 1-acetyl-4-[(4-methoxyphenyl)amino]-2-methyl-1,2,3,4-tetrahydroquinoline |
| 11 | 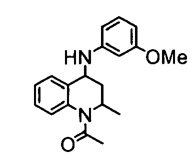 | 1-acetyl-4-[(3-methoxyphenyl)amino]-2-methyl-1,2,3,4-tetrahydroquinoline |
| 12 | 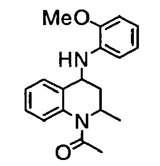 | 1-acetyl-4-[(2-methoxyphenyl) amino]-2-methyl-1,2,3,4-tetrahydroquinoline |
| 13 | 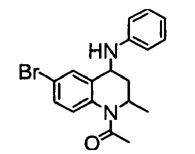 | 1-acetyl-6-bromo-2-methyl-4-phenylamino-1,2,3,4-tetrahydroquinoline |
| 14 | 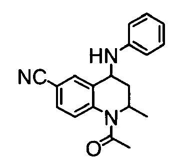 | 1-acetyl-6-cyano-2-methyl-4-phenylamino-1,2,3,4-tetrahydroquinoline |
| 15 | 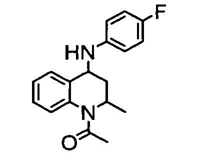 | 1-acetyl-4-[(4-fluorophenyl)amino]-2-methyl-1,2,3,4-tetrahydroquinoline |
| 16 | 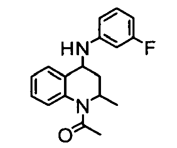 | 1-acetyl-4-[(3-fluorophenyl)amino]-2-methyl-1,2,3,4-tetrahydroquinoline |
| 17 | 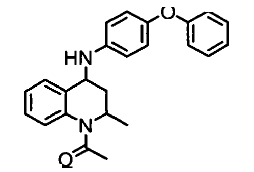 | 1-acetyl-4-[(4-phenoxyphenyl)amino]-2-methyl-1,2,3,4-tetrahydroquinoline |

**[Table 3]**

| Compound No. | Structural formula | Name |
|---|---|---|
| 18 | 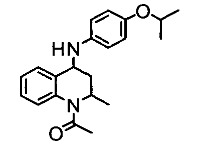 | 1-acetyl-4-[(4-isopropoxyphenyl)amino]-2-methyl-1,2,3,4-tetrahydroquinoline |
| 19 | 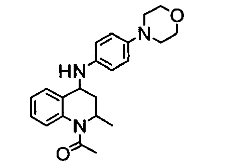 | 1-acetyl-2-methyl-4-[(4-morpholinophenyl)amino]-1,2,3,4-tetrahydroquinoline |
| 20 | 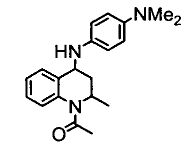 | 1-acetyl-4-[(4-N,N-dimethylaminophenyl)amino]-2-methyl-1,2,3,4-tetrahydroquinoline |
| 21 | 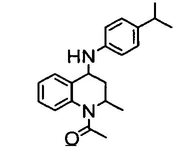 | 1-acetyl-4-[(4-isopropylphenyl)amino]-2-methyl-1,2,3,4-tetrahydroquinoline |
| 22 | 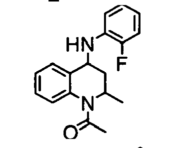 | 1-acetyl-4-[(2-fluorophenyl)amino]-2-methyl-1,2,3,4-tetrahydroquinoline |
| 23 | 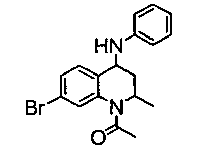 | 1-acetyl-7-bromo-2-methyl-4-phenylamino-1,2,3,4-tetrahydroquinoline |
| 24 | 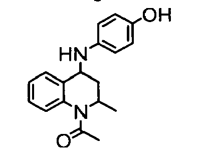 | 1-acetyl-4-[(4-hydroxyphenyl)amino]-2-methyl-1,2,3,4-tetrahydroquinoline |
| 25 | 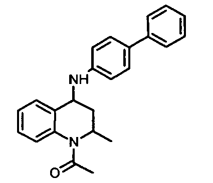 | 1-acetyl-2-methyl-4-[(1,1'-biphenyl-4-yl)amino]-1,2,3,4-tetrahydroquinoline |
| 26 | 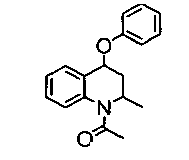 | 1-acetyl-2-methyl-4-phenoxy-1,2,3,4-tetrahydroquinoline |

**[Table 4]**

| Compound No. | Structural formula | Name |
|---|---|---|
| 27 | 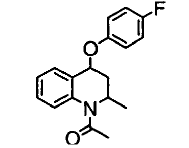 | 1-acetyl-4-(4-fluorophenoxy)-2-methyl-1,2,3,4-tetrahydroquinoline |
| 28 | 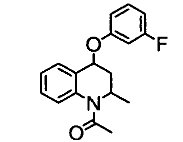 | 1-acetyl-4-(3-fluorophenoxy)-2-methyl-1,2,3,4-tetrahydroquinoline |
| 29 | 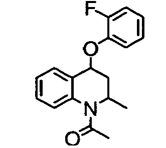 | 1-acetyl-4-(2-fluorophenoxy)-2-methyl-1,2,3,4-tetrahydroquinoline |
| 30 | 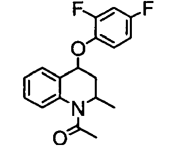 | 1-acetyl-4-(2,4-difluorophenoxy)-2-methyl-1,2,3,4-tetrahydroquinoline |
| 31 | 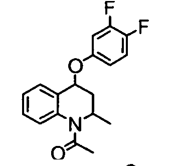 | 1-acetyl-4-(3,4-difluorophenoxy)-2-methyl-1,2,3,4-tetrahydroquinoline |
| 32 | 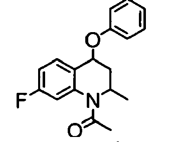 | 1-acetyl-7-fluoro-2-methyl-4-phenoxy-1,2,3,4-tetrahydroquinoline |
| 33 | 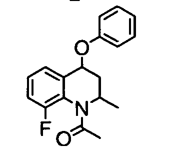 | 1-acetyl-8-fluoro-2-methyl-4-phenoxy-1,2,3,4-tetrahydroquinoline |
| 34 | 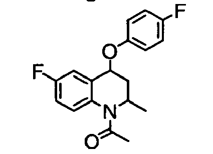 | 1-acetyl-4-(4-fluorophenoxy)-6-fluoro-2-methyl-1,2,3,4-tetrahydroquinoline |

**[Table 5]**

| Compound No. | Structural formula | Name |
|---|---|---|
| 35 | 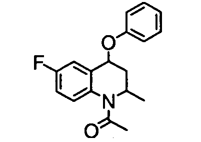 | 1-acetyl-6-fluoro-2-methyl-4-phenoxy-1,2,3,4-tetrahydroquinoline |
| 36 | 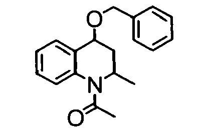 | 1-acetyl-2-methyl-4-benzyloxy-1,2,3,4-tetrahydroquinoline |
| 37 | 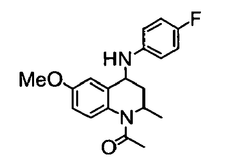 | 1-acetyl-4-[(4-fluorophenyl)amino]-2-methyl-6-methoxy-1,2,3,4-tetrahydroquinoline |
| 38 | 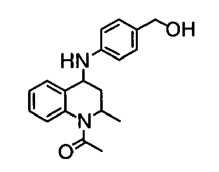 | 1-acetyl-4-[(4-hydroxymethylphenyl)amino]-2-methyl-1,2,3,4-tetrahydroquinoline |
| 39 | 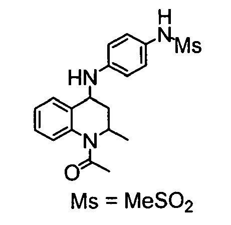 | 1-acetyl-4-[(4-methanesulfonylamidephenyl)amino]-2-methyl-1,2,3,4-tetrahydroquinoline |
| 40 | 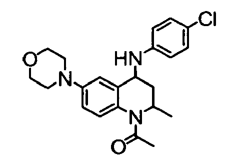 | 1-acetyl-4-[(4-chlorophenyl)amino]-2-methyl-6-morpholino-1,2,3,4-tetrahydroquinoline |
| 41 | 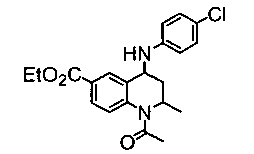 | ethyl 1-acetyl-4-[(4-chlorophenyl)amino]-2-methyl-1,2,3,4-tetrahydroquinoline-6-carboxylate |
| 42 | 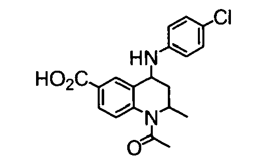 | 1-acetyl-4-[(4-chlorophenyl)amino]-2-methyl-1,2,3,4-tetrahydroquinoline-6-carboxylic acid |

**[Table 6]**

| Compound No. | Structural formula | Name |
|---|---|---|
| 43 | 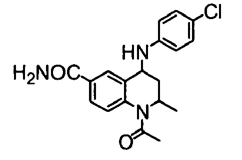 | 1-acetyl-4-[(4-chlorophenyl)amino]-2-methyl-1,2,3,4-tetrahydroquinoline-6-carboxamide |
| 44 | 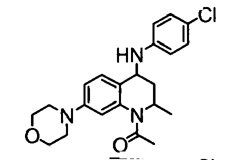 | 1-acetyl-4-[(4-chlorophenyl)amino]-2-methyl-7-morpholino-1,2,3,4-tetrahydroquinoline |
| 45 | 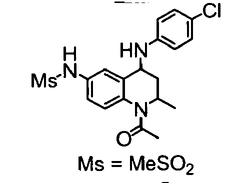 | 1-acetyl-4-[(4-chlorophenyl)amino]-2-methyl-6-methanesulfonylamino-1,2,3,4-tetrahydroquinoline |
| 46 | 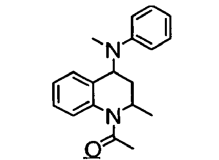 | 1-acetyl-4-(N-methyl-N-phenylamino)-2-methyl-1,2,3,4-tetrahydroquinoline |
| 47 | 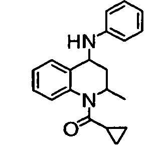 | 1-cyclopropanecarbonyl-2-methyl-4-phenylamino-1,2,3,4-tetrahydroquinoline |
| 48 | 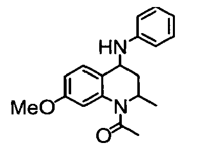 | 1-acetyl-2-methyl-7-methoxy-4-phenylamino-1,2,3,4-tetrahydroquinoline |
| 49 | 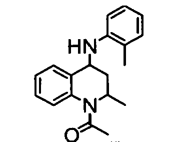 | 1-acetyl-4-[(2-methylphenyl)amino]-2-methyl-1,2,3,4-tetrahydroquinoline |
| 50 | 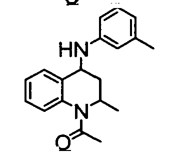 | 1-acetyl-4-[(3-methylphenyl)amino]-2-methyl-1,2,3,4-tetrahydroquinoline |
| 51 | 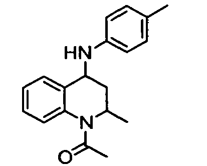 | 1-acetyl-4-[(4-methylphenyl)amino]-2-methyl-1,2,3,4-tetrahydroquinoline |

**[Table 7]**

| Compound No. | Structural formula | Name |
|---|---|---|
| 52 | 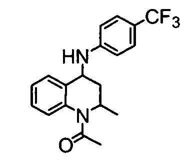 | 1-acetyl-2-methyl-4-[(4-trifluoromethylphenyl)amino]-1,2,3,4-tetrahydroquinoline |
| 53 | 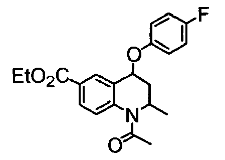 | ethyl 1-acetyl-4-(4-fluorophenoxy)-2-methyl-1,2,3,4-tetrahydroquinoline-6-carboxylate |
| 54 | 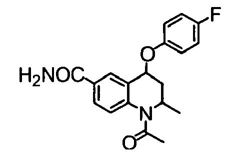 | 1-acetyl-4-(4-fluorophenoxy)-2-methyl-1,2,3,4-tetrahydroquinoline-6-carboxamide |
| 55 | 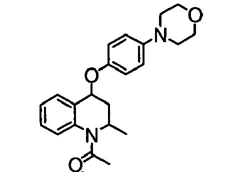 | 1-acetyl-4-(4-morpholinophenoxy)-2-methyl-1,2,3,4-tetrahydroquinoline |
| 56 | 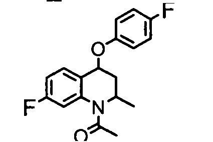 | 1-acetyl-7-fluoro-4-(4-fluorophenoxy)-2-methyl-1,2,3,4-tetrahydroquinoline |
| 57 | 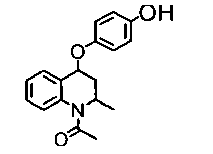 | 1-acetyl-4-(4-hydroxyphenoxy)-2-methyl-1,2,3,4-tetrahydroquinoline |
| 58 | 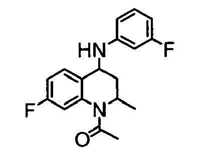 | 1-acetyl-7-fluoro-4-[(3-fluorophenyl)amino]-2-methyl-1,2,3,4-tetrahydroquin |
| 59 | 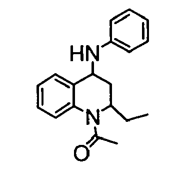 | 1-acetyl-2-ethyl-4-phenylamino-1,2,3,4-tetrahydroquinoline |

**[Table 8]**

| Compound No. | Structural formula | Name |
|---|---|---|
| 60 | 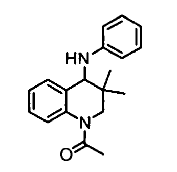 | 1-acetyl-3,3-dimethyl-4-phenylamino-1,2,3,4-tetrahydroquinoline |
| 61 | 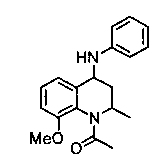 | 1-acetyl-4-phenylamino-8-methoxy-2-methyl-1,2,3,4-tetrahydroquinoline |
| 62 | 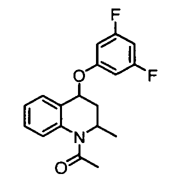 | 1-acetyl-4-(3,5-difluorophenoxy)-2-methyl-1,2,3,4-tetrahydroquinoline |
| 63 | 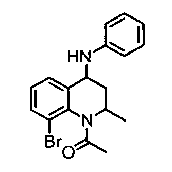 | 1-acetyl-8-bromo-4-phenylamino-2-methyl-1,2,3,4-tetrahydroquinoline |
| 64 | 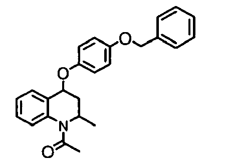 | 1-acetyl-4-(4-benzyloxyphenoxy)-2-methyl-1,2,3,4-tetrahydroquinoline |
| 65 | 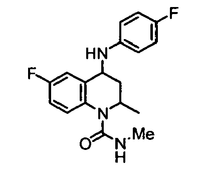 | 6-fluoro-4-[(4-fluorophenyl)amino]-2-methyl-1-N-methylcarbamoyl-1,2,3,4-tetrahydroquinoline |
| 66 | 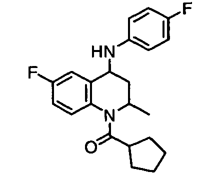 | 1-cyclopentanecarbonyl-6-fluoro-2-methyl-4-[(4-fluorophenyl)amino]-1,2,3,4-tetrahydroquinoline |
| 67 | 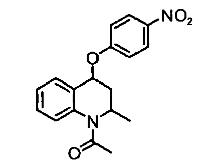 | 1-acetyl-2-methyl-4-(4-nitrophenoxy)-1,2,3,4-tetrahydroquinoline |

**[Table 9]**

| Compound No. | Structural formula | Name |
|---|---|---|
| 68 | 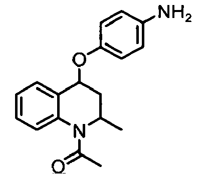 | 1-acetyl-4-(4-aminophenoxy)-2-methyl-1,2,3,4-tetrahydroquinoline |
| 69 | 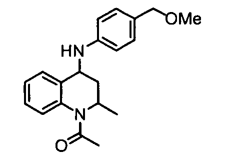 | 1-acetyl-4-[(4-methoxymethylphenyl)amino]-2-methyl-1,2,3,4-tetrahydroquinoline |
| 70 | 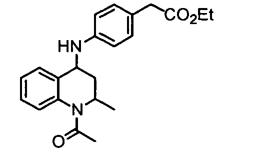 | 1-acetyl-4-[(4-ethoxycarbonylmethylphenyl)amino]-2-methyl-1,2,3,4-tetrahydroquinoline |
| 71 | 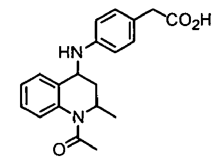 | 1-acetyl-4-[(4-carboxymethylphenyl)amino]-2-methyl-1,2,3,4-tetrahydroquinoline |
| 72 | 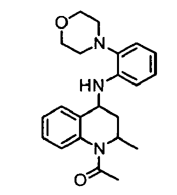 | 1-acetyl-2-methyl-4-[(2-morpholinophenyl)amino]-1,2,3,4-tetrahydroquinoline |
| 73 | 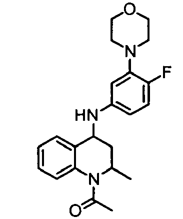 | 1-acetyl-4-[(4-fluoro-3-morpholinophenyl)amino]-2-methyl-1,2,3,4-tetrahydroquinoline |
| 74 | 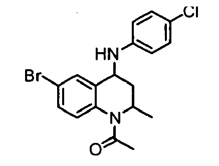 | 1-acetyl-6-bromo-4-[(4-chlorophenyl)amino]-2-methyl-1,2,3,4-tetrahydroquinoline |

**[Table 10]**

| Compound No. | Structural formula | Name |
|---|---|---|
| 75 | 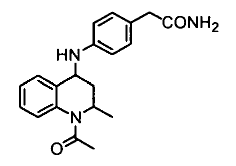 | 1-acetyl-4-[(4-carbamoylmethylphenyl)amino]-2-methyl-1,2,3,4-tetrahydroquinoline |
| 76 | 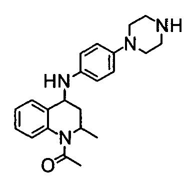 | 1-acetyl-2-methyl-4-[(4-piperazinylphenyl)amino]-1,2,3,4-tetrahydroquinoline |
| 77 | 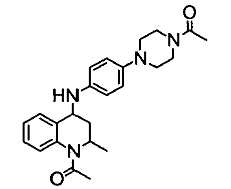 | 1-acetyl-4-{[4-(4-acetylpiperazinyl)phenyl]ammo}-2-methyl-1,2,3,4-tetrahydroquinoline |
| 78 | 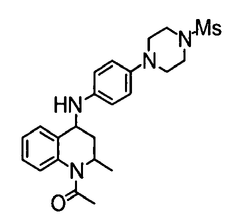 | 1-acetyl-4-{[4-(4-methanesulfonylpiperazinyl)phenyl]amino}-2-methyl-1,2,3,4-tetrahydroquinoline |
| 79 | 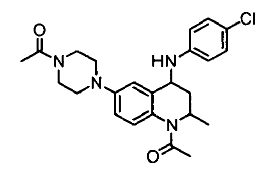 | 1-acetyl-6-[(4-acetyl)piperazino]-4-[(4-chlorophenyl)amino]-2-methyl-1,2,3,4-tetrahydroquinoline |
| 80 | 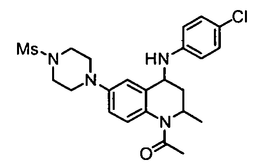 | 1-acetyl-6-[(4-methanesulfonyl)piperazino]-4-[(4-chlorophenyl)amino]-2-methyl-1,2,3,4-tetrahydroquinoline |
| 81 | 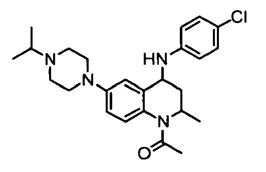 | 1-acetyl-4-[(4-chlorophenyl)amino]-6-[(4-isopropyl)piperazino]-2-methyl-1,2,3,4-tetrahydroquinoline |

**[Table 11]**

| Compound No. | Structural formula | Name |
|---|---|---|
| 82 | 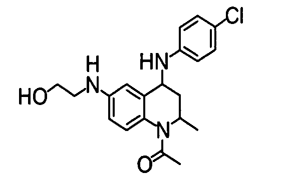 | 1-acetyl-4-[(4-chlorophenyl)amino]-6-[(2-hydroxy)ethylamino]-2-methyl-1,2,3,4-tetrahydroquinoline |
| 83 | 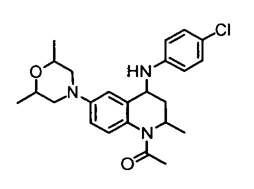 | 1-acetyl-4-[(4-chlorophenyl)amino]-6-[(cis-3,5-dimethyl)morpholino]-2-methyl-1,2,3,4-tetrahydroquinoline |
| 84 | 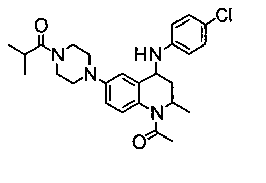 | 1-acetyl-4-[(4-chlorophenyl)amino]-6-[(4-isopropylcarbonyl)piperazino]-2-methyl-1,2,3,4-tetrahydroquinoline |
| 85 | 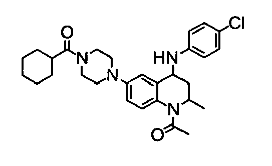 | 1-acetyl-4-[(4-chlorophenyl)amino]-6-[(4-cyclohexylcarbonyl)piperazino]-2-methyl-1,2,3,4-tetrahydroquinoline |
| 86 | 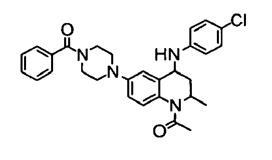 | 1-acetyl-6-[(4-benzoyl)piperazino]-2-methyl-4-[(4-chlorophenyl)amino]-1,2,3,4-tetrahydroquinoline |
| 87 | 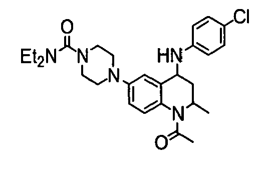 | 1-acetyl-4-[(4-chlorophenyl)amino]-6-[4-(N,N-diethylaminocarbonyl)piperazino]-2-methyl-1,2,3,4-tetrahydroquinoline |
| 88 | 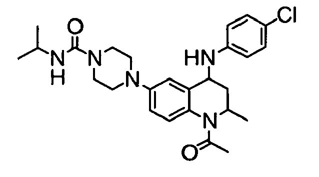 | 1-acetyl-4-[(4-chlorophenyl)amino]-6-[4-(isopropylaminocarbonyl)piperazino]-2-methyl-1,2,3,4-tetrahydroquinoline |

**[Table 12]**

| Compound No. | Structural formula | Name |
|---|---|---|
| 89 | 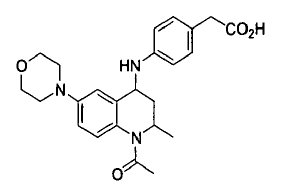 | 1-acetyl-4-[(4-carboxymethylphenyl)amino]-6-morpholino-2-methyl-1,2,3,4-tetrahydroquinoline |
| 90 | 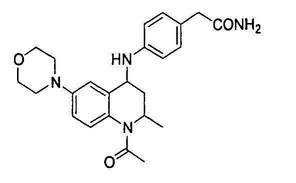 | 1-acetyl-4-[(4-carbamoylmethylphenyl)amino]-2-methyl-6-morpholino-1,2,3,4-tetrahydroquinoline |
| 91 | 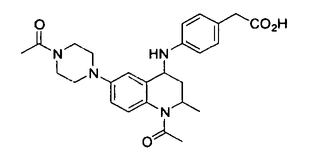 | 1-acetyl-6-(4-acetylpiperazinyl)-4-[(4-carboxymethylphenyl)amino]-2-methyl-1,2,3,4-tetrahydroquinoline |
| 92 | 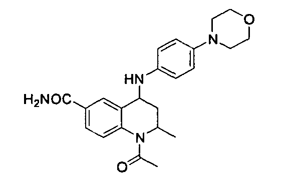 | 1-acetyl-2-methyl-4-[(4-morpholinophenyl)amino]-1,2,3,4-tetrahydroquinoline-6-carboxamide |
| 93 | 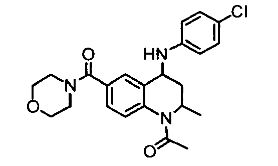 | 1-acetyl-4-[(4-chlorophenyl)amino]-2-methyl-6-[(1-morpholino)carbonyl]-1,2,3,4-tetrahydroquinoline |
| 94 | 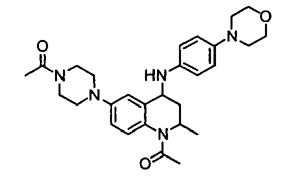 | 1-acetyl-6-[(4-acetyl)piperazino]-2-methyl-4-[(4-morpholinophenyl)amino]-1,2,3,4-tetrahydroquinoline |
| 95 | 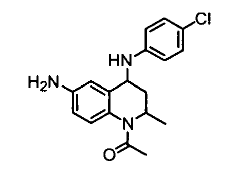 | 1-acetyl-6-amino-4-[(4-chlorophenyl)amino]-2-methyl-1,2,3,4-tetrahydroquinoline |

**[Table 13]**

| Compound No. | Structural formula | Name |
|---|---|---|
| 96 | 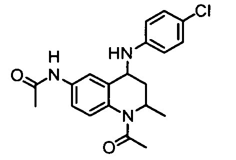 | 1-acetyl-6-acetylamino4-[(4-chlorophenyl)amino]-2-methyl-1,2,3,4-tetrahydroquinoline |
| 97 | 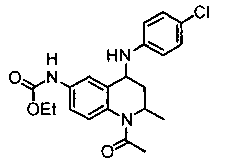 | 1-acetyl-4-[(4-chlorophenyl)amino]-2-methyl-1,2,3,4-tetrahydroquinoline-6-ethyl carbamate |
| 98 | 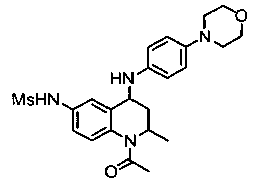 | 1-acetyl-6-methanesulfonylamino-2-methyl-4-[(4-morpholinophenyl)amino]-1,2,3,4-tetrahydroquinoline |
| 99 | 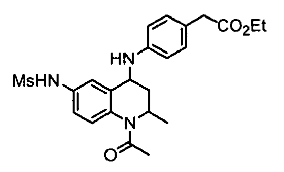 | 1-acetyl-6-methanesulfonylamino-2-methyl-4-[(4-ethoxycarbonylmethylphenyl)amino]-1,2,3,4-tetrahydroquinoline |
| 100 | 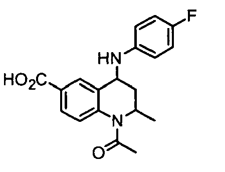 | 1-acetyl-4-[(4-fluorophenyl)amino]-2-methyl-1,2,3,4-tetrahydroquinoline-6-carboxyic acid |
| 101 | 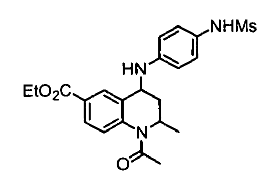 | ethyl 1-acetyl-4-[(4-methanesulfonylaminophenyl)amino]-2-methyl-1,2,3,4-tetrahydroquinoline-6-carboxylate |
| 102 | 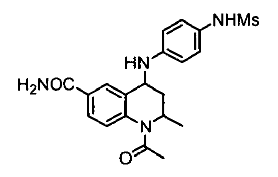 | 1-acetyl-4-[(4-methanesulfonylaminophenyl)amino]-2-methyl-1,2,3,4-tetrahydroquinoline-6-carboxamide |

**[Table 14]**

| Compound No. | Structural formula | Name |
|---|---|---|
| 103 | 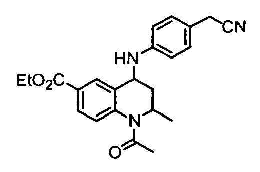 | ethyl 1-acetyl-4-[(4-cyanomethylphenyl)amino]-2-methyl-1,2,3,4-tetrahydroquinoline-6-carboxylate |
| 104 | 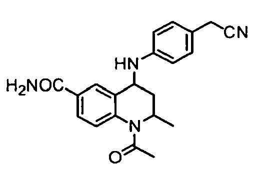 | 1-acetyl-4-[(4-cyanomethylphenyl)amino]-2-methyl-1,2,3,4-tetrahydroquinoline-6-carboxamide |
| 105 | 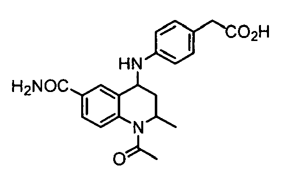 | 1-acetyl-4-[(4-carboxymethylphenyl)amino]-2-methyl-1,2,3,4-tetrahydroquinoline-6-carboxamide |
| 106 | 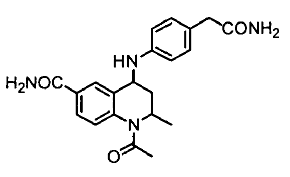 | 1-acetyl-4-[(4-carbamoylmethylphenyl)amino]-2-methyl-1,2,3,4-tetrahydroquinoline-6-carboxamide |
| 107 | 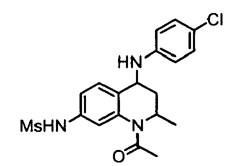 | 1-acetyl-4-[(4-chlorophenyl)amino]-7-methanesulfonylamino-2-methyl-1,2,3,4-tetrahydroquinoline |
| 108 | 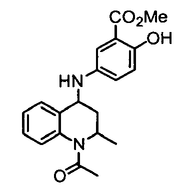 | 1-acetyl-4-[(4-hydroxy-3-methoxycarbonylphenyl)amino]-2-methyl-1,2,3,4-tetrahydroquinoline |
| 109 | 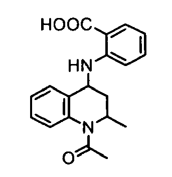 | 1-acetyl-4-[(2-carboxyphenyl)amino]-2-methyl-1,2,3,4-tetrahydroquinoline |

**[Table 15]**

| Compound No. | Structural formula | Name |
|---|---|---|
| 110 | 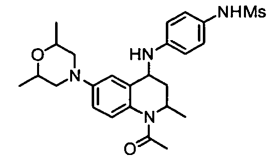 | 1-acetyl-6-[cis-2,6-dimethylmorpholin-4-yl]-4-[(4-methanesulfonylaminophenyl)amino]-2-methyl-1,2,3,4-tetrahydroquinoline |
| 111 | 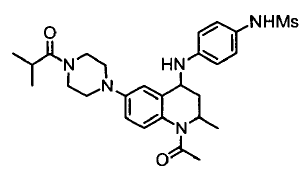 | 1-acetyl-6-[(4-isopropylcarbonyl)piperazino]-4-[(4-methanesulfonylaminophenyl)amino]-2-methyl-1,2,3,4-tetrahydroquinoline |
| 112 | 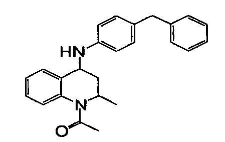 | 1-acetyl-4-[(4-benzylphenyl)amino]-2-methyl-1,2,3,4-tetrahydroquinoline |
| 113 | 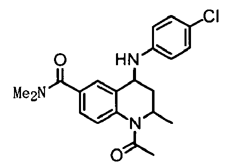 | 1-acetyl-4-[(4-chlorophenyl)amino]-N,N,2-trimethyl-1,2,3,4-tetrahydroquinoline-6-carboxamide |
| 114 | 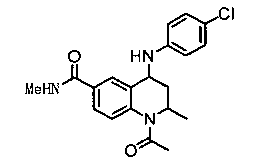 | 1-acetyl-4-[(4-chlorophenyl)amino]-N,2-dimethyl-1,2,3,4-tetrahydroquinoline-6-carboxamide |
| 115 | 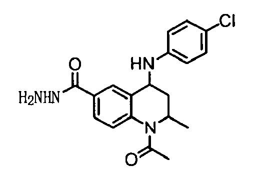 | 1-acetyl-4-[(4-chlorophenyl)amino]-2-methyl-1,2,3,4-tetrahydroquinoline-6-carbohydrazide |
| 116 | 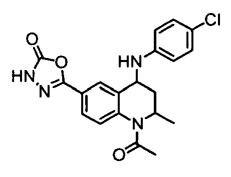 | 1-acetyl-4-[(4-chlorophenyl)amino]-2-methyl-6-[1,3,4-oxadiazol-2(3H)-on-5-yl]-1,2,3,4-tetrahydroquinoline |
| 117 | 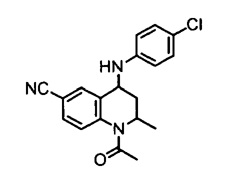 | 1-acetyl-4-[(4-chlorophenyl)amino]-6-cyano-2-methyl-1,2,3,4-tetrahydroquinoline |

**[Table 16]**

| Compound No. | Structural formula | Name |
|---|---|---|
| 118 | 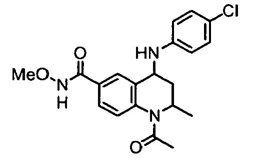 | 1-acetyl-4-[(4-chlorophenyl)amino]-N-methoxy-2-methyl-1,2,3,4-tetrahydroquinoline-6-carboxamide |
| 119 | 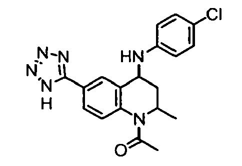 | 1-acetyl-4-[(4-chlorophenyl)amino]-2-methyl-6-(1H-tetrazol-5-yl)-1,2,3,4-tetrahydroquinoline |
| 120 | 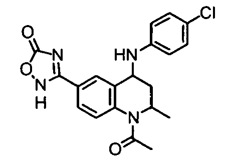 | 1-acetyl-4-[(4-chlorophenyl)amino]-2-methyl-6-[1,2,4-oxadiazol-5(2H)-on-3-yl]-1,2,3,4-tetrahydroquinoline |
| 121 | 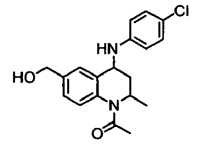 | 1-acetyl-4-[(4-chlorophenyl)amino]-6-hydroxymethyl-2-methyl-1,2,3,4-tetrahydroquinoline |
| 122 | 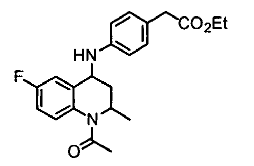 | 1-acetyl-4-[(4-ethoxycarbonylmethylphenyl)amino]-6-fluoro-2-methyl-1,2,3,4-tetrahydroquinoline |
| 123 | 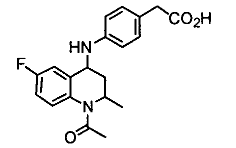 | 1-acetyl-4-[(4-carboxymethylphenyl)amino]-6-fluoro-2-methyl-1,2,3,4-tetrahydroquinoline |
| 124 | 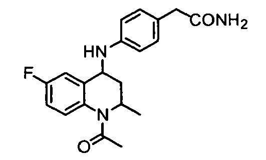 | 1-acetyl-4-[(4-carbamoylmethylphenyl)amino]-6-fluoro-2-methyl-1,2,3,4-tetrahydroquinoline |
| 125 | 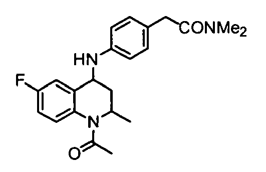 | 1-acetyl-4-[4-(N,N-dimethylaminocarbonylmethyl)phenylamino]-6-fluoro-2-methyl-1,2,3,4-tetrahydroquinoline |

Compounds 1, 2 and 3 can be obtained from ChemBridge and Compound 47 can be obtained from Princeton BioMolecular Research. Inc. Further, Compounds 5, 8, 9, 10, 11, 12, is, 16, 17, 21, 46, 48, 49, 50, 51 and 52 can be prepared according to the method described in the document (Patent Document 16) and Compounds 13 and 75 can be prepared according to the method described in the document (Patent Document 15).

The tetrahydroquinoline compound represented by the Formula (1) of the present invention can be preapred according to a known method. For example, it can be prepared according to the methods described below, or a method similar to them.

### [Preparation method 1] Preparation method for a case in which A in the Formula (1) is NH and N-alkyl

### 1-1.

According to the method shown in the following reaction scheme, Compound [D] of the present invention can be prepared.

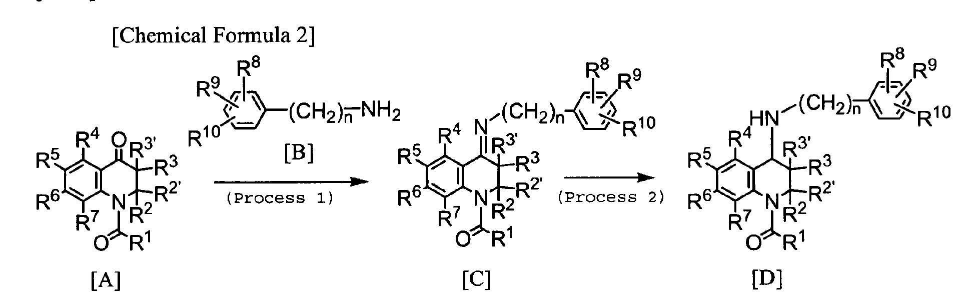

[wherein, R¹, R², R^{2'}, R³, R^{3'}, R⁴, R⁵, R⁶, R⁷, R⁸, R⁹ and R¹⁰ represent the Groups that are each as defined in the above.]
Compound [D] can be prepared by reductive amination of 4-oxo-1,2,3,4-tetrahydroquinoline represented by Formula [A]. Introduction of an amino group based on the reductive amination can be carried out with reference to Comprehensive Organic Synthesis, 1991, Vol. 8, page 21, etc., for example.
(Process 1) Compound [C] can be prepared by reacting Compound [A] with Compound [B] in a solvent under cooling to heating for 5 minutes to 40 hrs (preferably 1 to 18 hrs) in the presence of an acid. As for the acid, titanium tetrachloride, p-toluene sulfonic acid, trifluoroacetic acid and the like can be mentioned. As for the solvent, an organic solvent such as toluene, dichloromethane, benzene, tetrahydrofuran and the like can be used alone or in a combination thereof. (Process 2) Compound [D] can be prepared by reacting Compound [C] in a solvent under cooling to heating for 5 minutes to 40 hrs (preferably 1 to 18 hrs) in the presence of a reducing agent. As for a reducing method, a catalytic reduction with hydrogen gas using a metal catalyst such as palladium carbon, palladium black, palladium hydroxide, platinum oxide, Raney nickel and the like, or a method using sodium borohydride, sodium cyanoborohydride, sodium triacetoxyborohydride, zinc borohydride, borane, aluminum hydride, aluminum diisobutyl hydride, sodium-alcohol and the like can be mentioned. As for the solvent, an organic solvent such as methanol, ethanol, N,N-dimethylformamide, diethyl ether, 1,4-dioxane, tetrahydrofuran, acetic acid, ethyl acetate and the like, water or a mixture solvent thereof can be used alone or in a combination thereof.
Further, in addition to the method in which Process 1 and Process 2 described above are performed in order, Compound [D] can be also prepared from Compound [A] according to a method in which Process 1 and Process 2 are performed simultaneously in a single system.
Further, a compound in which A is N-alkyl can be prepared from Compound [D] according to a known method, for example, a reductive amination (Borch reaction [J. Amer. Chem. Soc., 2897 (1971)], Leuckart-Wallach reaction [Org. React., 301 (1949)], Eshweiler-Clarke reaction [J. Amer. Chem. Soc., 4571 (1933)]) or an alkylation of an amino group.

### 1-2.

4-Oxo-1,2,3,4-tetrahydroquinoline derivative [A], used for the preparation of Compound [D] of the present invention, can be prepared according to the following preparation method in known order, for example, in view of the pamphlet of WO2002/53557, when R^{3'} is a hydrogen atom.

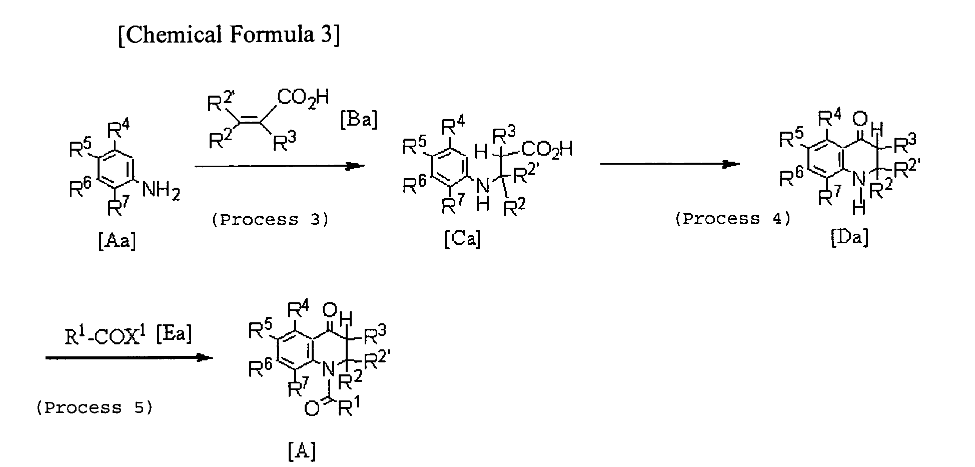

[wherein, R¹, R², R^{2'}, R³, R^{3'}, R⁴, R⁵, R⁶ and R⁷ represent the groups that are each as defined in the above and X¹ represents a leaving group.]
(Process 3) Compound [Ca] can be prepared by reacting Compound [Aa] with Compound [Ba] in a solvent under heating for 5 minutes to 40 hrs (preferably 1 to 18 hrs). As for the solvent, an organic solvent such as toluene, benzene, ethyl acetate, methylisobutyl ketone, methyl-tert-butyl ether and the like can be used alone or in a combination thereof.
(Process 4) Compound [Da] (R^{3'} is a hydrogen atom) can be prepared by reacting Compound [Ca] under heating for 5 minutes to 40 hrs (preferably 1 to 18 hrs) in the presence of an acid such as polyphosphoric acid and the like. As for the solvent, an organic solvent such as toluene, benzene and the like can be used alone or in a combination thereof.
(Process 5) Compound [A] (R^{3'} is a hydrogen atom) can be prepared by reacting Compound [Da] (R^{3'} is a hydrogen atom) with Compound [Ea] in a solvent under cooling to heating for 5 minutes to 40 hrs (preferably 1 to 18 hrs) in the presence or the absence of a base. As for the base, pyridine, triethylamine, N,N-diisopropylethylamine, N-methylmorpholine, N-methylpiperidine, picoline and the like can be mentioned. As for the solvent, an organic solvent such as dichloromethane, chloroform, toluene, diethyl ether, tetrahydrofuran, 1,4-dioxane, diisopropyl ether, dimethoxyethane, hexane, ethyl acetate, methyl-tert-butyl ether, N,N-dimethylformamide and the like, water or a mixture solvent thereof can be used alone or in a combination thereof. Herein, X¹ in Compound [Ea] represents an atom or a functional group which acts as a leaving group, and examples thereof include a halogen atom such as chlorine, bromine and the like or an acyl group such as pyvaloyl group and the like.

### 1-3.

4-Oxo-1,2,3,4-tetrahydroquinoline derivative [A], used for the preparation of Compound [C] of the present invention, can also be prepared according to the following production method in known order for example, in view of the pamphlet of WO2002/79165.

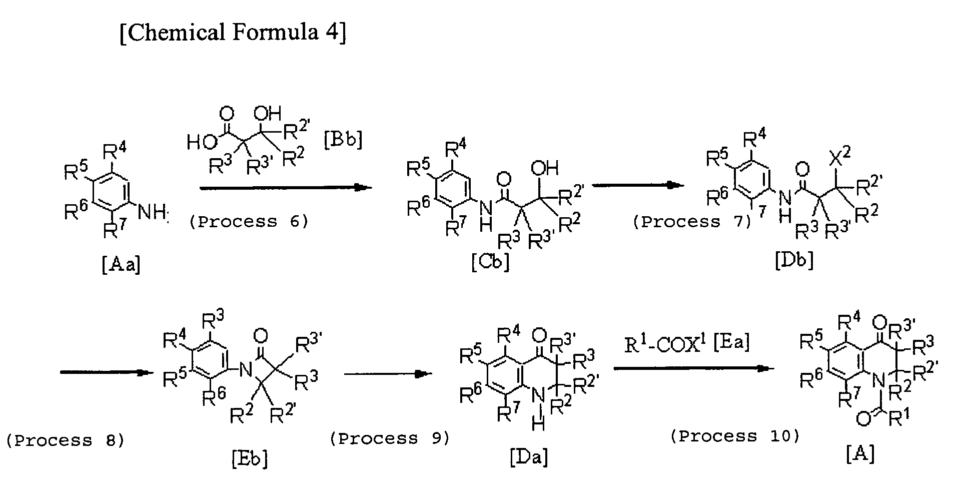

[wherein, R¹, R², R^{2'}, R³, R^{3'}, R⁴, R⁵, R⁶ and R⁷ represent the groups that are each as defined in the above and X¹ and X² represent a leaving group.]
(Process 6) Compound [Cb] can be obtained by reacting Compound [Aa] with Compound [Bb] in an equivalent amount or an excess amount, in a solvent under cooling to heating for 5 minutes to 40 hrs (preferably 1 to 18 hrs) in the presence or the absence of a base. In this case, as a reaction reagent, 1,3-dicyclohexylcarbodiimide (DCC), 1-ethyl-3-(3-dimethylaminopropyl)carbodiimide hydrochloride (WSC), oxalyl chloride, thionyl chloride and the like can be used. As for the base, an organic base such as pyridine, triethylamine, N,N-diisopropylethylamine and the like and an inorganic base such as potassium carbonate, sodium carbonate, potassium hydroxide, sodium hydroxide, lithium hydroxide and the like can be mentioned. As for the solvent, an organic solvent such as dichloromethane, chloroform, toluene, diethyl ether, tetrahydrofuran, 1,4-dioxane, diisopropyl ether, dimethoxyethane, hexane, ethyl acetate, methyl-tert-butyl ether, N,N-dimethylformamide and the like or water can be used alone or in a combination thereof.
(Process 7) Compound [Db] can be prepared by reacting Compound [Cb] with alkylsulfonyl halide, arylsulfonyl halide, alkylsulfonic anhydride, or arylsulfonic anhydride and the like in a solvent under cooling to heating for 5 minutes to 40 hrs (preferably 5 minutes to 18 hrs) in the presence of a base. As for the alkylsulfonyl halide, methanesulfonyl chloride, trifluoromethanesulfonyl chloride and the like can be mentioned. As for the arylsulfonyl halide, toluene sulfonyl chloride and the like can be mentioned. As for the alkylsulfonic anhydride, methanesulfonic anhydride, trifluoromethanesulfonic anhydride and the like can be mentioned. As for the arylsulfonic anhydride, toluene sulfonic anhydride and the like can be mentioned. As for the base, pyridine, triethylamine, N,N-diisopropylethylamine, N-methylmorpholine, N-methylpiperidine, picoline and the like can be mentioned. In addition, as for the solvent, an organic solvent such as dichloromethane, chloroform, toluene, diethyl ether, tetrahydrofuran, 1,4-dioxane, diisopropyl ether, dimethoxyethane, hexane, ethyl acetate, methyl-tert-butyl ether, N,N-dimethylformamide and the like or water can be used alone or in a combination thereof.

(Process 8) Compound [Eb] can be preapred by reacting Compound [Db] under cooling to heating for 5 minutes to 40 hrs (preferably 1 to 18 hrs) in the presence of a base. As for the base, sodium hydride, potassium hydride, sodium hydroxide, potassium hydroxide, lithium hydroxide and the like can be mentioned. Preferably, it is sodium hydroxide. As for the solvent, an organic solvent such as dichloromethane, chloroform, toluene, diethyl ether, tetrahydrofuran, 1,4-dioxane, diisopropyl ether, dimethoxyethane, hexane, ethyl acetate, methyl-tert-butyl ether, N,N-dimethylformamide and the like can be used alone or in a combination thereof. Preferably, it is N,N-dimethylformamide.
(Process 9) Compound [Da] can be prepared by reacting Compound [Eb] in a solvent under cooling to heating for 5 minutes to 40 hrs (preferably 1 to 18 hrs) in the presence of an acid such as trifluoromethanesulfonic acid and the like. As for the solvent, an organic solvent such as toluene, dichloromethane, benzene, tetrahydrofuran and the like can be used alone or in a combination thereof.
(Process 10) Compound [A] can be prepared from Compound [Da] according to the method described in Preparation method 1-2 (Process 5).

### 1-4.

Further, 4-oxo-1,2,3,4-tetrahydroquinoline derivative [A] in which R^{2'} and R^{3'} are a hydrogen atom and R² is -CH₂-R^{3"} can also be prepared according to the following preparation method in known order for example, in view of the descriptions in Journal of the Chemical Society. Perkin Transactions 1: Organic and Bio-Organic Chemistry, 1994, Vol. 59, page 9-13.

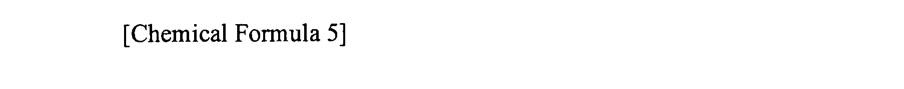

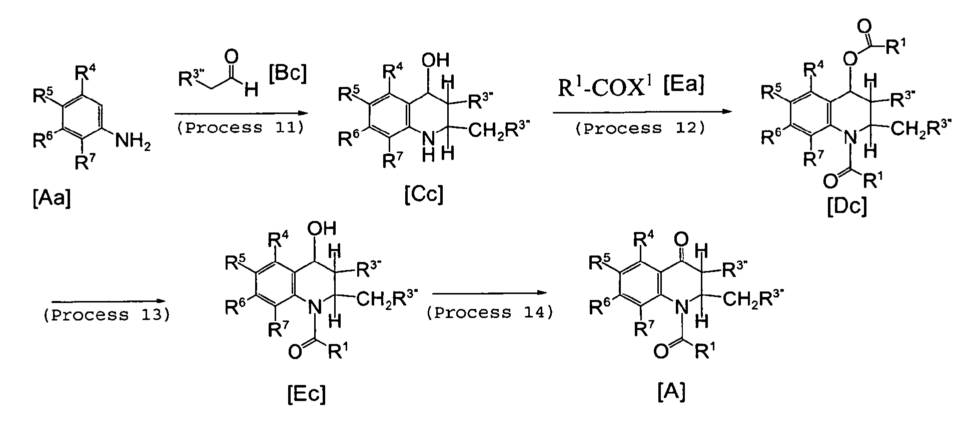

[wherein, R¹, R⁴, R⁵, R⁶ and R⁷ represent the groups that are each as defined in the above, R^{3"}is a hydrogen atom, an alkyl group, a cycloalkyl group, or a 5- to 10-membered heterocyclic group and X¹ represents a leaving group.]
(Process 11) Compound [Cc] can be prepared by reacting Compound [Aa] with Compound [Bc] (2 to 10 equivalents, preferably 3 to 4 equivalents) in a solvent under cooling to heating for 5 minutes to 40 hrs (preferably 1 to 18 hrs) in the presence of an acid. As for the acid, an inorganic acid such as hydrochloric acid, hydrobromic acid, sulfuric acid, phosphoric acid, nitric acid and the like, and an organic acid such as acetic acid, oxalic acid, citric acid, tartaric acid, maleic acid, benzoic acid and the like can be mentioned. As for the solvent, an organic solvent such as dichloromethane, chloroform, toluene, diethyl ether, tetrahydrofuran, 1,4-dioxane, diisopropyl ether, dimethoxyethane, hexane, ethyl acetate, methyl-tert-butyl ether, N,N-dimethylformamide and the like or water can be used alone or in a combination thereof.
(Process 12) Compound [Dc] can be prepared by reacting Compound [Cc] with Compound [Ea] in a solvent under cooling to heating for 5 minutes to 40 hrs (preferably 1 to 18 hrs) in the presence or the absence of a base. As for the base, pyridine, triethylamine, N,N-diisopropylethylamine, N-methylmorpholine, N-methylpiperidine, picoline and the like can be mentioned. As for the solvent, an organic solvent such as dichloromethane, chloroform, toluene, diethyl ether, tetrahydrofuran, 1,4-dioxane, diisopropyl ether, dimethoxyethane, hexane, ethyl acetate, methyl-tert-butyl ether, N,N-dimethylformamide and the like, water or a mixture solvent thereof can be used alone or in a combination thereof. Herein, X¹ in Compound [Ea] represents an atom or a functional group which acts as a leaving group, and examples thereof include a halogen atom such as fluorine, chlorine, bromine and the like or an acyl group such as pyvaloyl group and the like.

(Process 13) Compound [Ec] can be prepared from Compound [Dc] based on hydrolysis using a hydroxide ion or alcoholysis using an alkoxide. In this case, as for the base, potassium carbonate, sodium carbonate, potassium hydroxide, sodium hydroxide, lithium hydroxide and the like can be used. As for the solvent, water or an organic solvent such as methanol, ethanol, isopropyl alcohol, tetrahydrofuran, 1,4-dioxane, dimethoxyethane and the like can be used alone or in a combination with water. The reaction temperature and reaction time can be under cooling to heating for 5 minutes to 40 hrs (preferably 1 to 18 hrs).
(Process 14) Compound [A] (i.e., a compound in which R^{2'} and R^{3'} are a hydrogen atom and R² is -CH₂-R^{3"}) can be obtained by reacting Compound [Ec] with an oxidizing agent in a solvent under cooling to heating for 5 minutes to 40 hrs (preferably 1 to 18 hrs). As for the oxidizing agent, dimethyl sulfoxide-oxalyl chloride (or acetic anhydride, trifluoroacetic anhydride, DCC and the like)-triethylamine, hydrogen peroxide, tetraisopropylammonium perruthenate, manganese dioxide, pyridinium chlorochromate (PCC), pyridinium dichromate (PDC), potassium dichromate, potassium permangante and the like can be mentioned. As for the solvent, an organic solvent such as dichloromethane, chloroform, toluene, diethyl ether, tetrahydrofuran, 1,4-dioxane, diisopropyl ether, dimethoxyethane, hexane, ethyl acetate, methyl-tert-butyl ether, N,N-dimethylformamide and the like, water or a mixture solvent thereof can be used alone or in a combination thereof.

### 1-5.

Further, Compound [D], i.e., a tetrahydroquinoline compound in which R^{2'} and R^{3'} are a hydrogen atom, R² is -CH₂-R^{3"} and n is 0, can also be prepared according to the following production method in known order, i.e., double neutralization of Compound [Aa], for example, in view of the descriptions of JP-A No. 2002-53557.

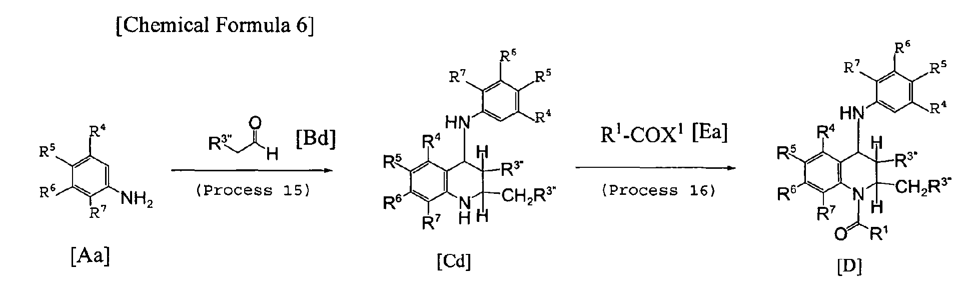

[wherein, R¹, R⁴, R⁵, R⁶ and R⁷ represent the groups that are each as defined in the above and , R^{3"} is a hydrogen atom, an alkyl group, a cycloalkyl group, or a 5- to 10-membered heterocyclic group and X¹ represents a leaving group.]
(Process 15) Compound [Cd] can be prepared by reacting Compound [Aa] with Compound [Bd] in a solvent under cooling to heating for 5 minutes to 4 days (preferably 1 hr to 3 days) in the presence of benztriazole.
(Process 16) Compound [D] can be prepared by reacting Compound [Cd] with Compound [Ea] in a solvent under cooling to heating for 5 minutes to 40 hrs (preferably 1 to 18 hrs) in the presence or the absence of a base. As for the base, pyridine, triethylamine, N,N-diisopropylethylamine, N-methylmorpholine, N-methylpiperidine, picoline and the like can be mentioned. As for the solvent, an organic solvent such as dichloromethane, chloroform, toluene, diethyl ether, tetrahydrofuran, 1,4-dioxane, diisopropyl ether, dimethoxyethane, hexane, ethyl acetate, methyl-tert-butyl ether, N,N-dimethylformamide and the like, water or a mixture solvent thereof can be used alone or in a combination thereof. Herein, X¹ in Compound [Ea] represents an atom or a functional group which acts as a leaving group, and examples thereof include a halogen atom such as fluorine, chlorine, bromine and the like or an acyl group such as pyvaloyl group and the like.

### [Preparation method 2] Preparation method for a case in which A in the Formula (1) is an oxygen atom

### 2-1.

Compound [G] of the present invention can be prepared from 4-hydroxy-1,2,3,4-tetrahydroquinoline represented by Formula [F].

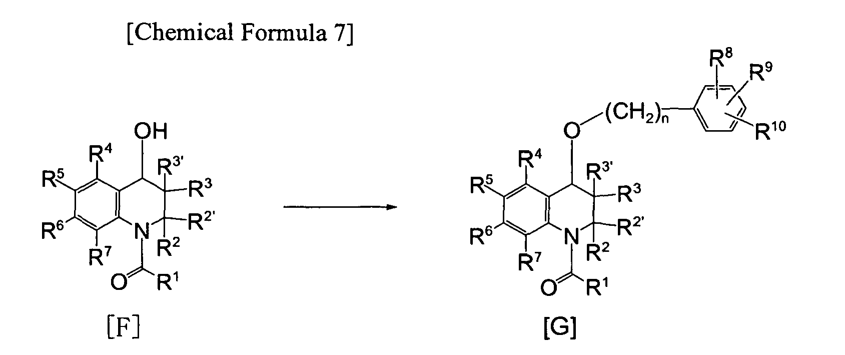

[wherein, R¹, R⁴, R⁵, R⁶ and R⁷ represent the groups that are each as defined in the above, R^{3'} is an alkyl group, a cycloalkyl group, or a 5- to 10-membered heterocyclic group and X¹ represents a leaving group.]
When n is 0, Compound [G] of the present invention can be prepared from 4-hydroxy-1,2,3,4-tetrahydroquinoline represented by Formula [F] based on Mitsunobu reaction or a radical substitution reaction.

### 2-1-1. Mitsunobu reaction

Compound [G] can be prepared from Compound [F] and phenols indicated by ArOH based on Mitsunobu reaction (exemplary reference document: Organic Reactions, 42, 2, 335-395 or Synthesis, 1981, 1-28). Specifically, Compound [B] can be obtained by reacting Compound [A] and 0.5 to 5 equivalents (preferably 1 to 1.5 equivalents) of ArOH in a solvent under cooling to heating for 5 minutes to 40 hrs (preferably 1 to 18 hrs) in the presence of 0.5 to 5 equivalents (preferably 1 to 1.5 equivalents) of azodicarboxylic acid derivatives and triaryl phosphine or trialkyl phosphine. As for the azodicarboxylic acid derivatives, dimethyl azodicarboxylate, ethyl azodicarboxylate, diisopropyl azodicarboxylate, 1,1-azodicarbonyldipiperidine and the like can be mentioned. As for the phosphines, triphenylphosphine, tributylphosphine and the like can be mentioned. As for the solvent, an organic solvent such as dichloromethane, chloroform, toluene, ether, tetrahydrofuran, 1,4-dioxane, diisopropyl ether, dimethoxyethane, hexane, ethyl acetate, methyl-tert-butyl ether, N,N-dimethylformamide and the like can be used alone or in a combination thereof.

### 2-1-2. Radical substitution reaction

Compound [G] can be synthesized from Compound [F] and an aryl radical source based on radical substitution reaction (exemplary reference document: Chemical Reviews, 1989, 89, 1487-1501). Specifically, it can be obtained by reacting Compound [F] and 1 to 10 equivalents (preferably 1 to 2 equivalents) of an aryl radical source like tri(aryl) bismuth diacetate and the like with or without a solvent under cooling to heating for 5 minutes to 40 hrs (preferably 1 to 18 hrs) in the presence of a metal salt such as copper acetate and the like. As for the solvent, tetrahydrofuran, dioxane, chloroform, dichloromethane and the like can be used alone or in a combination thereof.

### 2-1-3.

When n is 1, Compound [G] of the present invention can be prepared from 4-hydroxy-1,2,3,4-tetrahydroquinoline represented by Formula [F] based on alkylation reaction. Specifically, it can be obtained by reacting Compound [F] and 1 to 10 equivalents (preferably 1 to 2 equivalents) of a base and 1 to 10 equivalents (preferably 1 to 2 equivalents) of an alkylating agent in a solvent under cooling to heating for 5 minutes to 40 hrs (preferably 1 to 18 hrs). As for the base, sodium hydride, an alkyl lithium such as n-butyl lithium and the like, a Grignard reagent such as phenyl magnesium bromide and the like, and an amide base such as lithium N,N-diisopropylamide, potassium hexamethyldisilazide and the like can be mentioned. As for the solvent, tetrahydrofuran, 1,4-dioxane, dimethoxyethane and the like can be used alone or in a combination thereof.

### 2-2.

Herein, when R^{3'} is a hydrogen atom, 4-hydroxy-1,2,3,4-tetrahydroquinoline, that is indicated by Formula [F], can be prepared according to the following production method in known order for example, in view of the pamphlet of W02002/053557, etc.

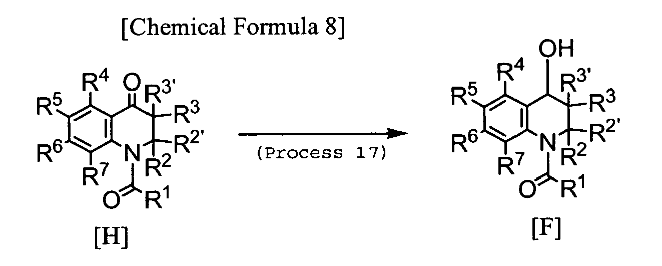

(Process 17) Compound [F] can be synthesized by reacting Compound [H] in a solvent under cooling to heating for 5 minutes to 40 hrs (preferably 1 to 18 hrs) in the presence of a reducing agent. As for a reducing method, a catalytic reduction with hydrogen gas using a metal catalyst such as palladium carbon, palladium black, palladium hydroxide, platinum oxide, Raney nickel and the like, or a method using sodium borohydride, sodium cyanoborohydride, sodium triacetoxyborohydride, zinc borohydride, borane, aluminum hydride, aluminum diisobutyl hydride, sodium-alcohol and the like can be mentioned. As for the solvent, an organic solvent such as methanol, ethanol, N,N-dimethylformamide, diethyl ether, 1,4-dioxane, tetrahydrofuran, acetic acid, ethyl acetate and the like, water or a mixture solvent thereof can be used alone or in a combination thereof.

The EPO production potentiator, hemoglobin expression potentiator or the therapeutic agent for anemia of the present invention comprises as an effective component 1-acyl-4-(phenoxy, benzyloxy, or phenylamino)-1,2,3,4-tetrahydroquinoline derivatives, more specifically the tetrahydroquinoline compound represented by the Formula (1), salts of the tetrahydroquinoline derivatives, or solvates of the tetrahydroquinoline derivatives and the salts, and they can be used as a pharmaceutical composition. For such case, the compound of the present invention can be used alone. But it is generally used in combination with a pharmaceutically acceptable carrier and/or a diluent.

Administration route is not specifically limited, and it can be appropriately selected depending on therapeutic purpose. For example, any one of an oral preparation, an injection solution, a suppository preparation, an inhaling agent and the like can be used. The pharmaceutical composition which is suitable for such administration form can be prepared by using a formulation method known in the art.

In preparing a solid preparation for oral administration, a pharmaceutically acceptable excipient and, if necessary, a binder, a disintegrating agent, a lubricant, a coloring agent, a corrigent for taste and smell, etc. are added to the 1-acyl-4-(phenoxy, benzyloxy, or phenylamino)-1,2,3,4-tetrahydroquinoline derivatives, more specifically the compound represented by the Formula (1) above, followed by subjecting to a common method whereupon tablets, coated tablets, granules, powders, capsules and the like are able to be manufactured. With regard to such additive, those which have been commonly used in the related field may be used and, for example, lactose, white sugar, sodium chloride, glucose, starch, calcium carbonate, kaolin, microcrystalline cellulose, silicic acid, and the like may be exemplified as an excipient. Water, ethanol, propanol, simple syrup, glucose solution, starch solution, gelatin solution, carboxymethyl cellulose, hydroxypropyl cellulose, hydroxypropyl starch, methyl cellulose, ethyl cellulose, shellac, calcium phosphate, polyvinylpyrrolidone, and the like may be exemplified as a binder. Dry starch, sodium alginate, agar powder, sodium hydrogen carbonate, calcium carbonate, sodium laurylsulfate, stearic acid monoglyceride, lactose and the like may be exemplified as a disintegrating agent. Pure talc, stearate, borax, polyethylene glycol and the like may be exemplified as a lubricant. White sugar, dried orange peel, citric acid, tartaric acid and the like may be exemplified as a corrigent for taste.

In preparing a liquid preparation for oral administration, a corrigent for taste, a buffering agent, a stabilizer, a corrigent for smell and the like are added to the 1-acyl-4-(phenoxy, benzyloxy, or phenylamino)-1,2,3,4-tetrahydroquinoline derivatives, more specifically the compound represented by the Formula (1) above, followed by subjecting to a common method whereupon a liquid preparation for oral use, syrup, elixir and the like are able to be manufactured. In this case, those described above may be exemplified as a corrigent for taste. Sodium citrate and the like may be exemplified as buffers, and tragacanth, gum arabic, gelatin and the like may be exemplified as a stabilizer.

In preparing an injection preparation, a pH adjusting agent, a buffering agent, a stabilizer, an isotonicity agent, topical anesthetics and the like are added to the 1-acyl-4-(phenoxy, benzyloxy, or phenylamino)-1,2,3,4-tetrahydroquinoline derivatives, more specifically the compound represented by the Formula (1) above, followed by subjecting to a common method whereupon subcutaneous, intramuscular and intravenous injection preparations are able to be manufactured. In this case, sodium citrate, sodium acetate, sodium phosphate and the like may be exemplified as a pH adjusting agent and a buffering agent. Sodium pyrosulfite, EDTA, thioglycolate, thiolactic acid and the like may be exemplified as a stabilizer. Procaine hydrochloride, lidocaine hydrochloride and the like may be exemplified as topical anesthetics. Further, sodium chloride, glucose, and the like may be exemplified as an isotonicity agent.
In preparing a suppository preparation, a well-known carrier for suppository preparation, for example, polyethylene glycol, lanolin, cacao butter, fatty acid triglyceride and the like and, if necessary, a surface active agent (for example Tween (registered trademark)) and the like can be added to the 1-acyl-4-(phenoxy, benzyloxy, or phenylamino)-1,2,3,4-tetrahydroquinoline derivatives, more specifically the compound represented by the Formula (1) above, followed by subjecting to a common method whereupon a suppository preparation is able to be manufactured.

By using a common method other than those described above, a desired preparation can be also appropriately prepared.

Although the dose of the 1-acyl-4-(phenoxy, benzyloxy, or phenylamino)-1,2,3,4-tetrahydroquinoline derivatives of the present invention, more specifically the compound represented by the Formula (1) above, may vary depending upon age, body weight and symptom of a patient, dosage form, frequency of administration and the like, it is usually preferred to administer 1 mg to 1000 mg per day of the compound of Formula (1) to an adult once daily or by dividing into several times a day either orally or parenterally.

Descriptions of Japanese Patent Application No. 2007-252727, which is the basic application of the present application, are incorporated herein by reference in their entirety.
The present invention will now be illustrated in detail by way of the following Examples and Test examples. However, the present invention is not limited thereto.

### Example 1

Preparation of cis-1-acetyl-8-fluoro-4-[(2-fluorophenyl)amino]-2-methyl-1,2,3,4-tetrahydroquinoline (Compound 4)

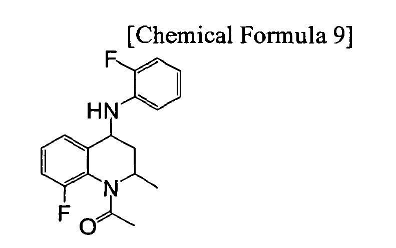

[Process 1] 2-Fluoroaniline (1 g), acetaldehyde (484 mg) and benzotriazole (238 mg) were dissolved in ethanol and stirred for 14 hrs at room temperature. Upon completion of the reaction, the reaction mixture was concentrated under reduced pressure. The resulting residue was purified by silica gel chromatography (diethyl ether:hexane = 1:5) to obtain 8-fluoro-4-[(2-fluorophenyl)amino]-2-methyl-1,2,3,4-tetrahydroquinoline as a yellow oil (163 mg, 13%).
[Process 2] Cis-8-fluoro-4-[(2-fluorophenyl)amino]-2-methyl-1,2,3,4-tetrahydroquinoline (60 mg) was dissolved in pyridine (0.5 mL) and dichloromethane (2 mL), added with acetyl chloride (16 mg) under ice cooling, and stirred for 2 hrs. Upon completion of the reaction, water was added to the reaction mixture and extracted with chloroform. The organic layer was washed with saturated sodium hydrogen carbonate solution and saturated brine, dried over anhydrous sodium sulfate, and concentrated under reduced pressure. The resulting residue was purified by silica gel chromatography (chloroform) to obtain the title compound as a colorless oil (48 mg, 70%).

¹H-NMR(400MHz), CDCl₃) δ:
1.16 (3H, d, J = 6.6 Hz), 1.25-1.33 (1H, m), 2.11 (3H, s), 2.68 (1H, ddd, J = 4.0, 8.4, 12.4 Hz), 4.10 (1H, brs), 4.19-4.21 (1H, m), 4.98 (1H, brs), 6.58-6.72 (2H, m), 6.82-6.86 (1H, m), 7.01-7.07 (1H, m), 6.91-7.11 (2H, m), 7.17-7.22 (1H, m).

### Example 2

Preparation of cis-1-acetyl-4-[(2-fluorophenyl)amino]-2-methyl-1,2,3,4-tetrahydroquinoline (Compound 22)

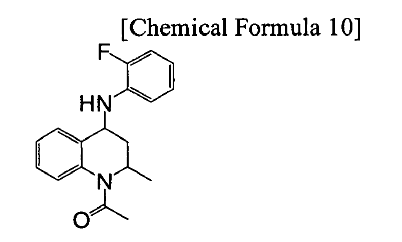

[Process 1] 1-Acetyl-4-oxo-2-methyl-1,2,3,4-tetrahydroquinoline (50 mg) and 2-fluoroaniline (83 mg) were dissolved in toluene (2 mL). Under ice cooling, titanium tetrachloride (1.0 M dichloromethane solution, 125 µL) was added thereto, stirred for 1 hr, and further stirred for 3 hrs at 80°C. Upon completion of the reaction, the reaction mixture was filtered through Celite, and then concentrated under reduced pressure. The resulting residue was purified by silica gel chromatography (diethyl ether:hexane = 2:1) to obtain 1-acetyl-2-methyl-4-[(2-fluorophenyl)imino]-1,2,3,4-tetrahydroquinoline as a yellow oil (60 mg, 81 %).
[Process 2] 1-Acetyl-2-methyl-4-[(2-fluorophenyl)imino]-1,2,3,4-tetrahydroquinoline (60 mg) and sodium cyanoborohydride (28 mg) were dissolved in methanol (2 mL) and stirred for 18 hrs at 50°C. Upon completion of the reaction, 1 N hydrochloric acid was added to the reaction solution, which was then stirred for 30 minutes. After neutralizing with a saturated aqueous solution of sodium hydrogen carbonate, the solution was extracted with chloroform. The organic layer was washed with saturated sodium hydrogen carbonate solution and saturated brine, dried over anhydrous sodium sulfate, and concentrated under reduced pressure. The resulting residue was purified by silica gel chromatography (diethyl ether:hexane = 2:1) to obtain the title compound as a colorless oil (53 mg, 88%).

1.17 (3H, d, J = 6.4 Hz), 1.28-1.37 (1H, m), 2.19 (3H, s), 2.68 (1H, ddd, J = 4.1, 8.5, 12.4 Hz), 4.09 (1H, brs), 4.23 (1H, dd, J = 4.2, 11.5 Hz), 4.92 (1H, brs), 6.61-6.71 (2H, m), 6.91-6.98 (1H, m), 7.01-7.07 (1H, m), 7.14-7.23 (2H, m), 7.45 (2H, d, J = 3.8 Hz).

### Example 3

Preparation of cis-1-acetyl-7-bromo-2-methyl-4-phenylamino-1,2,3,4-tetrahydroquinoline (Compound 23)

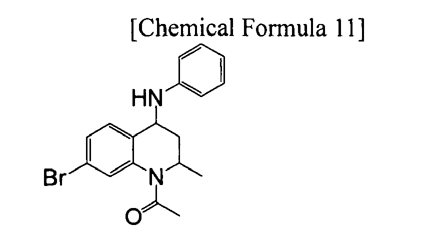

[Process 1] By using 7-bromo-4-oxo-2-methyl-1,2,3,4-tetrahydroquinoline (48 mg), the reaction was carried out in the same manner as [Process 2] of Example 1 to obtain 1-acetyl-7-bromo-4-oxo-2-methyl-1,2,3,4-tetrahydroquinoline (56 mg, 100%).
[Process 2] By using 1-acetyl-7-bromo-4-oxo-2-methyl-1,2,3,4-tetrahydroquinoline (23 mg), the reaction was carried out in the same manner as Example 2 to obtain the title compound as a yellow oil(y substance) (12 mg, 60%).

¹H-NMR(400MHz, CDCl₃) δ:
1.17 (3H, d, J = 6.3Hz), 1.28(1H, m), 2.22 (3H, s), 2.65 (1H, ddd, J = 4.4, 8.4, 12.4 Hz), 3.80 (1H, m), 4.11 (1H, m), 4.86 (1H, m), 6.61 (2H, d, J = 8.5Hz), 6.77 (1H, dd, J = 7.3, 7.3Hz), 7.16-7.2 (5H, m).

### Example 4

Preparation of 1-acetyl-6-cyano-2-methyl-4-phenylamino-1,2,3,4-tetrahydroquinoline (Compound 14)

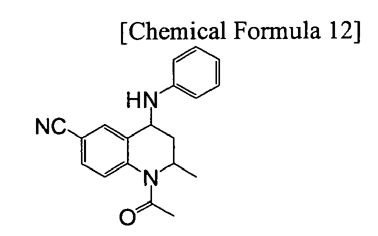

1-Acetyl-6-bromo-2-methyl-4-phenylamino-1,2,3,4-tetrahydroquinoline (100 mg, 0.278 mmoL), zinc cyanide (36 mg, 0.31 mmoL) and tetrakis(triphenylphosphine) palladium (0) (18 mg, 0.015 mmoL) were added to DMF (1.5 mL), and stirred for 2 hrs at 120°C under argon atmosphere. Upon completion of the reaction, the reaction solution was added with water, and then extracted with ethyl acetate. The organic layer was washed with saturated brine, dried over anhydrous sodium sulfate, and then concentrated under reduced pressure. The resulting residue (104 mg) was purified by silica gel fractional thin layer chromatography (ether/hexane = 5/1) to obtain the title compound as a colorless amorphous solid (63 mg, cis:trans = 3:1, 74.1%).

¹H-NMR(400 MHz□CDCl₃) δ:
1.20 (3H, d, J = 6.3 Hz), 1.36 (1H, m), 2.23 (3H, s), 2.68 (1H, ddd, J = 4.4, 8.5, 12.4 Hz), 3.80 (1H, m), 4.19 (1H, m), 4.82 (1H, m), 6.60-6.79 (3H, m), 7.18-7.28 (3H, m), 7.50-7.68 (2H, m).

### Example 5

Preparation of cis-1-acetyl-7-cyano-2-methyl-4-phenylammo-1,2,3,4-tetrahydroquinoline (Compound 7)

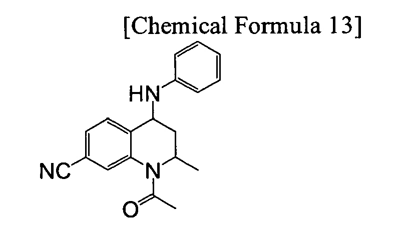

By using cis-1-acetyl-7-bromo-2-methyl-4-phenylamino-1,2,3,4-tetrahydroquinoline obtained from Example 3 (Compound 23, 12 mg), the reaction was carried out in the same manner as Example 4 to obtain the title compound as a pale yellow amorphous solid (6 mg, 60%).

¹H-NMR(400MHz, CDCl₃) δ:
1.19 (3H, d, J = 6.3 Hz), 1.33 (1H, m), 2.23 (3H, s), 2.70 (1H, ddd, J = 4.4, 8.3, 12.5 Hz), 3.83 (1H, m), 4.21 (1H, m), 4.86 (1H, m), 6.60 (2H, d, J = 7.8 Hz), 6.80 (1H, dd, J = 7.3, 7.3 Hz), 7.16-7.26 (3H, m), 7.44-7.49 (2H, m).

### Example 6

Preparation of cis-1-acetyl-4-[(4-isopropoxyphenyl)amino]-2-methyl-1,2,3,4-tetrahydroquinoline · hydrochloride (Compound 18)

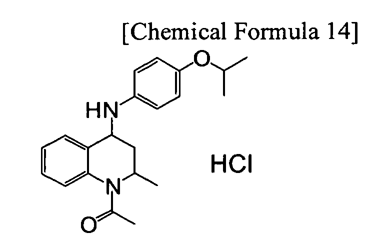

By using 1-acetyl-4-oxo-2-methyl-1,2,3,4-tetrahydroquinoline (60 mg) and 4-isopropoxyaniline (136 mg), the compound was produced in the same manner as Example 2 and converted into the hydrochloride according to a method well known in the art to obtain the title compound as a white solid (53 mg, 47%).

¹H-NMR(400MHz, CD₃OD) δ:
1.14 (3H, d, J = 6.4 Hz), 1.34 (6H, d, J = 6.1 Hz), 1.30-1.35 (1H, m), 2.14 (3H, s), 2.55 (1H, ddd, J = 3.8, 8.6, 12.3 Hz), 4.61-4.74 (3H, m), 7.10 (2H, d, J = 9.0 Hz), 7.41-7.52 (6H, m).

### Example 7

Preparation of cis-1-acetyl-2-methyl-4-[(4-morpholinophenyl)amino]-1,2,3,4-tetrahydroquinoline · hydrochloride (Compound 19)

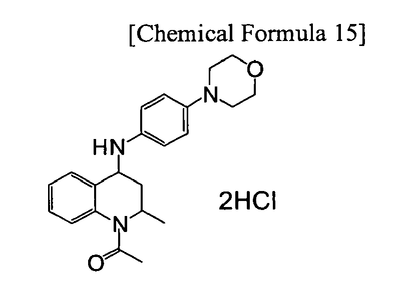

By using 1-acetyl-4-oxo-2-methyl-1,2,3,4-tetrahydroquinoline (60 mg) and 4-morpholinoaniline (107 mg), the compound was produced in the same manner as Example 2 and converted into the hydrochloride according to a method well known in the art to obtain the title compound as a white solid (42 mg, 33%).

¹H-NMR(400MHz, CD₃OD) δ:
1.14 (3H, d, J = 6.4 Hz), 1.29-1.36 (1H, m), 2.18 (3H, s), 2.66 (1H, ddd, J = 3.9, 8.6, 12.4 Hz), 3.63-3.66 (4H, m), 4.03-4.07 (4H, m), 4.27 (1H, dd, J = 4.2, 12.0 Hz), 6.82 (2H, d, J = 9.0 Hz), 7.16-7.22 (2H, m), 7.28-7.35 (2H, m), 7.42 (2H, d, J = 9.0 Hz).

### Example 8

Preparation of cis-1-acetyl-4-[(4-hydroxyphenyl)amino]-2-methyl-1,2,3,4-tetrahydroquinoline · hydrochloride (Compound 24)

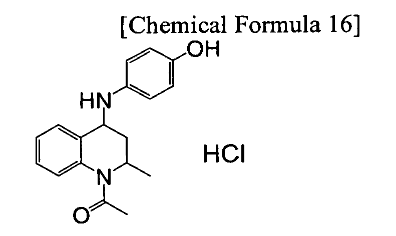

[Process 1] 1-Acetyl-4-oxo-2-methyl-1,2,3,4-tetrahydroquinoline (100 mg) and 4-acetoxyaniline (227 mg) were dissolved in toluene (3 mL). Under ice cooling, titanium tetrachloride (1.0 M dichloromethane solution, 250 µL) was added thereto, stirred for 1 hr, and further stirred for 1 hr at room temperature. Upon completion of the reaction, the reaction mixture was filtered through Celite, and then concentrated under reduced pressure. The resulting residue was purified by silica gel chromatography (chloroform) to obtain 1-acetyl-2-methyl-4-[(4-acetoxyphenyl)imino]-1,2,3,4-tetrahydroquinoline as a hot yellow liquid substance (50 mg, 30%).
[Process 2] 1-Acetyl-2-methyl-4-[(4-acetoxyphenyl)imino]-1,2,3,4-tetrahydroquinoline (45 mg) and sodium cyanoborohydride (17 mg) were dissolved in methanol (2 mL) and stirred for 18 hrs at 50°C. Upon completion of the reaction, 1 N hydrochloric acid was added to the reaction solution, which was then stirred for 30 minutes. After neutralizing with a saturated aqueous solution of sodium hydrocarbonate, the reaction mixture was extracted with chloroform. The organic layer was washed with saturated sodium hydrogen carbonate solution and saturated brine, dried over anhydrous sodium sulfate, and concentrated under reduced pressure. The resulting residue was purified by silica gel chromatography (diethyl ether). After the purification, the resultant was dissolved in 4 N hydrochloric acid - ethyl acetate solution and concentrated under reduced pressure to give the hydrochloride, which was then recrystallized from chloroform · hexane to obtain the title compound as a white solid (18 mg, 40%).

¹H-NMR(400MHz, CD₃OD) δ:
1.14 (3H, d, J = 6.3 Hz), 1.29-1.37 (1H, m), 2.14 (3H, s), 2.54 (1H, ddd, J = 3.9, 8.4, 12.3 Hz), 4.61 (1H, dd, J = 3.9, 12.4 Hz), 4.61-4.73 (1H, m), 6.97 (2H, d, J = 8.8 Hz), 7.37 (2H, d, J = 9.0 Hz), 7.41-7.52 (4H, m).

### Example 9

Preparation of cis-1-cyclohexanecarbonyl-6-fluoro-4-[(4-fluorophenyl)amino]-2-methyl-1,2,3,4-tetrahydroquinoline (Compound 6)

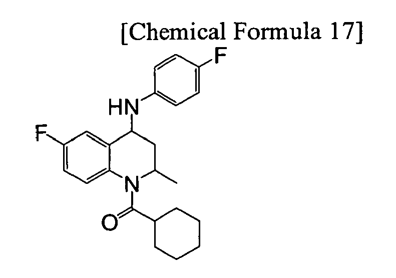

Cis-6-fluoro-4-[(4-fluorophenyl)amino]-2-methyl-1,2,3,4-tetrahydroquinoline (88 mg) was dissolved in dichloromethane (3 mL), added with triethylamine (117 mg) and cyclohexanecarbonyl chloride (168 mg) under ice cooling, and stirred for 16 hrs. Upon completion of the reaction, the reaction solution was added with a saturated aqueous solution of ammonium chloride, extracted with dichloromethane, and dried over anhydrous magnesium sulfate. After carrying out the concentration under reduced pressure, the resulting residue was recrystallized from chloroform to obtain the title compound as a colorless scaly crystal (21 mg, 17%).

¹H-NMR(400MHz, CDCl₃) δ:
1.09 (3H, d, J = 6.4 Hz), 1.14-1.30 (1H, m), 1.39-1.93 (10H, m), 2.63-2.74 (2H, m), 3.69 (1H, brs), 3.98-4.05 (1H, m), 4.93 (1H, brs), 6.51-6.54 (2H, m), 6.88-7.11 (5H, m).

### Example 10

Preparation of 1-acetyl-4-[(4-N,N-dimethylaminophenyl)amino]-2-methyl-1,2,3,4-tetrahydroquinoline · 2 hydrochloride (Compound 20)

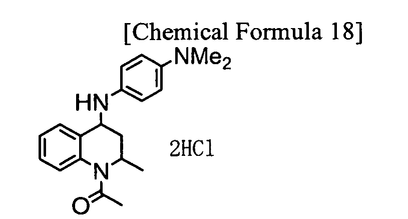

By using 1-acetyl-4-oxo-2-methyl-1,2,3,4-tetrahydroquinoline (100 mg) and N,N-dimethyl-p-phenylenediamine (201 mg), the compound was produced in the same manner as Example 2 and converted into the hydrochloride according to a method well known in the art to obtain the title compound as a white solid (55 mg, cis:trans = 3:1, 28%).

¹H-NMR(400MHz, CDCl₃) δ:
1.16 (3H, d, J = 6.4 Hz), 1.24-1.38 (1H, m), 2.20 (3H, s), 2.65 (1H, m), 3.23 (6H, s), 4.14-4.22 (1H, m), 4.06 (1H, m), 6.70 (2H, d, J = 8.3 Hz), 7.15-7.25 (3H, m), 7.28-7.38 (1H, m), 7.28-7.38 (2H, m).

### Example 11

Preparation of cis-1-acetyl-2-methyl-4-phenoxy-1,2,3,4-tetrahydroquinoline (Compound 26)

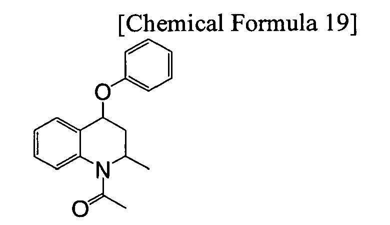

Cis-1-acetyl-4-hydroxy-2-methyl-1,2,3,4-tetrahydroquinoline (100 mg), triphenylbismuth diacetate (300 mg) and copper acetate (9.0 mg) were dissolved in dichloromethane (4 mL), and stirred for 3 hrs at room temperature. The reaction solution was added with water and extracted with chloroform. The organic layer was washed with saturated brine, dried over anhydrous sodium sulfate, and then concentrated under reduced pressure. The resulting residue was purified by silica gel chromatography (ethyl acetate:hexane = 1:1) to obtain the title compound as a white solid (56 mg, yield 49%).

¹H-NMR(400MHz, CDCl₃) δ:
1.18 (3H, d, J = 6.4 Hz), 1.53 (1H, m), 2.19 (3H, s), 2.76 (1H, ddd, J = 4.6, 8.2, 12.8 Hz), 4.89 (1H, brs), 5.07 (1H, dd, J = 4.4, 10.8 Hz), 6.98-7.03 (3H, m), 7.17 (1H, s br), 7.23 (1H, d, J = 6.0 Hz), 7.30-7.34 (3H, m),7.45 (1H, d, J = 7.6 Hz).

### Example 12

Preparation of cis-1-acetyl-4-(4-fluorophenoxy)-2-methyl-1,2,3,4-tetrahydroquinoline (Compound 27)

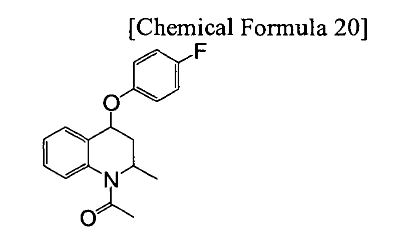

Trans-1-acetyl-4-hydroxy-2-methyl-1,2,3,4-tetrahydroquinoline (50 mg), 4-fluorophenol (112 mg), 1,1-azodicarbonyldipiperidine (126 mg) and tributylphosphine (125 mg) were dissolved in toluene (2 mL) and stirred for 2 hrs at 50°C. The reaction solution was filtered to remove undissolved matters and concentrated under reduced pressure. The resulting residue was purified by silica gel chromatography (diethyl ether) to obtain the title compound as a colorless oil (26 mg, yield 36%).

¹H-NMR(400MHz, CDCl₃) δ:
1.18 (3H, d, J = 6.4 Hz), 1.53 (1H, m), 2.19 (3H, s), 2.73 (1H, ddd, J = 4.6, 8.3, 12.8 Hz), 4.88 (1H, brs), 4.98 (1H, dd, J = 4.7, 10.8 Hz), 6.93 (2H, dd, J = 4.4, 9.3 Hz), 7.01 (2H, t, J = 8.5 Hz), 7.16-7.29 (2H, m), 7.33 (1H, t, J = 7.6 Hz), 7.44 (1H, d, J = 7.1 Hz).

### Example 13

Preparation of cis-1-acetyl-4-(3-fluorophenoxy)-2-methyl-1,2,3,4-tetrahydroquinoline (Compound 28)

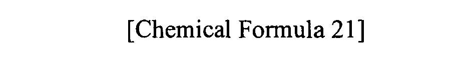

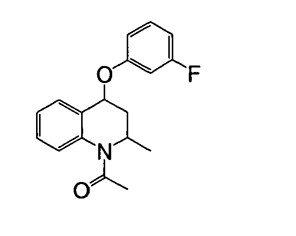

By using trans-1-acetyl-4-hydroxy-2-methyl-1,2,3,4-tetrahydroquinoline (100 mg) and 3-fluoroaniline (112 mg), the compound was produced in the same manner as Example 1 and obtained as a colorless oil (70 mg, 47%).

¹H-NMR(400MHz, CDCl₃) δ:
1.18 (3H, d, J= 6.4 Hz), 1.54 (1H, m), 2.20 (3H, s), 2.75 (1H, ddd, J = 4.6, 8.2, 12.8 Hz), 4.90 (1H, brs), 5.05 (1H, dd, J = 4.6, 10.8 Hz), 6.71 (2H, d, J = 8.3 Hz), 6.77 (1H, d, J = 9.0 Hz), 7.19 (1H, s), 7.23-7.28 (2H, m), 7.33 (1H, t, J = 8.1 Hz), 7.40 (1H, d, J = 7.1 Hz).

### Example 14

Preparation of cis-1-acetyl-4-(2-fluorophenoxy)-2-methyl-1,2,3,4-tetrahydroquinoline (Compound 29)

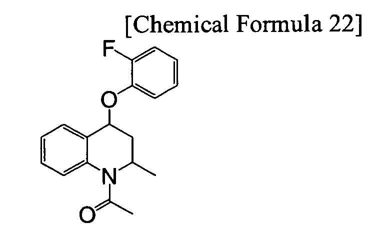

By using trans-1-acetyl-4-hydroxy-2-methyl-1,2,3,4-tetrahydroquinoline (100 mg) and 2-fluoroaniline (112 mg), the compound was produced in the same manner as Example 12 and obtained as a colorless oil (77 mg, 53%).

¹H-NMR(400MHz, CDCl₃) δ:
1.19 (3H, d, J = 6.4 Hz), 1.64 (1H, m), 2.18 (3H, s), 2.76 (1H, ddd, J = 4.7, 8.1, 12.6 Hz), 4.88 (1H, brs), 5.06 (1H, dd, J = 4.8, 10.6 Hz), 6.96-7.09 (3H, m), 7.12-7.16 (2H, m), 7.28 (1H, d, J = 7.6 Hz), 7.33 (1H, t, J = 7.1 Hz), 7.58 (1H, d, J = 7.6 Hz).

### Example 15

Preparation of cis-1-acetyl-4-(2,4-difluorophenoxy)-2-methyl-1,2,3,4-tetrahydroquinoline (Compound 30)

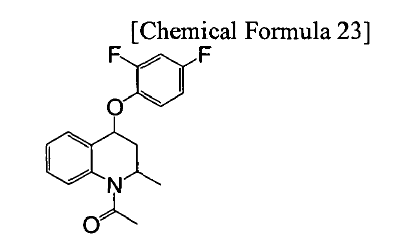

By using trans-1-acetyl-4-hydroxy-2-methyl-1,2,3,4-tetrahydroquinoline (60 mg) and 2,4-fluoroaniline (112 mg), the compound was produced in the same manner as Example 12 and obtained as a colorless oil (55 mg, 59%).

¹H-NMR(400MHz, CDCl₃) δ:
1.19 (3H, d, J = 6.4 Hz), 1.61 (1H, m), 2.17 (3H, s), 2.71 (1H, ddd, J = 4.8, 8.1, 12.9 Hz), 4.86 (1H, brs), 4.98 (1H, dd, J = 4.5, 10.4 Hz), 6.78-6.84 (1H, m), 6.89-6.95 (1H, m), 6.98-7.03 (1H, m), 7.13 (1H, m), 7.28-7.36 (1H, m), 7.58 (1H, d, J = 7.2 Hz).

### Example 16

Preparation of cis-1-acetyl-4-(3,4-difluorophenoxy)-2-methyl-1,2,3,4-tetrahydroquinoline (Compound 31)

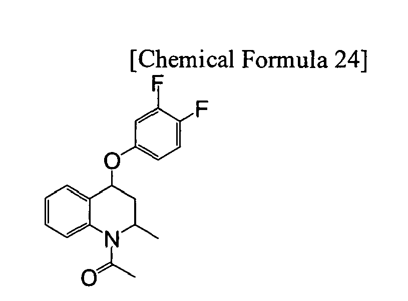

By using trans-1-acetyl-4-hydroxy-2-methyl-1,2,3,4-tetrahydroquinoline (60 mg) and 3,4-difluoroaniline (112 mg), the compound was produced in the same manner as Example 12 and obtained as a colorless oil (59 mg, 64%).

¹H-NMR(400MHz, CDCl₃) δ:
1.19 (3H, d, J = 6.4 Hz), 1.61 (1H, m), 2.19 (3H, s), 2.73 (1H, ddd, J = 4.6, 8.4, 12.8 Hz), 4.89 (1H, brs), 4.97 (1H, dd, J = 4.6, 10.6 Hz), 6.67-6.70 (1H, m), 6.79-6.84 (1H, m), 7.10 (1H, dd, J = 9.2, 18.9 Hz), 7.17-7.21 (1H, m), 7.23-7.29 (2H, m), 7.30-7.35 (1H, m), 7.38 (1H, d, J = 7.3 Hz).

### Example 17

Preparation of cis-1-acetyl-7-fluoro-2-methyl-4-phenoxy-1,2,3,4-tetrahydroquinoline (Compound 32)

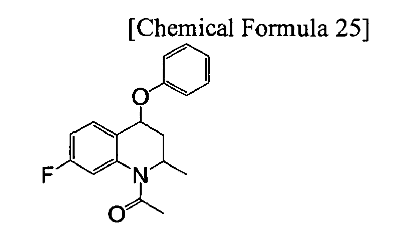

(Process 1) 3-Fluoroaniline (1.1 g) and acetaldehyde (12 g) were dissolved in 1 N hydrochloric acid (20 mL) and stirred for 1.5 hrs at 0°C. The reaction solution was added with a 4N sodium hydroxide aqueous solution, extracted with chloroform, dried over anhydrous sodium sulfate, and concentrated under reduced pressure. The resulting residue was purified by silica gel chromatography (ethyl acetate:hexane = 1:5) to obtain 7-fluoro-4-hydroxy-2-methyl-1,2,3,4-tetrahydroquinoline as a brown oil (860 mg, yield 47%).
(Process 2) The brown oil of 7-fluoro-4-hydroxy-2-methyl-1,2,3,4-tetrahydroquinoline (860 mg) was dissolved in pyridine (0.5 mL) and dichloromethane (10 mL), added with acetyl chloride (16 mg) under ice cooling, and stirred for 1.5 hrs. Upon completion of the reaction, the reaction solution was added with water and extracted with chloroform. The organic layer was dried over anhydrous sodium sulfate and concentrated under reduced pressure. The resulting residue was purified by silica gel chromatography (ethyl acetate:hexane = 1:2) to obtain 1-acetyl-7-fluoro-4-acetoxy-2-methyl-1,2,3,4-tetrahydroquinoline as an oily (1.4 g, 100%).
(Process 3) 1-Acetyl-7-fluoro-4-acetoxy-2-methyl-1,2,3,4-tetrahydroquinoline (1.4 g) was dissolved in ethanol (10 mL), added with a 1N sodium hydroxide aqueous solution (10 mL), and stirred for 1.5 hrs. Upon completion of the reaction, the reaction solution was added with water and extracted with chloroform. The organic layer was dried over anhydrous sodium sulfate and concentrated under reduced pressure. The resulting residue was purified by silica gel chromatography (ethyl acetate:hexane = 1:1) to obtain trans-1-acetyl-7-fluoro-4-hydroxy-2-methyl-1,2,3,4-tetrahydroquinoline as a white solid (400 mg, 34%).
(Process 4) By using trans-1-acetyl-7-fluoro-4-hydroxy-2-methyl-1,2,3,4-tetrahydroquinoline (100 mg) and phenol (84 mg), the compound was produced in the same manner as Example 1 and the title compound was obtained as a white solid (35 mg, 26%).

¹H-NMR(400MHz, CDCl₃) δ:
1.20 (3H, d, J = 6.2 Hz), 1.60 (1H, m), 2.23 (3H, s), 2.71 (1H, ddd, J = 5.1, 7.8, 13.0 Hz), 4.84 (1H, brs), 5.05 (1H, dd, J = 4.6, 9.8 Hz), 6.92-7.04 (5H, m), 7.32 (1H, dd, J = 7.6, 8.8 Hz), 7.41 (1H, dd, J = 6.4, 8.1 Hz).

### Example 18

Preparation of cis-1-acetyl-8-fluoro-2-methyl-4-phenoxy-1,2,3,4-tetrahydroquinoline (Compound 33)

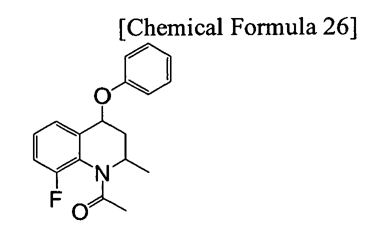

By using 2-fluoroaniline, the reaction was carried out in the same manner as Example 17 to obtain cis-1-acetyl-8-fluoro-4-hydroxy-2-methyl-1,2,3,4-tetrahydroquinoline. By using cis-1-acetyl-8-fluoro-4-hydroxy-2-methyl-1,2,3,4-tetrahydroquinoline (100 mg), the reaction was carried out in the same manner as Example 1 to obtain the title compound as a white solid (52 mg, 39%).

¹H-NMR(400MHz, CDCl₃) δ:
1.17 (3H, d, J = 6.3 Hz), 1.45 (1H, m), 2.10 (3H, s), 2.81 (1H, ddd, J = 4.6, 8.6, 13.0 Hz), 4.93 (1H, m), 5.04 (1H, dd, J = 4.4, 11.5 Hz), 6.95-7.04 (3H, m), 7.11 (1H, m), 7.20-7.28 (2H, m), 7.32 (2H, dd, J = 7.6, 8.6 Hz).

### Example 19

Preparation of cis-1-acetyl-4-(4-fluorophenoxy)-6-fluoro-2-methyl-1,2,3,4-tetrahydroquinoline (Compound 34)

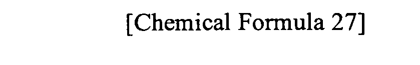

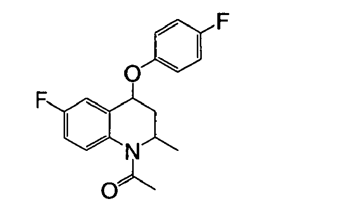

By using 4-fluoroaniline, the reaction was carried out in the same manner as Example 17 to obtain trans-1-acetyl-6-fluoro-4-hydroxy-2-methyl-1,2,3,4-tetrahydroquinoline. By using trans-1-acetyl-6-fluoro-4-hydroxy-2-methyl-1,2,3,4-tetrahydroquinoline (60 mg) and 4-fluorophenol (112 mg), the compound was produced in the same manner as Example 1 and obtained as a colorless oil (58 mg, 61%).

¹H-NMR(400MHz, CDCl₃) δ:
1.17 (3H, d, J = 6.4 Hz), 1.46 (1H, m), 2.16 (3H, s), 2.74 (1H, ddd, J = 4.8, 8.2, 12.9 Hz), 4.88 (1H, brs), 4.94 (1H, dd, J = 4.6, 10.7 Hz), 6.91-6.95 (2H, m), 6.99-7.04 (3H, m), 7.12 (1H, m), 7.19 (1H, d, J = 9.6 Hz).

### Example 20

Preparation of cis-1-acetyl-6-fluoro-2-methyl-4-phenoxy-1,2,3,4-tetrahydroquinoline (Compound 35)

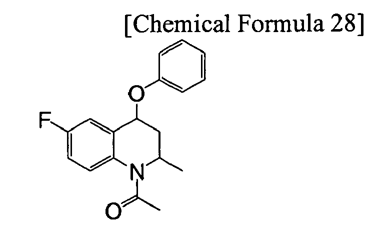

By using cis-1-acetyl-6-fluoro-4-hydroxy-2-methyl-1,2,3,4-tetrahydroquinoline (39 mg) and phenol, the compound was produced in the same manner as Example 12 and obtained as a white solid (33 mg, 63%).

¹H-NMR(400MHz, CDCl₃) δ:
1.18 (3H, d, J = 6.4 Hz), 1.53 (1H, m), 2.17 (3H, s), 2.77 (1H, ddd, J = 4.6, 8.3, 12.5 Hz), 4.88 (1H, brs), 5.02 (1H, dd, J = 4.7, 10.6 Hz), 6.97-7.04 (4H, m), 7.13 (1H, s), 7.21 (1H, dd, J = 2.0, 8.8 Hz), 7.33 (2H, t, J = 8.1 Hz).

### Example 21

Preparation of cis-1-acetyl-2-methyl-4-benzyloxy-1,2,3,4-tetrahydroquinoline (Compound 36)

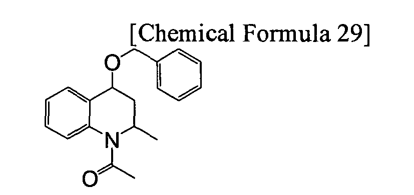

Cis-1-acetyl-4-hydroxy-2-methyl-1,2,3,4-tetrahydroquinoline (20 mg) was dissolved in tetrahydrofuran (2 mL), added with sodium hydride (3 mg) under ice cooling, and stirred for 30 minutes. Further, the solution was added with benzyl bromide (15 µL) and stirred for one day. The reaction solution was then added with water and extracted with ethyl acetate. The organic layer was washed with saturated brine, dried over anhydrous sodium sulfate, and then concentrated under reduced pressure. The resulting residue was purified by silica gel chromatography (ethyl acetate:hexane = 1:2) to obtain the title compound as a yellow solid (9 mg, yield 31%).

¹H-NMR(400MHz, CDCl₃) δ:
1.14 (3H, d, J = 6.3 Hz), 1.26-1.35 (1H, m), 2.12 (3H, s), 2.81 (1H, ddd, J = 4.5, 8.3, 12.6 Hz), 4.31 (1H, dd, J = 4.8, 11.2 Hz), 4.73 (1H, brs), 4.77 (2H, dd, J = 12.0, 19.6 Hz), 7.10 (1H, brs), 7.26-7.34 (2H, m), 7.37-7.44 (4H, m), 7.58-7.61 (1H, m).

### Example 22

Preparation of 1-acetyl-4-[(4-fluorophenyl)amino]-2-methyl-6-methoxy-1,2,3,4-tetrahydroquinoline (Compound 37)

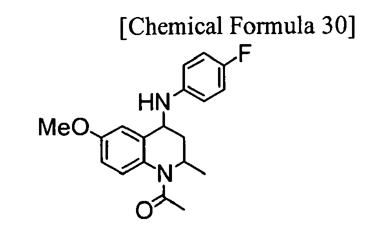

By using 1-acetyl-6-methoxy-4-oxo-2-methyl-1,2,3,4-tetrahydroquinoline (70 mg), the reaction and the processing were carried out in the same manner as Example 2 to obtain the title compound as a yellow oil (59 mg, 60%) (cis:trans = 3:1).

¹H-NMR(400MHz, CDCl₃) δ:
1.13 (2.5H, d, J = 6.4 Hz), 1.16 (0.5H, d, J = 6.6 Hz), 1.19-1.28 (1H, m), 2.13 (0.5H, s), 2.15 (2.5H, s), 2.44-2.53 (0.17H, m), 2.62 (0.83H, ddd, J = 4.2, 8.6, 12.3 Hz), 3.66-3.69 (1H, m), 3.75 (2.5H, s), 3.80 (0.5H, s), 4.06-4.14 (0.83H, m), 4.44-4.49 (0.17H, m), 4.91 (1H, brs), 6.56-6.59 (2H, m), 6.78-6.93 (4.17H, m), 7.02-7.07 (0.83H, m).

### Example 23

Preparation of 1-acetyl-4-[(4-hydroxymethylphenyl)amino]-2-methyl-1,2,3,4-tetrahydroquinoline (Compound 38)

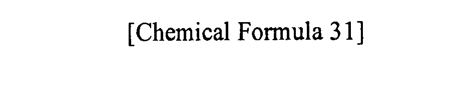

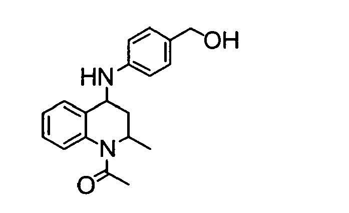

[Process 1] 1-Acetyl-4-oxo-2-methyl-1,2,3,4-tetrahydroquinoline (610 mg) and 4-aminobenzyl(tert-butyldiphenylsilyl) ether (3.25 g) were dissolved in toluene (15 mL). Under ice cooling, titanium tetrachloride (1.0 M dichloromethane solution, 3 mL) was added thereto, stirred for 1 hr, and further stirred for 3 hrs at 80°C. Upon completion of the reaction, the mixture was filtered through Celite and concentrated under reduced pressure. The resulting residue was purified by silica gel chromatography (diethyl ether:hexane = 2:1) to obtain 1-acetyl-2-methyl-4-[(4-tert-butyldiphenylsilyloxymethylphenyl)imino]-1,2,3,4-tetrahydroquinoline as a yellow oil (1.5 g, 91 %).
[Process 2] 1-Acetyl-2-methyl-4-[(4-tert-butyldiphenylsilyloxymethylphenyl)imino]-1,2,3,4-tetrahydroquinoline (715 mg), sodium borohydride (74 mg), and cerium trichloride heptahydrate (488 mg) were dissolved in methanol (8 mL) and stirred for 2 hrs at 50°C. Upon completion of the reaction, 1 N hydrochloric acid was added to the reaction solution, which was then stirred for 30 minutes. After neutralizing with a saturated aqueous solution of sodium hydrocarbonate, the mixture was extracted with chloroform. The organic layer was washed with saturated sodium hydrogen carbonate solution and saturated brine, dried over anhydrous sodium sulfate, and concentrated under reduced pressure. The resulting residue was purified by silica gel chromatography (hexane:ethyl acetate = 1:1) to obtain 1-acetyl-4-[(4-tert-butyldiphenylsilyloxymethylphenyl)amino]-2-methyl-1,2,3,4-tetrahydroquinoline as a yellow oil (615 mg, 85%) (cis:trans = 3:1).
[Process 3] 1-Acetyl-4-[(4-tert-butyldiphenylsilyloxymethylphenyl)amino]-2-methyl-1,2,3,4-tetrahydroquinoline (615 mg) was dissolved in tetrahydrofuran (2 mL), added with tetrabutylammonium fluoride (1.0 M tetrahydrofuran solution, 2.8 mL), and stirred for 3 hrs at room temperature. Upon completion of the reaction, the reaction mixture was extracted with ethyl acetate. The organic layer was washed with saturated sodium hydrogen carbonate solution and saturated brine, dried over anhydrous sodium sulfate, and concentrated under reduced pressure. The resulting residue was purified by silica gel chromatography (eluent; ether only) to obtain 1-acetyl-4-[(4-hydroxymethylphenyl)amino]-2-methyl-1,2,3,4-tetrahydroquinoline as a yellow oil (264 mg, 79%) (cis:trans = 3:1).

¹H-NMR(400MHz, CDCl₃) δ:
1.16 (2.25H, d, J = 6.4Hz), 1.19 (0.75H, d, J = 6.4Hz), 1.23-1.28 (1H, m), 2.16 (0.75H, s), 2.19 (2.25H, s), 2.52 (0.25H, ddd, J = 5.2, 7.6, 13.4 Hz), 2.65 (0.75H, ddd, J = 4.3, 8.7, 12.2 Hz), 3.89-3.91 (1H, m), 4.18-4.25 (0.75H, m), 4.54-4.60 (2.25H, m), 4.91 (1H, brs), 6.62-6.65 (2H, m), 7.14-7.22 (4H, m), 7.26-7.30 (1.75H, m), 7.40 (0.25H, dd, J = 1.5, 7.8 Hz).

### Example 24

Preparation of cis-4-[(4-methanesulfonylaminophenyl)amino]-2-methyl-1,2,3,4-tetrahydroquinoline (Compound 39)

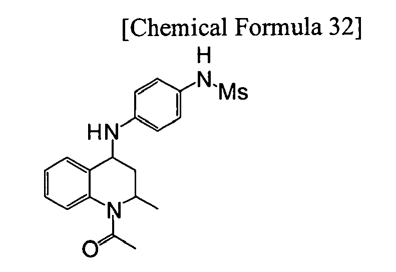

The reaction and the processing were carried out in the same manner as Example 2 to obtain the title compound as a white solid (20 mg, 53%).

¹H-NMR(400MHz, CDCl₃) δ:
1.17 (3H, d, J = 6.4 Hz), 1.28-1.37 (1H, m), 2.19 (3H, s), 2.66 (1H, ddd, J = 4.1, 8.5, 12.4 Hz), 2.95 (3H, s), 3.91 (1H, d, J = 7.6 Hz), 4.18 (1H, dd, J = 4.5, 12.5 Hz), 4.92 (1H, brs), 6.02 (1H, s), 6.61 (2H, d, J = 8.8 Hz), 7.11 (2H, d, J = 8.5 Hz), 7.14-7.31 (4H, m).

### Example 25

Preparation of 1-acetyl-4-[(4-chlorophenyl)amino]-2-methyl-6-morpholino-1,2,3,4-tetrahydroquinoline · hydrochloride (Compound 40)

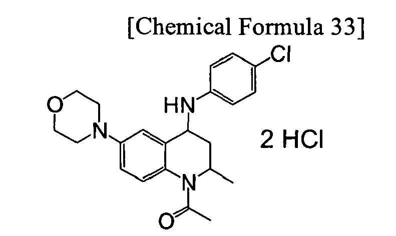

The compound was produced in the same manner as Example 2, converted into the hydrochloride according to a method well known in the art, and recrystallized from chloroform · ether to obtain the title compound as a brown solid (19 mg, cis:trans = 10:1, 23%).

¹H-NMR(400MHz, CDCl₃) δ:
1.12-1.26 (3.3H, m), 1.56-1.75 (1.1H, m), 2.14 (0.3H, s), 2.16 (3H, s), 2.40-2.50 (0.1H, m), 2.60 (1H, ddd, J = 4.2, 8.3, 12.5Hz), 3.00-3.26 (4.4H, m), 3.72-3.95 (4.4H, m), 4.08-4.17 (1H, m), 4.44-4.52 (0.1H, m), 4.82-4.96 (1.1H, brs), 6.78-6.90 (3.3H, m), 7.01-7.28 (2.2H, m).

### Example 26

Preparation of ethyl cis-1-acetyl-4-[(4-chlorophenyl)amino]-2-methyl-1,2,3,4-tetrahydroquinoline-6-carboxylate (Compound 41)

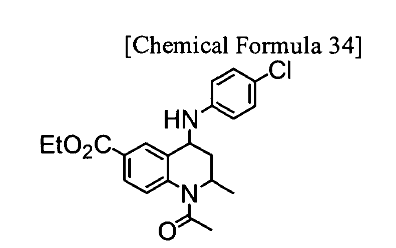

The reaction and the processing were carried out in the same manner as Example 2 to obtain the title compound as a colorless oil (13 mg, 72%).

¹H-NMR(400MHz, CDCl₃) δ:
1.17 (3H, d, J = 6.3 Hz), 1.23-1.33 (1H, m), 1.35 (3H, t, J = 7.1 Hz), 2.21 (3H, s), 2.68 (1H, ddd, J = 4.1, 8.5, 12.4 Hz), 3.86 (1H, d, J = 7.8 Hz), 4.15-4.25 (1H, m), 4.30-4.40 (2H, m), 4.80-4.91 (1H, m), 6.55-6.62 (2H, m), 7.10-7.18 (2H, m), 7.22 (1H, d, J = 8.1 Hz), 7.95-8.01 (2H, m).

### Example 27

Preparation of 1-acetyl-4-[(4-chlorophenyl)amino]-2-methyl-1,2,3,4-tetrahydroquinoline-6-carboxylic acid (Compound 42)

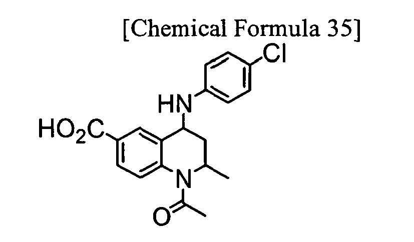

Compound 41 was hydrolyzed according to a method well known in the art to obtain the title compound as a white solid (17 mg, 61 %) (cis:trans = 10:1).

¹H-NMR(400MHz, CDCl₃) δ:
1.19 (3H, d, J = 6.3 Hz), 1.23-1.33 (1H, m), 2.23 (3H, s), 2.69 (1H, ddd, J = 4.2, 8.5, 12.4 Hz), 4.21 (1H, dd, J = 4.2, 12.0 Hz), 4.80-4.92 (1H, m), 6.55-6.62 (2H, m), 7.10-7.28 (3H, m), 7.98-8.07 (2H, m).

### Example 28

Preparation of 1-acetyl-4-[(4-chlorophenyl)amino]-2-methyl-1,2,3,4-tetrahydroquinoline-6-carboxamide (Compound 43)

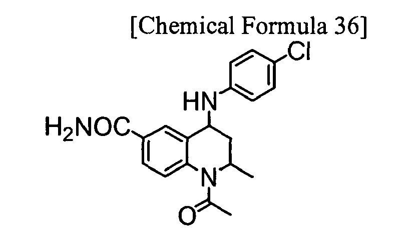

Compound 42 was amidated according to a method well known in the art to obtain the title compound as a white solid (30 mg, 50%) (cis:trans = 3:1).

¹H-NMR(400MHz, CDCl₃) δ:
1.12-1.38 (4H, m), 1.91 (0.25H, m), 2.21 (3H, s), 2.41 (0.25H, m), 2.68 (0.75H, ddd, J = 4.1, 8.5, 12.4 Hz), 3.87 (1H, d, J = 7.1 Hz), 4.17 (0.75H, m), 4.63 (0.25H, brs), 4.86 (1H, m), 5.63 (1H, brs), 5.97 (1H, brs), 6.54-6.61 (3H, m), 7.10-7.40 (3H, m), 7.66-7.94 (1H, m).

### Example 29

Preparation of 1-acetyl-4-[(4-chlorophenyl)amino]-2-methyl-7-morpholino-1,2,3,4-tetrahydroquinoline (Compound 44)

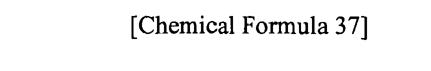

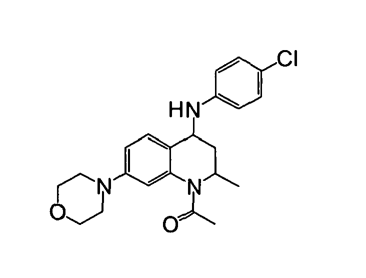

The bromine atom of Compound 74 was substituted with a morpholino group according to a method well known in the art to obtain the title compound as a yellow solid (31 mg, cis:trans = 5:1, 61%).

¹H-NMR(400MHz, CDCl₃) δ:
1.15-1.20 (3.6H, m),1.63-1.79 (1.2H, m), 2.18 (3H, s), 2.21 (0.6H, s), 2.38-2.48 (0.2H, m), 2.60 (1H,ddd, J = 4.4, 8.0, 12.4Hz), 3.09-3.23 (4.8H, m), 3.62-3.90 (5.5H, m), 4.08-4.16 (1H, m), 4.46-4.53 (0.2H, m), 4.80-4.96 (1.2H, brs), 6.54-6.57 (2.4H, m), 6.60-6.73 (2.4H, m), 7.05-7.16 (3.6H, m).

### Example 30

Preparation of cis-4-[(4-chlorophenyl)amino]-2-methyl-6-methanesulfonylamino-1,2,3,4-tetrahydroquinoline (Compound 45)

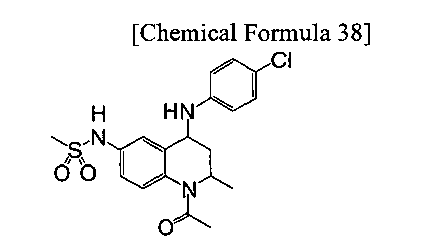

The reaction and the processing were carried out in the same manner as Example 2 to obtain the title compound as a white solid (46 mg, 56%).

¹H-NMR(400MHz, CDCl₃) δ:
1.16 (3H, d, J = 6.1 Hz), 1.25-1.33 (1H, m), 2.19 (3H, s), 2.66 (1H, ddd, J = 4.2, 8.5, 12.5 Hz), 2.96 (3H, s), 3.82-3.84 (1H, m), 4.13 (1H, dd, J = 5.4, 13.7 Hz), 4.89 (1H, brs), 6.33 (1H, s), 6.55 (2H, d, J = 8.8 Hz), 7.00 (1H, s), 7.16 (2H, d, J = 8.8 Hz), 7.10-7.21 (4H, m).

### Example 31

Preparation of ethyl 1-acetyl-4-(4-fluorophenoxy)-2-methyl-1,2,3,4-tetrahydroquinoline-6-carboxylate (Compound 53)

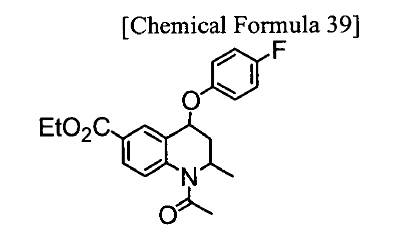

The reaction and the processing were carried out in the same manner as Example 12 to obtain the title compound as a colorless oil (150 mg, 90%).

¹H-NMR(400MHz, CDCl₃) δ:
1.20 (3H, d, J = 6.3 Hz), 1.37 (3H, t, J = 7.0 Hz), 1.50-1.63 (1H, m), 2.21 (3H, s), 2.71 (1H,m), 4.33-4.43 (2H, m), 4.75-4.90 (1H, m), 4.98-5.07 (1H, m), 6.90-7.10 (5H, m), 8.00-8.05 (1H, m), 8.17 (1H, m).

### Example 32

Preparation of cis-1-acetyl-4-(4-fluorophenoxy)-2-methyl-1,2,3,4-tetrahydroquinoline-6-carboxamide (Compound 54)

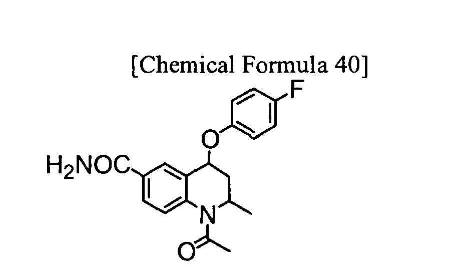

Compound 53 was amidated according to a method well known in the art to obtain the title compound as a white solid (14 mg, 19%).

¹H-NMR(400MHz, CDCl₃) δ:
1.20 (3H, d, J = 6.3 Hz), 1.50-1.63 (1H, m), 2.21 (3H, s), 2.73 (1H, ddd, J = 4.6, 8.0, 12.7Hz), 4.74-4.88 (1H, m), 5.03 (1H, dd, J = 4.7,10.2Hz), 5.91 (1H,brs), 6.11 (1H,brs), 6.80-7.08 (4H, m), 7.20-7.40 (1H, m), 7.80-7.86 (1H, m), 7.91 (1H, m).

### Example 33

Preparation of 1-acetyl-4-(4-morpholinophenoxy)-2-methyl-1,2,3,4-tetrahydroquinoline (Compound 55)

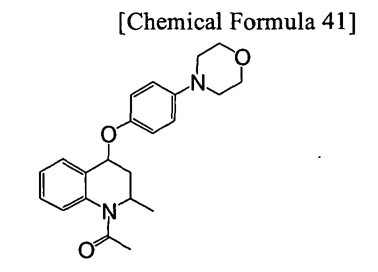

A morpholine ring was contructed for Compound 68 according to a method well known in the art to obtain the title compound as a pale brown oil (27 mg, cis:trans = 3:1, 73%).

¹H-NMR(400MHz, CDCl₃) δ:
1.15-1.80 (4H, m), 1.40-1.64 (1.3H, m), 2.12 (1H, s), 2.18 (3H, s), 2.68-2.82 (1.3H, m), 2.98-3.25 (5.3H, m), 3.75-4.00 (5.3H, m), 4.80-5.18 (2.3H, m), 5.20 (0.3H, dd, 3.68 , 3.54 Hz), 6.70-7.55 (10.6H, m).

### Example 34

Preparation of cis-1-acetyl-7-fluoro-4-(4-fluorophenoxy)-2-methyl-1,2,3,4-tetrahydroquinoline (Compound 56)

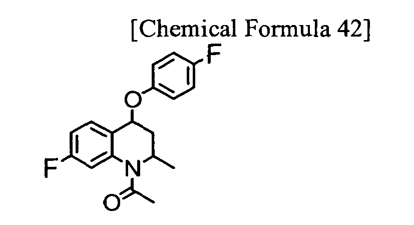

The reaction and the processing were carried out in the same manner as Example 12 to obtain the title compound as a white solid (60 mg, 42%).

¹H-NMR(400MHz, CDCl₃) δ:
1.19 (3H, d, J = 6.3 Hz), 1.50-1.70 (1H, m), 2.22 (3H, s), 2.68 (1H, m), 4.83 (1H, brs), 4.53 (1H, dd, J = 4.4, 9.3 Hz), 6.88-7.04 (6H, m), 7.34-7.45 (1H, m).

### Example 35

Preparation of cis-1-acetyl-4-(4-hydroxyphenoxy)-2-methyl-1,2,3,4-tetrahydroquinoline (Compound 57)

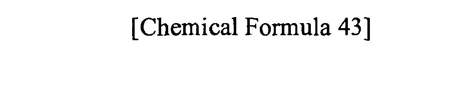

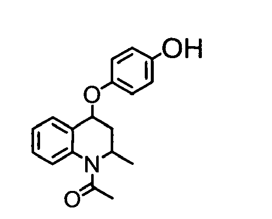

Compound 64 was processed to obtain the title compound as a colorless oil (66 mg, 86%).

¹H-NMR(400MHz, CDCl₃) δ:
1.18 (3H, d, J = 6.4 Hz), 1.40-1.60 (1H, m), 2.18 (3H, s), 2.72 (1H, ddd, J = 4.6, 8.3, 12.7 Hz), 4.86 (1H, brs), 4.90-5.00 (2H, m), 6.75-6.82 (2H, m), 6.85-6.90 (2H, m), 7.10-7.35 (3H, m), 7.49 (1H, d, J = 7.3 Hz).

### Example 36

Preparation of cis-1-acetyl-7-fluoro-4-[(3-fluorophenyl)amino]-2-methyl-1,2,3,4-tetrahydroquinoline (Compound 58)

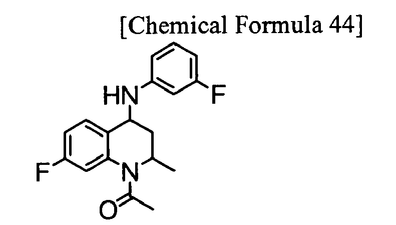

The reaction and the processing were carried out in the same manner as Example 1 to obtain the title compound as a white solid (80 mg, 87%).

¹H-NMR(400MHz, CDCl₃) δ:
1.17 (3H, d, J = 6.3 Hz), 1.23-1.35 (1H, m), 2.22 (3H, s), 2.65 (1H, ddd, J = 4.7, 8.7, 12.7 Hz), 3.93 (1H, brs), 4.06-4.18 (1H, m), 4.80-4.96 (1H, m), 6.30 (1H, dt, J = 2.2, 11.2 Hz), 6.40 (1H, dd, J = 2.2, 8.1 Hz), 6.44 (1H, dt, J = 2.4, 8.4 Hz), 6.85-6.95 (2H, m), 7.09-7.16 (1H, m), 7.20-7.27 (1H, m).

### Example 37

Preparation of 1-aetyl-2-ethyl-4-phenylamino-1,2,3,4-tetrahydroquinoline (Compound 59)

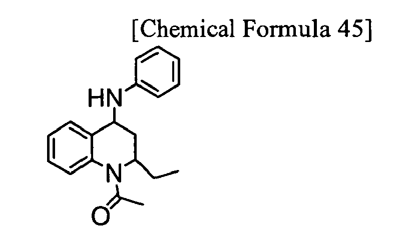

The reaction and the processing were carried out in the same manner as Example 2 to obtain the title compound as a white solid (36 mg, 79%) (cis:trans = 5:1).

¹H-NMR(400MHz, CDCl₃) δ:
0.83-0.92 (3H, m), 1.25-1.65 (3H, m), 1.82-1.96 (0.15H, brs), 2.14-2.19 (3H, s), 2.37-2.47 (0.15H, m), 2.65 (0.85H, ddd, J = 4.6, 8.8, 12.2 Hz), 3.84 (1H, brs), 4.22 (0.85H, d, J = 10.0 Hz), 4.62 (0.15H, t, J = 5.1 Hz), 4.85 (1H, brs), 6.62-6.78 (3H, m), 7.12-7.44 (6H, m).

### Example 38

Preparation of cis-1-acetyl-3,3-dimethyl-4-phenylamino-1,2,3,4-tetrahydroquinoline (Compound 60)

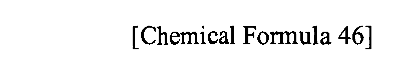

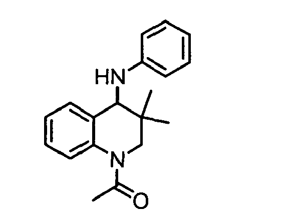

The reaction and the processing were carried out in the same manner as Example 2 to obtain the title compound as a white solid (31 mg, 70%).

¹H-NMR(400MHz, CDCl₃) δ:
0.91 (3H, s), 1.12 (3H, s), 2.32 (3H, s), 3.63 (1H, d, J = 13.6 Hz), 3.71 (1H, brs), 3.77 (1H, d, J = 8.8 Hz), 4.30 (1H, d, J = 8.8Hz), 6.62-6.76 (3H, m), 7.05-7.35 (6H, m).

### Example 39

Preparation of 1-acetyl-4-phenylamino-8-methoxy-2-methyl-1,2,3,4-tetrahydroquinoline (Compound 61)

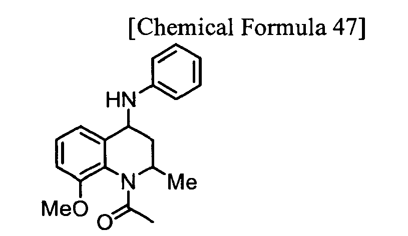

The reaction and the processing were carried out in the same manner as Example 2 to obtain the title compound as a yellow oil (145 mg, 85%) (cis:trans = 3:1).

¹H-NMR(400MHz, CDCl₃) δ:
1.05-1.25 (4H, m), 1.93 (1H, s), 2.02 (2H, s), 2.60-2.72 (1H, m), 3.78-3.88 (4H, m), 4.17 (0.75H, m), 4.52 (0.25H, m), 4.94-5.06 (1H, m), 6.58-6.78 (2H, m), 6.86-7.01 (2H, m), 7.10-7.24 (4H, m).

### Example 40

Preparation of cis-1-acetyl-4-(3,5-difluorophenoxy)-2-methyl-1,2,3,4-tetrahydroquinoline (Compound 62)

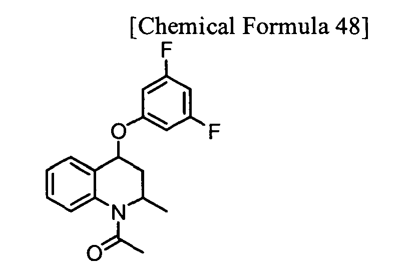

The reaction and the processing were carried out in the same manner as Example 12 to obtain the title compound as a white solid (58 mg, 61%).

¹H-NMR(400MHz, CDCl₃) δ:
1.19 (3H, d, J = 6.6 Hz), 1.56-1.60 (1H, m), 2.20 (3H, s), 2.74 (1H, ddd, J = 4.7, 8.2, 12.7 Hz), 4.90 (1H, brs), 5.02 (1H, dd, J = 5.0, 10.6 Hz), 6.67-6.70 (1H, m), 6.79-6.84 (1H, m), 7.10 (1H, dd, J = 9.2 18.9 Hz), 7.17-7.21 (1H, m), 7.23-7.29 (2H, m), 7.30-7.35 (1H, m), 7.38 (1H, d, J = 7.3 Hz).

### Example 41

Preparation of 1-acetyl-8-bromo-4-phenylamino-2-methyl-1,2,3,4-tetrahydroquinoline (Compound 63)

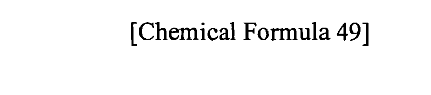

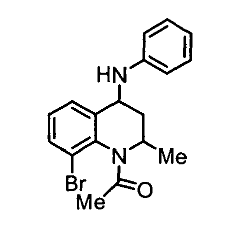

The reaction and the processing were carried out in the same manner as Example 2 to obtain the title compound as a pale brown amorphous substance (215 mg, 70%) (cis:trans = 3:1).

¹H-NMR(400MHz, CDCl₃) δ:
1.13-1.22 (3.75H, m), 1.48-1.55 (0.25H, m), 1.93 (1H, s), 2.10 (2H, s), 2.62-2.74 (1H, m), 3.85 (1H, m), 4.10 (0.75H, m), 4.49 (0.25H, brs), 5.08-5.22 (1H, m), 6.58-6.80 (3H, m), 7.10-7.30 (4H, m), 7.38-7.42 (0.25H, m), 7.52-7.60 (0.75H, m).

### Example 42

Preparation of cis-1-acetyl-4-(4-benzyloxyphenoxy)-2-methyl-1,2,3,4-tetrahydroquinoline (Compound 64)

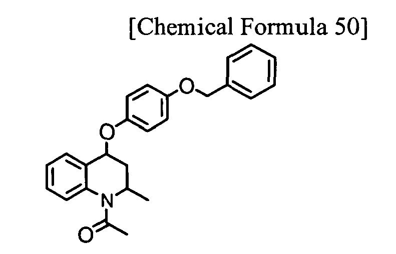

The reaction and the processing were carried out in the same manner as Example 12 to obtain the title compound as a colorless oil (200 mg, 71 %).

¹H-NMR(400MHz, CDCl₃) δ:
1.17 (3H, d, J = 6.6 Hz), 1.40-1.70 (1H, m), 2.18 (3H, s), 2.72 (1H, m), 4.85 (1H, brs), 4.92-4.98 (1H, m), 5.03 (2H, s), 6.29 (4H, s), 7.10-7.50 (9H, m).

### Example 43

Preparation of 6-fluoro-4-[(4-fluorophenyl)amino]-2-methyl-1-N-methylcarbamoyl-1,2,3,4-tetrahydroquinoline (Compound 65)

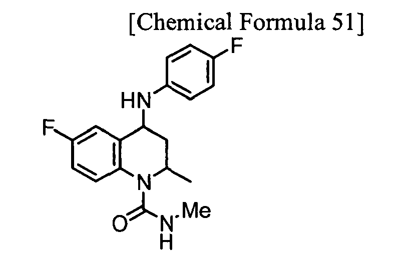

Cis-6-fluoro-4-[(4-fluorophenyl)amino]-2-methyl-1,2,3,4-tetrahydroquinoline (41 mg) was dissolved in dichloromethane (1.5 mL), added with triphosgen (29 mg) under ice cooling, and stirred for 30 minutes. Upon completion of the reaction, the solution was concentrated under reduced pressure. The resulting residue was dissolved in THF (1.5 mL), added with THF solution (0.74 mL) of methylamine under ice cooling, and stirred for 1 hr. Upon completion of the reaction, the solution was concentrated under reduced pressure, and the resulting residue was purified by silica gel chromatography (diethyl ether:hexane = 2:1) to obtain the title compound as a white solid (15 mg, 30%).

¹H-NMR(400MHz, CDCl₃) δ:
1.17 (3H, d, 6.3Hz), 1.15-1.40 (1H, m), 1.56-1.77 (1H, brs), 2.16 (1H, ddd, 4.4, 8.4, 12.4 Hz), 2.83 (3H, d, 4.6 Hz), 3.62-3.84 (1H, brs), 4.02-4.17 (1H, m), 4.70-4.90 (2H, m), 6.49-6.63 (2H, m), 6.75-7.11 (3H, m), 7.24-7.34 (2H, m).

### Example 44

Preparation of 1-cyclopentanecarbonyl-6-fluoro-2-methyl-4-[(4-fluorophenyl)amino]-1,2,3,4-tetrahydroquinoline (Compound 66)

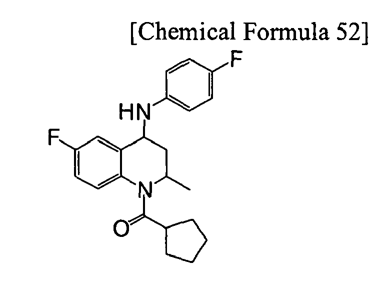

The reaction was carried out in the same manner as Example 9 and recrystallized from chloroform · hexane to obtain the title compound as a white powder crystal (20 mg, 24%).

¹H-NMR(400MHz, CDCl₃) δ:
1.10 (3H, d, J = 6.3 Hz), 1.14-2.10 (9H, m), 2.66 (1H, ddd, 4.4, 9.3, 14.4 Hz), 3.00-3.10 (1H, m), 3.98-4.10 (1H, m), 4.84-5.02 (1H, brs), 6.57 (2H, m), 6.80-7.19 (5H, m).

### Example 45

Preparation of 1-acetyl-2-methyl-4-(4-nitrophenoxy)-1,2,3,4-tetrahydroquinoline (Compound 67)

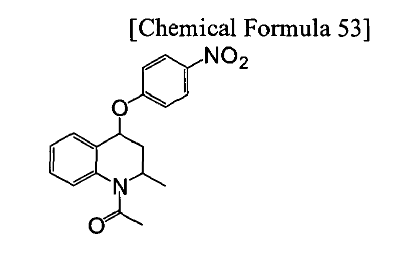

The reaction and the processing were carried out in the same manner as Example 12 to obtain the title compound as a yellow solid (90 mg, cis:trans = 2:1, 40%).

¹H-NMR(400MHz, CDCl₃) δ:
1.18-1.21 (4.5H, m), 1.50-1.75 (1.5H, m), 2.07 (1.5H, s), 2.22 (3H,s), 2.74-2.90 (1.5H, m), 4.86-5.60 (1.5H, m), 5.19 (1H, dd, 4.6, 10.6 Hz), 5.44 (0.5H, dd, 3.2, 3.4 Hz), 6.98 (1H, d, 4.9 Hz), 7.06 (2H, d, 7.1 Hz), 7.18-7.41 (6H, m), 8.15 (1H, d, 4.9 Hz), 8.24 (2H, d, 7.1 Hz).

### Example 46

Preparation of 1-acetyl-4-(4-aminophenoxy)-2-methyl-1,2,3,4-tetrahydroquinoline (Compound 68)

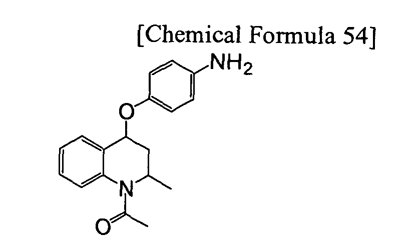

Compound 67 was reduced according to a method well known in the art to obtain the title compound as a pale brown solid (121 mg, cis:trans = 2:1, 100%).

¹H-NMR(400MHz, CDCl₃) δ:
1.09-1.25 (4.5H, m), 1.41-1.65 (1.5H, m), 2.05 (1.5H, s), 2.13 (3H, s), 2.66-2.80 (1.5H, m), 3.00-3.50 (3H, brs), 4.75-5.00 (2.5H, m), 5.13 (0.5H, dd, 3.4, 2.0 Hz), 6.51-6.88 (6H, m), 7.11-7.41 (6H, m).

### Example 47

Preparation of 1-acetyl-4-[(4-methoxymethylphenyl)amino]-2-methyl-1,2,3,4-tetrahydroquinoline (Compound 69)

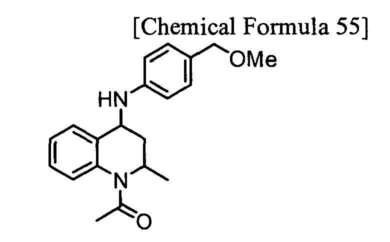

[Process 1] 1-Acetyl-4-[(4-hydroxymethylphenyl)amino]-2-methyl-1,2,3,4-tetrahydroquinoline (16 mg) was dissolved in THF, cooled to 0°C, added with sodium hydride (44 mg) and iodomethane (121 mg), and stirred for 2 days at 40°C. Upon the completion of the reaction, the solution was concentrated under reduced pressure, and the resulting residue was purified by silica gel chromatography (eluent; ether only) to obtain the title compound as a yellow oil (5 mg, 29%) (cis:trans = 4:1).

¹H-NMR(40OMHz, CDCl₃) δ:
1.16 (2.4H, d, J = 6.4 Hz), 1.19 (0.6H, d, J = 6.6 Hz), 1.22-1.31 (1H, m), 2.16 (0.6H, s), 2.19 (2.4H, s), 2.52 (0.2H, ddd, J = 5.2, 7.2, 13.6 Hz), 2.66 (0.8H, ddd, J = 4.2, 8.6, 12.3 Hz), 3.34 (0.6H, s), 3.36 (2.4H, s), 3.88 (1H, brs), 4.18-4.23 (1H, m), 4.31 (0.4H, s), 4.34 (1.6H, s), 4.90 (1H, brs), 6.61-6.64 (2H, m), 7.13-7.20 (4H, m), 7.26-7.30 (1.8H, m), 7.39 (0.2H, d, J = 7.6Hz).

### Example 48

Preparation of 1-acetyl-4-[(4-ethoxycarbonylmethylphenyl)amino]-2-methyl-1,2,3,4-tetrahydroquinoline (Compound 70)

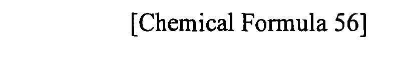

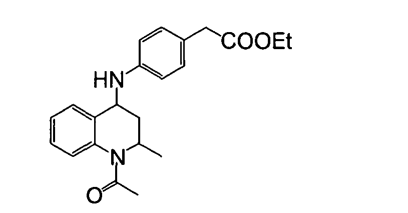

The reaction and the processing were carried out in the same manner as Example 2 to obtain the title compound as a yellow oil (106 mg, 96%) (cis:trans = 3:1).

¹H-NMR(400MHz, CDCl₃) δ:
1.16 (2.25H, d, J = 6.4 Hz), 1.19 (0.75H, d, J = 6.4 Hz), 1.23-1.28 (4H, m), 2.17 (0.75H, s), 2.18 (2.25H, s), 2.51 (0.25H, ddd, J = 5.6, 7.4, 13.4 Hz), 2.65 (0.75H, ddd, J = 4.0,8.4,12.1 Hz), 3.48 (0.5H, s), 3.51 (1.5H, s), 3.79-3.81 (1H, m), 4.10-4.26 (3.75H, m), 4.54-4.60 (0.25H, m), 4.91 (1H, brs), 6.58-6.61 (2H, m), 7.05-7.21 (4H, m), 7.28-7.32 (1.75H, m), 7.38 (0.25H, d, J = 7.1 Hz).

### Example 49

Preparation of 1-acetyl-4-[(4-carboxymethylphenyl)amino]-2-methyl-1,2,3,4-tetrahydroquinoline (Compound 71)

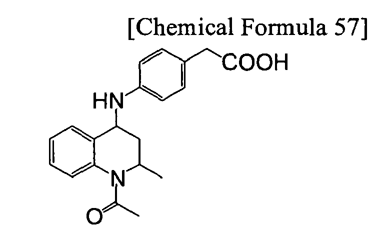

Compound 70 was processed to obtain the title compound as a yellow oil (70 mg, 84%) (cis:trans = 4:1).

¹H-NMR(400MHz, CDCl₃) δ:
1.16 (2.4H, d, J = 6.3 Hz), 1.19 (0.6H, d, J = 6.4 Hz), 1.24-1.28 (1H, m), 2.16 (0.6H, s), 2.19 (2.4H, s), 2.51 (0.2H, ddd, J = 5.1, 7.3, 13.6 Hz), 2.64 (0.8H, ddd, J = 3.2, 9.3, 11.4 Hz), 3.52 (0.4H, s), 3.57 (1.6H, s), 3.79-3.81 (1H, m), 4.20 (0.8H, dd, J = 4.2, 12.0 Hz), 4.57 (0.2H, dd, J = 4.8, 4.8 Hz), 4.91 (1H, brs), 6.60-6.63 (2H, m), 7.07-7.20 (4H, m), 7.27-7.32 (1.8H, m), 7.38 (0.2H, d, J = 7.3 Hz).

### Example 50

Preparation of 1-acetyl-2-methyl-4-[(2-morpholinophenyl)amino]-1,2,3,4-tetrahydroquinoline (Compound 72)

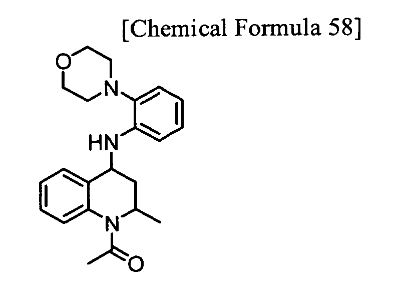

The reaction and the processing were carried out in the same manner as Example 2 to obtain the title compound as a yellow oil (42 mg, 23%) (cis:trans = 6:1).

¹H-NMR(400MHz, CDCl₃) δ:
1.18 (2.6H, d, J = 6.4 Hz), 1.22 (0.4H, d, J = 6.6 Hz), 1.29-1.38 (1H, m), 2.14 (0.4H, s), 2.19 (2.6H, s), 2.61-2.79 (1H, m), 2.86-2.90 (2H, m), 3.01-3.11 (2H, m), 3.75-3.86 (5H, m), 4.13-4.19 (0.86H, m), 4.55 (0.14H, brs), 4.86-4.99 (1H, m), 6.55 (1H, dd, J = 1.2, 8.1 Hz), 6.68-6.81 (1H, m), 6.99-7.03 (1H, m), 7.09 (1H, dd, J = 1.2, 7.8 Hz), 7.14-7.32 (4H, m).

### Example 51

Preparation of 1-acetyl-4-[(4-fluoro-3-morpholinophenyl)amino]-2-methyl-1,2,3,4-tetrahydroquinoline (Compound 73)

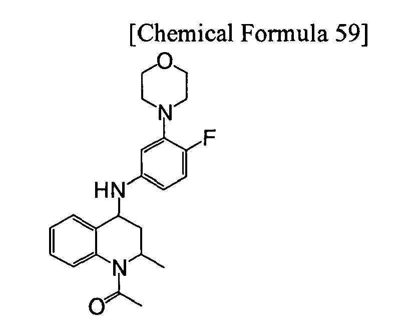

The reaction and the processing were carried out in the same manner as Example 2 to obtain the title compound as a yellow solid (80 mg, cis:trans = 5:1, 43%).

¹H-NMR(400MHz, CDCl₃) δ:
1.15-1.18 (3.6H, m), 1.39-1.66 (1.2H, m), 2.16 (0.6H, s), 2.18 (3H, s), 2.66-2.64 (1.2H, m), 2.90-3.30 (4.8H, m), 3.60-3.72 (1H, brs), 3.73-3.94 (4.8H, m), 4.10-4.20 (1.2H, brs), 4.46-4.54 (0.2H, brs), 4.65-4.96 (1.2H, brs), 6.16-6.32 (2.4H, m), 6.72-6.89 (1.2H, m), 7.23-7.42 (4.8H, m).

### Example 52

Preparation of 1-acetyl-2-methyl-4-[(1,1'-biphenyl-4-yl)amino]-1,2,3,4-tetrahydroquinoline (Compound 25)

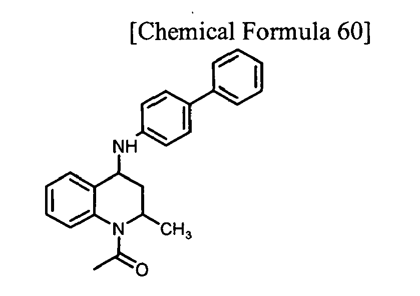

The reaction and the processing were carried out in the same manner as Example 2 to obtain the title compound (32 mg, 7%).

¹H-NMR(400MHz, CDCl₃) δ:
1.12-1.18 (2s, 3H), 1.20-1.85 (m, 1H), 2.12-2.20 (2s, 3H), 2.55-2.86 (m, 1H), 3.90 (s, 1H), 4.25-4.65 (m, 1H), 4.85-4.95 (m, 1H), 6.55-7.65 (m, 13H).

### Example 53

Preparation of 1-acetyl-6-bromo-4-[(4-chlorophenyl)amino]-2-methyl-1,2,3,4-tetrahydroquinoline (Compound 74)
The reaction and the processing were carried out in the same manner as Example 2 to obtain the title compound as a colorless oil (1.1 g, 66%) (cis:trans = 3:1).

¹H-NMR(400MHz, CDCl₃) δ:
1.15 (2.25H, d, J = 6.4 Hz), 1.20 (0.75H, d, J = 6.6 Hz), 1.22-1.34 (0.75H, m), 1.75-1.90 (0.25H, m), 2.16 (0.75H, s), 2.18 (2.25H, s), 2.38-2.47 (0.25H, m), 2.64 (0.75H, ddd, J = 4.3, 8.6, 12.6 Hz), 3.76-3.86 (1H, m), 4.07-4.16 (0.75H, m), 4.49-4.55 (0.25H, m), 4.80-4.95 (1H, m), 6.52-6.58 (2H, m), 7.00-7.18 (3H, m), 7.37-7.45 (1.75H, m), 7.54 (0.25H, d, J = 2.2 Hz).

### Example 54

Preparation of 1-acetyl-2-methyl-4-[(4-piperazinylphenyl)amino]-1,2,3,4-tetrahydroquinoline (Compound 76)
The reaction and the processing were carried out in the same manner as Example 2 to obtain the title compound as a colorless oil (42 mg, 8%).

¹H-NMR(400MHz, CDCl₃) δ:
1.16 (3H, d, J = 6.4 Hz), 1.20-1.26 (1H, m), 2.18 (3H, s), 2.64 (1H, ddd, J = 4.2, 8.7, 12.5 Hz), 2.90-3.25 (9H, m), 3.50-3.70 (1H, m), 4.10-4.20 (1H, m), 4.80-5.00 (1H, m), 6.61 (2H, d, J = 8.8 Hz), 6.86 (2H, d, J = 9.0 Hz), 7.04-7.40 (4H, m).

### Example 55

Preparation of cis-1-acetyl-4-{[4-(4-acetylpiperazinyl)phenyl]amino}-2-methyl-1,2,3,4-tetrahydroquinoline (Compound 77)
Compound 76 was acetylated according to a method well known in the art to obtain the title compound as a colorless oil (7 mg, 70%).

¹H-NMR(400MHz, CDCl₃) δ:
1.15 (3H, d, J = 6.4 Hz), 1.18-1.34 (1H, m), 2.14 (3H, s), 2.18 (3H, s), 2.64 (1H, ddd, J = 3.9, 8.5, 12.3 Hz), 2.90-3.13 (4H, m), 3.50-3.70 (5H, m), 4.10-4.22 (1H, m), 4.83-4.95 (1H, m), 6.62 (2H, d, J = 8.5 Hz), 6.80-6.95 (2H, m), 7.10-7.36 (4H, m).

### Example 56

Preparation of cis-1-acetyl-4-{[4-(4-methanesulfonylpiperazinyl)phenyl]amino}-2-methyl-1,2,3,4-tetrahydroquinoline (Compound 78)
Compound 76 was mesylated according to a method well known in the art to obtain the title compound as a colorless oil (4 mg, 33%).

¹H-NMR(400MHz, CDCl₃) δ:
1.15 (3H, d, J = 6.4 Hz), 1.18-1.30 (1H, m), 2.18 (3H, s), 2.64 (1H, ddd, J = 4.1, 8.5, 12.4 Hz), 2.82 (3H, s), 3.00-3.25 (4H, m), 3.25-3.50 (4H, m), 3.50-3.75 (1H, m), 4.20 (1H, dd, J = 4.1, 12.0 Hz), 4.80-5.00 (1H, m), 6.62 (2H, d, J = 8.8 Hz), 6.88 (2H, d, J = 8.0 Hz), 7.10-7.35 (4H, m).

### Example 57

Preparation of cis-1-acetyl-6-[(4-acetyl)piperazino]-4-[(4-chlorophenyl)amino]-2-methyl-1,2,3,4-tetrahydroquinoline (Compound 79)
The bromine atom of Compound 74 was substituted with a piperazino group according to a method well known in the art, followed by acetylation to obtain the title compound as a pale yellow oil (8 mg, 31 %).

¹H-NMR(400MHz, CDCl₃) δ:
1.14 (3H, d, J = 6.4 Hz), 1.20-1.30 (1H, m), 2.12 (3H, s), 2.16 (3H, s), 2.61 (1H, ddd, J = 4.3, 8.8, 12.6 Hz), 3.00-3.16 (4H, m), 3.53-3.60 (2H, m), 3.66-3.82 (3H, m), 4.06-4.15 (1H, m), 4.80-5.00 (1H, m), 6.54-6.60 (2H, m), 6.77-6.85 (2H, m), 7.00-7.10 (1H, m), 7.12-7.17 (2H, m).

### Example 58

Preparation of 1-acetyl-6-[(4-methanesulfonyl)piperazino]-4-[(4-chlorophenyl)amino]-2-methyl-1,2,3,4-tetrahydroquinoline (Compound 80)
The reaction and the processing were carried out in the same manner as Example 57 to obtain the title compound as a pale yellow oil (12 mg, 45%) (cis:trans = 3:1).

¹H-NMR(400MHz, CDCl₃) δ:
1.14 (2.25H, d, J = 6.4 Hz), 1.17 (0.75H, d, J = 6.6 Hz), 1.20-1.30 (0.75H, m), 1.67-1.83 (0.25H, m), 2.15 (0.75H, s), 2.16 (2.25H, s), 2.38-2.52 (0.25H, m), 2.62 (0.75H, ddd, J = 4.3, 8.8, 12.6 Hz), 2.78-2.85 (3H, m), 3.14-3.42 (8H, m), 3.60-3.90 (1H, m), 4.05-4.15 (0.75H, m), 4.46-4.51 (0.25H, m), 4.80-5.00 (1H, m), 6.53-6.66 (2H, m), 6.75-7.00 (2H, m), 7.00-7.25 (3H, m).

### Example 59

Preparation of 1-acetyl-4-[(4-chlorophenyl)amino]-6-[(4-isopropyl)piperazino]-2-methyl-1,2,3,4-tetrahydroquinoline (Compound 81)
The reaction and the processing were carried out in the same manner as Example 57 to obtain the title compound as a pale yellow oil (15 mg, 51 %) (cis:trans = 7:3).

¹H-NMR(400MHz, CDCl₃) δ:
1.12-1.30 (10H, m), 2.13 (0.9H, s), 2.15 (2.1H, s), 2.40-2.80 (6H, m), 3.06-3.28 (4H, m), 3.70-3.80 (1H, m), 3.14-3.42 (8H, m), 3.60-3.90 (1H, m), 4.06-4.18 (0.7H, m), 4.44-4.50 (0.3H, m), 4.80-5.00 (1H, m), 6.50-6.70 (2H, m), 6.70-6.95 (2H, m), 6.95-7.20 (3H, m).

### Example 60

Preparation of 1-acetyl-4-[(4-chlorophenyl)amino]-6-[(2-hydroxy)ethylamino]-2-methyl-1,2,3,4-tetrahydroquinoline (Compound 82)
The reaction and the processing were carried out in the same manner as Example 57 to obtain the title compound as a colorless oil (5 mg, 56%) (cis:trans = 3:1).

¹H-NMR(400MHz, CDCl₃) δ:
1.12 (2.25H, d, J = 6.4 Hz), 1.15 (0.75H, d, J = 6.6 Hz), 1.17-1.24 (1H, m), 2.11 (0.75H, s), 2.14 (2.25H, s), 2.42-2.53 (0.25H, m), 2.58 (0.75H, ddd, J = 4.2, 8.7, 12.5 Hz), 3.23 (1.5H, dd, J = 1.7, 5.6 Hz), 3.29 (0.5H, t, J = 5.3 Hz), 3.72-3.88 (3H, m), 4.05-4.12 (0.75H, m), 4.40-4.45 (0.25H, m), 4.80-4.95 (1H, m), 6.50-6.64 (4H, m), 6.90-6.96 (1H, m), 7.07-7.15 (2H, m).

### Example 61

Preparation of 1-acetyl-4-[(4-chlorophenyl)amino]-6-[(cis-3,5-dimethyl)morpholino]-2-methyl-1,2,3,4-tetrahydroquinoline (Compound 83)
The bromine atom of Compound 74 was substituted with an amino group according to a method well known in the art to obtain the target compound as a colorless oil (75 mg, 69%) (cis:trans = 8:3).

¹H-NMR(400MHz, CDCl₃) δ:
1.10-1.30 (10H, m), 2.14 (0.82H, s), 2.15 (2.18H, s), 2.30-2.50 (2.27H, m), 2.61 (0.73H, ddd, J = 4.2, 8.6, 12.5 Hz), 3.27-3.45 (2H, m), 3.70-3.84 (3H, m), 4.10-4.18 (0.73H, m), 4.45-4.52 (0.27H, m), 4.84-4.95 (1H, m), 6.52-6.62 (2H, m), 6.74-6.90 (2H, m), 6.98-7.18 (3H, m).

### Example 62

Preparation of 1-acetyl-4-[(4-chlorophenyl)amino]-6-[(4-isopropylcarbonyl)piperazino]-2-methyl-1,2,3,4-tetrahydroquinoline (Compound 84)
The reaction and the processing were carried out in the same manner as Example 57 to obtain the title compound as a yellow amorphous substance (45 mg, 42%) (cis:trans = 8:3).

¹H-NMR(400MHz, CDCl₃) δ:
1.10-1.18 (9H, m), 1.18-1.37 (0.73H, m), 1.64-1.83 (0.27H, m), 2.14 (1.13H, s), 2.16 (1.87H, s), 2.40-2.50 (0.27H, m), 2.56-2.66 (0.73H, m), 2.74-2.88 (1H, m), 3.00-3.24 (4H, m), 3.58-3.86 (5H, m), 4.05-4.22 (0.73H, m), 4.42-4.61 (0.27H, m), 4.78-5.97 (1H, m), 6.50-6.68 (2H, m), 6.68-6.88 (2H, m), 6.88-7.27 (3H, m).

### Example 63

Preparation of 1-acetyl-4-[(4-chlorophenyl)amino]-6-[(4-cyclohexylcarbonyl)piperazino]-2-methyl-1,2,3,4-tetrahydroquinoline (Compound 85)
The reaction and the processing were carried out in the same manner as Example 57 to obtain the title compound as a yellow amorphous substance (57 mg, 50%) (cis:trans = 7:3).

¹H-NMR(400MHz, CDCl₃) δ:
1.10-1.20 (3H, m), 1.20-1.85 (10.7H, m), 1.90-2.00 (0.3H, m), 2.14 (0.9H, s), 2.16 (2.1H, s), 2.30-2.40 (0.3H, m), 2.40-2.55 (1.3H, m), 2.55-2.66 (0.7H, m), 3.00-3.20 (4H, m), 3.55-3.80 (5H, m), 4.05-4.20 (0.7H, m), 4.46-4.58 (0.3H, m), 4.75-5.00 (1H, m), 6.57 (1.4H, d, J = 8.5 Hz), 6.65 (0.6H, d, J = 8.3 Hz), 6.70-6.95 (2H, m), 6.95-7.20 (3H, m).

### Example 64

Preparation of 1-acetyl-6-[(4-benzoyl)piperazino]-2-methyl-4-[(4-chlorophenyl)amino]-1,2,3,4-tetrahydroquinoline (Compound 86)
The reaction and the processing were carried out in the same manner as Example 57 to obtain the title compound as a yellow amorphous substance (68 mg, 55%) (cis:trans = 3:1).

¹H-NMR(400MHz, CDCl₃) δ:
1.10-1.30 (3.75H, m), 1.81-1.97 (0.25H, m), 2.14(0.75H, s), 2.16 (2.25H, s), 2.41-2.50 (0.25H, m), 2.56-2.66(0.75H, m), 2.90-3.40 (4H, m), 3.40-4.00 (4H, m), 4.05-4.20 (0.75H, m), 4.44-4.58 (0.25H, m), 4.75-5.00 (1H, m), 6.55-6.67 (2H, m), 6.70-6.95 (2H, m), 7.00-7.25 (3H, m), 7.38-7.45 (5H, m).

### Example 65

Preparation of 1-acetyl-4-[(4-chlorophenyl)amino]-6-[4-(N,N-diethylaminocarbonyl)piperazino]-2-methyl-1,2,3,4-tetrahydroquinoline (Compound 87)
The reaction and the processing were carried out in the same manner as Example 57 to obtain the title compound as a yellow amorphous substance (56 mg, 50%) (cis:trans = 3:1).

¹H-NMR(400MHz, CDCl₃) δ:
1.10-1.18 (10H, m), 2.14 (0.75H, s), 2.15 (2.25H, s), 2.40-2.50 (0.25H, m), 2.56-2.66 (0.75H, m), 3.05-3.40 (12H, m), 3.64-3.90 (1H, m), 4.06-4.22 (0.75H, m), 4.44-4.58 (0.25H, m), 4.75-5.00 (1H, m), 6.54-6.68 (2H, m), 6.68-6.98 (2H, m), 7.00-7.25 (3H, m).

### Example 66

Preparation of 1-acetyl-4-[(4-chlorophenyl)amino]-6-[4-(isopropylaminocarbonyl)piperazino]-2-methyl-1,2,3,4-tetrahydroquinoline (Compound 88)
The reaction and the processing were carried out in the same manner as Example 57 to obtain the title compound as a yellow amorphous substance (50 mg, 46%) (cis:trans = 7:3).

¹H-NMR(400MHz, CDCl₃) δ:
1.10-1.30 (9.7H, m), 1.61-1.85 (0.3H, m), 2.14 (0.9H, s), 2.16 (2.1H, s), 2.40-2.50 (0.3H, m), 2.56-2.66 (0.75H, m), 3.00-3.20 (4H, m), 3.40-3.55 (4H, m), 3.65-3.90 (1H, m), 3.94-4.04 (1H, m), 4.05-4.15 (0.75H, m), 4.15-4.30 (1H, m), 4.45-4.58 (0.25H, m), 4.80-5.00 (1H, m), 6.54-6.67 (2H, m), 6.70-6.95 (2H, m), 7.00-7.25 (3H, m).

### Example 67

Preparation of 1-acetyl-4-[(4-carboxymethylphenyl)amino]-6-morpholino-2-methyl-1,2,3,4-tetrahydroquinoline (Compound 89)
The reaction and the processing were carried out in the same manner as Example 57 to obtain the title compound as a yellow amorphous substance (27 mg) (cis:trans = 3:1).

¹H-NMR(400MHz, CDCl₃) δ:
1.10-1.30 (3.75H, m), 1.64-1.81 (0.25H, m), 2.15 (3H, s), 2.40-2.45 (0.25H, m), 2.60 (0.75H, ddd, J = 4.4, 8.8, 12.7 Hz), 3.00-3.20 (4H, m), 3.52 (0.75H, s), 3.55 (2.25H, s), 3.81 (2.25H, t, 4.8 Hz), 3.85 (0.75H, t, 4.8 Hz), 4.10-4.20 (0.75H, m), 4.46-4.54 (0.25H, m), 4.80-5.00 (1H, m), 6.55-6.65 (2H, m), 6.75-6.85 (1H, m), 6.85-6.95 (1H, m), 6.95-7.15 (3H, m).

### Example 68

Preparation of 1-acetyl-4-[(4-carbamoylmethylphenyl)amino]-2-methyl-6-morpholino-1,2,3,4-tetrahydroquinoline (Compound 90)
Compound 89 was amidated according to a method well known in the art to obtain the title compound as a pale brown solid (10 mg, 56%) (cis:trans = 3:1).

¹H-NMR(400MHz, CDCl₃) δ:
1.10-1.30 (3.75H, m), 1.64-1.81 (0.25H, m), 2.15 (0.75H, s), 2.16 (2.25H, s), 2.45-2.55 (0.25H, m), 2.62 (0.75H, ddd, J = 4.2, 8.5, 12.4 Hz), 3.05-3.20 (4H, m), 3.46 (0.75H, s), 3.49 (2.25H, s), 3.75-3.92 (5H, m), 4.12-4.22 (0.75H, m), 4.50-4.57 (0.25H, m), 4.78-5.00 (1H, m), 5.47 (2H, s), 6.65 (2H, d, J = 8.3 Hz), 6.80-6.89 (1H, m), 6.89-6.97 (1H, m), 7.02-7.14 (3H, m).

### Example 69

Preparation of 1-acetyl-6-(4-acetylpiperazinyl)-4-[(4-carboxymethylphenyl)amino]-2-methyl-1,2,3,4-tetrahydroquinoline (Compound 91)
The reaction and the processing were carried out in the same manner as Example 57 to obtain the title compound as a yellow amorphous substance (37 mg).

¹H-NMR(400MHz, CDCl₃) δ:
1.13 (3H, d, J = 6.4 Hz), 1.16-1.27 (1H, m), 2.11 (3H, s), 2.16 (3H, s), 2.63 (1H, ddd, J = 4.4, 8.7, 12.6 Hz), 2.88-2.97 (1H, m), 3.00-3.16 (3H, m), 3.44-3.72 (6H, m), 3.72 (1H, dd, J = 4.1, 12.0 Hz), 4.82-4.98 (1H, m), 6.56-6.64 (2H, m), 6.74-6.82 (2H, m), 6.98-7.06 (1H, m), 7.13 (2H, d, J = 8.5 Hz).

### Example 70

Preparation of 1-acetyl-2-methyl-4-[(4-morpholinophenyl)amino]-1,2,3,4-tetrahydroquinoline-6-carboxamide (Compound 92)
The reaction and the processing were carried out in the same manner as Example 28 to obtain the title compound as a pale yellow amorphous substance (22 mg, 44%) (cis:trans = 4:1).

¹H-NMR(400MHz, CDCl₃) δ:
1.10-1.32 (3.8H, m), 1.82-1.22 (0.2H, m), 2.15 (2.4H, s), 2.21 (0.6H, s), 2.34-2.47 (0.2H, m), 2.60-2.74 (0.8H, m), 2.83-3.20 (4H, m), 3.41-3.80 (1H, m), 3.80-3.90 (4H, m), 4.00-4.27 (0.8H, m), 4.55-4.65 (0.2H, m), 4.74-4.92 (1H, m), 5.67 (1H, brs), 6.02 (1H, brs), 6.63 (2H, brs), 6.82 (2H, brs), 7.23-7.52 (1H, m), 7.68-7.92 (2H, m).

### Example 71

Preparation of 1-acetyl-4-[(4-chlorophenyl)amino]-6-[(1-morpholino)carbonyl]-2-methyl-1,2,3,4-tetrahydroquinoline · hydrochloride (Compound 93)
Compound 42 was amidated and subsequently converted into the hydrochloride according to a method well known in the art to obtain the title compound as a pale brown powder (50 mg, 46%) (cis:trans = 3:1).

¹H-NMR(400MHz, CDCl₃) δ:
1.07-1.20 (3H, m), 1.27-1.44 (0.75H, m), 1.78-1.92 (0.25H, m), 2.22 (2.25H, s), 2.31 (0.73H, s), 2.47-2.78 (1H, m), 3.10-3.78 (9H, m), 4.10-4.34 (0.75H, m), 4.64-4.71 (0.25H, m), 4.78-4.95 (1H, m), 6.65 (1H, brs), 7.00-7.31 (4H, m), 7.31-8.00 (3H, m).

### Example 72

Preparation of cis-1-acetyl-6-[(4-acetyl)piperazino]-2-methyl-4-[(4-morpholinophenyl)amino]-1,2,3,4-tetrahydroquinoline · 3 hydrochloride (Compound 94)
The reaction and the processing were carried out in the same manner as Example 57 to obtain the title compound as a yellow amorphous substance (25 mg).

¹H-NMR(400MHz, CDCl₃) δ:
1.10-1.34 (4H, m), 2.00-2.27 (6H, m), 2.61 (1H, ddd, J = 4.1, 8.5, 12.4 Hz), 2.96-3.20 (8H, m), 3.44-3.92 (9H, m), 4.02-4.18 (1H, m), 4.80-4.96 (1H, m), 6.64 (2H, d, J = 8.8 Hz), 6.76-6.90 (3H, m), 6.90-7.10 (2H, m).

### Example 73

Preparation of 1-acetyl-6-amino-4-[(4-chlorophenyl)amino]-2-methyl-1,2,3,4-tetrahydroquinoline (Compound 95)
The bromine atom of Compound 74 was substituted with an amino group according to a method well known in the art to obtain the title compound as a pale brown powder (55 mg, 66%) (cis:trans = 8:3).

¹H-NMR(400MHz, CDCl₃) δ:
1.12 (2.18H, d, J = 6.4 Hz), 1.15 (0.82H, d, J = 6.6 Hz), 1.17-1.30 (1H, m), 2.11 (0.82H, s), 2.14 (2.18H, s), 2.40-2.51 (0.27H, m), 2.58 (0.73H, ddd, J = 4.3, 8.7, 12.5 Hz), 3.58-3.92 (3H, m), 4.00-4.14 (0.73H, m), 4.37-4.47 (0.27H, m), 4.78-5.00 (1H, m), 6.51-6.68 (4H, m), 6.81-6.98 (1H, m), 7.08-7.16(2H, m).

### Example 74

Preparation of 1-acetyl-6-acetylamino-4-[(4-chlorophenyl)amino]-2-methyl-1,2,3,4-tetrahydroquinoline (Compound 96)
Compound 95 was acetylated according to a method well known in the art to obtain the title compound as a yellow amorphous substance (20 mg, 88%) (cis:trans = 3:1).

¹H-NMR(400MHz, CDCl₃) δ:
1.13 (2.25H, d, J = 6.3 Hz), 1.17 (0.25H, d, J = 6.6 Hz), 1.20-1.30 (1H, m), 2.11 (2.25H, s), 2.13 (0.75H, s), 2.16 (0.75H, s), 2.17 (2.25H, s), 2.38-2.50 (0.25H, m), 2.60 (0.75H, ddd, J = 4.1, 8.7, 12.4 Hz), 3.85-3.95 (1H, m), 4.06-4.16 (0.75H, m), 4.45-4.55 (0.25H, m), 4.78-4.96 (1H, m), 6.50-6.57 (2H, m), 6.85-7.44 (3.75H, m), 7.58-7.81 (2H, m), 8.55-8.62 (0.25H, m).

### Example 75

Preparation of 1-acetyl-4-[(4-chlorophenyl)amino]-2-methyl-1,2,3,4-tetrahydroquinoline-6-ethylcarbamate (Compound 97)
Compound 95 was reacted and processed according to a method well known in the art to obtain the title compound as a yellow amorphous substance (26 mg, 76%) (cis:trans = 3:1).

¹H-NMR(400MHz, CDCl₃) δ:
1.14 (2.25H, d, J = 6.3 Hz), 1.17 (0.25H, d, J = 6.6 Hz), 1.20-1.35 (3.75H, m), 1.68-1.78 (0.25H, m), 2.13 (0.75H, s), 2.17 (2.25H, s), 2.43-2.52 (0.25H, m), 2.62 (0.75H, ddd, J = 4.3, 8.6, 12.6 Hz), 3.75-3.90 (1H, m),
4.07-4.27 (2.75H, m), 4.45-4.55 (0.25H, m), 4.78-5.00 (1H, m), 6.50-6.64 (3H, m), 7.04-7.18 (3H, m), 7.44-7.58 (1H, m).

### Example 76

Preparation of 1-acetyl-6-methanesulfonylamino-2-methyl-4-[(4-morpholinophenyl)amino]-1,2,3,4-tetrahydroquinoline (Compound 98)
The reaction and the processing were carried out in the same manner as Example 2 to obtain the title compound as a yellow amorphous substance (66 mg, 43%) (cis:trans = 3:1).

¹H-NMR(400MHz, CDCl₃) δ:
1.15 (2.25H, d, J = 6.4 Hz), 1.19 (0.25H, d, J = 6.6 Hz), 1.20-1.30 (0.75H, m), 1.72-1.83 (0.25H, m), 2.18 (3H, s), 2.40-2.52 (0.25H, m), 2.57-2.71 (0.75H, m), 2.96 (2.25H, s), 2.98 (0.75H, s), 2.98-3.19 (4H, m), 3.50-3.72 (1H, m), 3.78-3.92 (4H, m), 4.00-4.20 (0.75H, m), 4.46-4.58 (0.75H, m), 4.72-5.00 (1H, m), 6.50-6.70 (3H, m), 6.70-6.93 (2H, m), 7.04-7.30 (3H, m).

### Example 77

Preparation of 1-acetyl-6-methanesulfonylamino-2-methyl-4-[(4-ethoxycarbonylmethylphenyl)amino]-1,2,3,4-tetrahydroquinoline (Compound 99)
The reaction and the processing were carried out in the same manner as Example 2 to obtain the title compound as a yellow amorphous substance (100 mg, 21%) (cis:trans = 3:1).

¹H-NMR(400MHz, CDCl₃) δ:
1.00-1.34 (6.75H, m), 2.75-2.88 (0.25H, m), 2.18 (2.25H, s), 2.19 (0.75H, s), 2.41-2.51 (0.25H, m), 2.65 (0.75H, ddd, J = 4.2, 8.7, 12.5 Hz), 2.93 (2.25H, s), 2.98 (0.75H, s), 3.48 (0.75H, s), 3.50 (2.25H, s), 3.76-3.96 (1H, m), 4.08-4.20 (2.75H, m), 4.51-4.64 (0.25H, m), 4.72-4.98 (1H, m), 6.47-6.78 (3H, m), 7.00-7.41 (4H, m).

### Example 78

Preparation of 1-acetyl-4-[(4-fluorophenyl)amino]-2-methyl-1,2,3,4-tetrahydroquinoline-6-carboxylic acid (Compound 100)
The reaction and the processing were carried out in the same manner as Example 27 to obtain the title compound as a white solid (126 mg, 58%) (cis:trans = 6:1).

¹H-NMR(400MHz, CDCl₃) δ:
1.17 (2.6H, d, J = 6.4 Hz), 1.22 (0.4H, d, J = 6.6 Hz), 1.23-1.31 (1H, m), 2.22 (0.4H, s), 2.23 (2.6H, s), 2.48-2.54 (0.14H, m), 2.64-2.74 (0.86H, m), 4.10-4.28 (2H, m), 4.55-4.64 (0.14H, m), 4.84-4.94 (0.86H, m), 6.55-6.62 (2H, m), 6.82-6.93 (2H, m), 7.24-7.30 (1H, m), 7.99-8.13 (2H, m).

### Example 79

Preparation of ethyl 1-acetyl-4-[(4-methanesulfonylaminophenyl)amino]-2-methyl-1,2,3,4-tetrahydroquinoline-6-carboxylate (Compound 101)
The reaction and the processing were carried out in the same manner as Example 26 to obtain the title compound as a pale yellow oil (251 mg, 79%) (cis:trans = 6:1).

¹H-NMR(400MHz, CDCl₃) δ:
1.18 (2.6H, d, J = 6.4 Hz), 1.22 (0.4H, d, J = 6.6 Hz), 1.33-1.42 (4H, m), 2.22 (3H, m), 2.44-2.53 (0.14H, m), 2.69 (0.86H, ddd, J = 4.2, 8.6,12.5 Hz), 2.93 (0.4H, s), 2.95 (2.6H, s), 4.20-4.26 (0.86H, m), 4.30-4.40 (2.14H, m), 4.62-4.66 (0.14H, m), 4.83-4.92 (0.86H, m), 6.64 (2H, d, J = 8.8 Hz), 7.10-7.16 (2H, m), 7.22-7.25 (1H, m), 7.94-8.06 (2H, m).

### Example 80

Preparation of cis-1-acetyl-4-[(4-methanesulfonylaminophenyl)amino]-2-methyl-1,2,3,4-tetrahydroquinoline-6-carboxamide (Compound 102)
The reaction and the processing were carried out in the same manner as Example 28 to obtain the title compound as a pale yellow oil (32 mg, 35%).

¹H-NMR(400MHz, CDCl₃) δ:
1.18 (3H, d, J = 6.4 Hz), 1.23-1.33 (1H, m), 2.22 (3H, s), 2.69 (1H, ddd, J = 4.2, 8.6, 12.6 Hz), 2.95 (3H, s), 3.92-3.96 (1H, m), 4.16-4.24 (1H, m), 4.83-4.94 (1H, m), 5.61 (1H, brs), 5.98 (1H, brs), 6.62 (2H, d, J = 8.8 Hz), 7.12 (2H, d, J = 8.8 Hz), 7.22-7.26 (1H, m), 7.74-7.79 (2H, m).

### Example 81

Preparation of ethyl 1-acetyl-4-[(4-cyanomethylphenyl)amino]-2-methyl-1,2,3,4-tetrahydroquinoline-6-carboxylate (Compound 103)
The reaction and the processing were carried out in the same manner as Example 2 to obtain the title compound as a pale yellow oil (250 mg, 64%) (cis:trans = 4:1).

¹H-NMR(400MHz, CDCl₃) δ:
1.18 (2.4H, d, J = 6.3 Hz), 1.21-1.45 (4.6H, m), 2.21 (0.6H, s), 2.22 (2.4H, s), 2.44-2.54 (0.2H, m), 2.69 (0.8H, ddd, J = 4.1, 8.4, 12.5 Hz), 3.61-6.68 (3H, m), 4.26-4.42 (3H, m), 4.61-4.68 (0.2H, m),4.83-4.94 (0.8H, m), 6.63-6.70 (2H, m), 7.08-7.29 (3H, m), 7.97-8.16 (2H, m).

### Example 82

Preparation of 1-acetyl-4-[(4-cyanomethylphenyl)amino]-2-methyl-1,2,3,4-tetrahydroquinoline-6-carboxamide (Compound 104)
The reaction and the processing were carried out in the same manner as Example 28 to obtain the title compound as a pale yellow oil (8 mg, 80%) (cis:trans = 5:1).

¹H-NMR(400MHz, CDCl₃) δ:
1.17 (2.5H, d, J = 6.4 Hz), 1.23-1.36 (1.5H, m), 2.21 (3H, brs), 2.38-2.46 (0.17H, m), 2.68 (0.83H, ddd, J = 4.0, 8.4, 12.3 Hz), 3.61 (0.33H, s), 3.64 (1.67H, s), 4.04-4.33 (2H, m), 4.67 (0.17H, brs), 4.85 (0.83H, brs), 5.93 (1H, brs), 6.14 (1H, brs), 6.60-6.68 (2H, m), 7.09-7.14 (2H, m), 7.24-7.26 (1H, m), 7.73-7.80 (2H, m).

### Example 83

Preparation of cis-1-acetyl-4-[(4-carboxymethylphenyl)amino]-2-methyl-1,2,3,4-tetrahydroquinoline-6-carboxamide (Compound 105)
Compound 104 was hydrolyzed according to a method well known in the art to obtain the title compound as a yellowish brown oil (14 mg, 52%).

¹H-NMR(400MHz, CDCl₃) δ:
1.18-1.35 (4H, m), 2.24 (3H, s), 2.69 (1H, ddd, J = 4.1, 8.4, 12.4 Hz), 3.65 (2H, s), 4.22-4.36 (2H, m), 4.83-4.93 (1H, m), 6.64 (2H, d, J = 8.4 Hz), 7.14 (2H, d, J = 8.4 Hz), 7.23-7.27 (1H, m), 8.03-8.08 (2H, m).

### Example 84

Preparation of cis-1-acetyl-4-[(4-carbamoylmethylphenyl)aminol-2-methyl-1,2,3,4-tetrahydroquinoline-6-carboxamide (Compound 106)
Compound 105 was amidated according to a method well known in the art to obtain the title compound as a yellowish brown oil (2.5 mg, 25%).

¹H-NMR(400MHz, CDCl₃) δ:
1.18 (3H, d, J = 6.4 Hz), 1.24-1.38 (1H, m), 2.22 (3H, s),
2.69 (1H, ddd, J = 4.2, 8.4, 12.5 Hz), 3.65 (2H, s), 3.93-3.96 (1H, m), 4.18-4.27 (1H, m), 4.83-4.93 (1H, m), 6.63 (2H, d, J = 6.3 Hz), 7.15 (2H, d, J = 6.3 Hz), 7.23-7.27 (1H, m), 7.70-7.83 (2H, m).

### Example 85

Preparation of 1-acetyl-4-[(4-chlorophenyl)amino]-7-methanesulfonylamino-2-methyl-1,2,3,4-tetrahydroquinoline · hydrochloride (Compound 107)
The reaction and processing were carried out in the same manner as Example 2. The resulting compound was converted into a hydrochloride according to a method well known in the art, and recrystallized from ethyl acetate to obtain the title compound as a pale yellow crystalline powder (15 mg, 15%) (cis:trans = 6:1).
Melting point 105.0-106.0°C

¹H-NMR(400MHz, CD₃OD) δ:
1.13-1.26 (3.86H, m), 1.85-1.96 (0.14H, m), 2.20(0.42H, s), 2.25 (2.58H, s), 2.40-2.47 (0.14H, m), 2.72 (0.86H, ddd, J = 3.9, 8.3, 14.8 Hz), 2.96-2.98(3H, m), 4.49-4.52 (0.14H, m), 4.80-4.98 (1H, m), 5.26-5.34 (0.86H, m), 6.84 (0.28H, d, J = 8.8 Hz), 7.03 (1.72H, d, J = 8.8 Hz), 7.19-7.50 (5H, m).

### Example 86

Preparation of 1-acetyl-4-[(4-hydroxy-3-methoxycarbonylphenyl)amino]-2-methyl-1,2,3,4-tetrahydroquinoline · hydrochloride (Compound 108)
The reaction and processing were carried out in the same manner as Example 2.
The resulting compound was converted into a hydrochloride according to a method well known in the art to obtain title compound as a white crystalline powder (30 mg, 75%) (cis:trans = 3:1).
Melting point 94.0-96.0°C

¹H-NMR(400MHz, CDCl₃) δ: 1.09-1.28 (3.75H, m), 1.70-1.75 (0.25H, m), 2.16 (0.75H, s), 2.19 (2.25H, s), 2.50-2.59 (0.25H, m), 2.65 (0.75H, ddd, J = 4.2, 8.4, 12.4 Hz), 3.46-3.70 (1H, brs), 3.91 (2.75H, s), 3.93 (0.25H, s), 4.18 (0.75H, dd, J = 3.9, 11.8 Hz), 4.51 (0.25H, dd, J = 4.4, 4.4 Hz), 4.82-4.97 (1H, m), 6.81-7.37 (7H, m), 10.18 (0.25H, s), 10.22 (0.75H, s).

### Example 87

Preparation of cis-1-acetyl-4-[(2-carboxyphenyl)amino]-2-methyl-1,2,3,4-tetrahydroquinoline (Compound 109)
The reaction and the processing were carried out in the same manner as Example 2 to obtain the title compound as a white solid (7 mg, 28%).
Melting point 122.0-122.7°C

¹H-NMR(400MHz, CDCl₃) δ:
1.19 (3H, dd, 6.4 Hz), 1.37-1.51 (1H, m), 2.22 (1H, s), 2.73 (1H, ddd, J = 4.4, 8.3, 12.4 Hz), 4.24-4.40 (1H, brs), 4.86-5.04 (1H, brs), 6.61-6.71 (2H, m), 7.17-7.53 (4H, m), 8.00-8.25 (2H, m).

### Example 88

Preparation of 1-acetyl-6-[cis-2,6-dimethylmorpholin-4-yl]-4-[(4-methanesulfonylaminophenyl)amino]-2-methyl-1,2,3,4-tetrahydroquinoline · 2 hydrochloride (Compound 110)
The reaction and processing were carried out in the same manner as Example 61. The resulting compound was converted into a hydrochloride according to a method well known in the art, and washed with ethyl acetate to obtain the title compound as a reddish brown solid (56 mg, 45%) (cis:trans = 10:1).
Melting point 141.5-143.2°C

¹H-NMR(400MHz, CD₃OD) δ:
1.13-1.35 (9.91H, m,), 1.65-1.71 (0.09H, brs), 2.21-2.24 (3H, m), 2.53-2.75 (1H, m), 2.89 (0.91 H, s), 3.00 (0.09H, s), 3.12-3.63 (4H, m), 3.94-4.02 (2H, m), 4.26-4.34 (0.91H, brs), 4.83-4.93 (1.09H, m), 6.78-6.81 (2H, m), 7.12-7.14 (2H, m), 7.28-7.56 (3H, m).

### Example 89

Preparation of 1-acetyl-6-[(4-isopropylcarbonyl)piperazino]-4-[(4-methanesulfonylaminophenyl)amino]-2-methyl-1,2,3,4-tetrahydroquinoline (Compound 111)
The reaction and the processing were carried out in the same manner as Example 62 to obtain the title compound as a pale yellow amorphous substance (8 mg, 67%)(cis:trans= 10:1).

¹H-NMR(400MHz, CDCl₃) δ:
0.96-1.25 (9.91H, m), 1.50-1.75 (0.09H, brs), 2.03 (0.27H, s), 2.15 (2.73H, s), 2.45-2.65 (1H, m), 2.80 (1H, m), 2.93 (0.27H, s), 2.95 (2.73H, s), 3.08-3.20 (4H, brs), 3.55-3.80 (4H, brs), 4.06-4.18 (0.91H, brs), 4.49-4.53 (0.09H, m), 4.80-4.98 (0.91H, m), 5.11-5.13 (0.09H, m), 6.30 (0.09H, s), 6.35 (0.91H, s), 6.60-6.64 (2H, m), 6.79-6.86 (2H, m), 7.00-7.13 (3H, m).

### Example 90

Preparation of cis-1-acetyl-4-[(4-benzylphenyl)amino]-2-methyl-1,2,3,4-tetrahydroquinoline (Compound 112)
The reaction and the processing were carried out in the same manner as Example 2 to obtain the title compound as a white solid (10 mg, 8%).

¹H-NMR(400MHz, CDCl₃) δ: 1.16 (3H, d, J = 6.6 Hz), 1.65-1.68 (1H, m), 2.10 (3H, s), 2.22 (1H, ddd, J = 5.6, 7.5, 13.5 Hz), 3.77 (2H, dd, J = 15.6, 19.8 Hz), 4.49 (1H, brs), 4.82 (1H, brs), 6.73 (1H, t, J = 6.8 Hz), 6.80 (1H, d, J = 8.3 Hz), 7.06 (1H, d, J = 7.3 Hz), 7.09-7.26 (9H, m).

### Example 91

Preparation of 1-acetyl-4-[(4-chlorophenyl)amino]-N,N,2-trimethyl-1,2,3,4-tetrahydroquinoline-6-carboxamide (Compound 113)
Compound 41 was hydrolyzed and processed according to a method well known in the art to obtain the title compound as a pale yellow solid (74 mg, 64%) (cis:trans = 3:1).

¹HNMR(400MHz, CDCl₃) δ:
1.16 (2.25H, d, J = 6.4 Hz), 1.21 (0.75H, d, J = 6.6 Hz), 1.23-1.34 (0.75H, m), 1.80-1.90 (0.25H, m), 2.20 (0.75H, s), 2.22 (2.25H, s), 2.45 (0.25H, ddd, J = 6.6, 6.6, 13.4 Hz), 2.66 (0.75H, ddd, J = 4.0, 8.6, 12.4 Hz), 2.91 (2.25H, s), 3.00 (0.75H, s), 3.07 (2.25H, s), 3.11 (0.75H, s), 3.89 (1H, brs), 4.13-4.22 (0.75H, m), 4.54-4.60 (0.25H, m), 4.87 (1H, brs), 6.52-6.58 (2H, m), 7.08-7.16 (2H, m), 7.18-7.41 (2.75H, m), 7.49 (0.25H, d, J = 1.7 Hz).

### Example 92

Preparation of 1-acetyl-4-[(4-chlorophenyl)amino]-N,2-dimethyl-1,2,3,4-tetrahydroquinoline-6-carboxamide (Compound 114)
Compound 42 was hydrolyzed and amidated according to a method well known in the art to obtain the title compound as a pale yellow amorphous substance (71 mg, 64%) (cis:trans = 3:1).

¹H-NMR(400MHz, CDCl₃) δ:
1.16 (2.25H, d, J = 6.4 Hz), 1.21 (0.75H, d, J = 6.6 Hz), 1.23-1.33 (0.75H, m), 1.83-1.92 (0.25H, m), 2.20 (0.75H, s), 2.20 (2.25H, s), 2.42 (0.25H, ddd, J = 7.2, 7.2, 14.4 Hz), 2.67 (0.75H, ddd, J = 4.0, 8.4, 12.3 Hz), 2.98 (2.25H, d, J = 4.6 Hz), 3.02 (0.75H, d, J = 4.9 Hz), 3.86 (1H, brs), 4.02-4.20 (0.75H, m), 4.59-4.64 (0.25H, m), 4.80-4.92 (1H, m), 6.01 (0.75H, brs), 6.09(0.25H, brs), 6.54-6.60 (2H, m), 7.10-7.35 (3H, m), 7.63(0.75H, s), 7.66 (0.25H, dd, J = 2.3, 8.4 Hz), 7.74 (0.75H, dd, J = 2.0, 8.1 Hz), 7.85 (0.25H, d, J = 2.0 Hz).

### Example 93

Preparation of 1-acetyl-4-[(4-chlorophenyl)amino]-2-methyl-1,2,3,4-tetrahydroquinoline-6-carbohydrazide (Compound 115)
Compound 42 was amidated and processed according to a method well known in the art to obtain the title compound as a pale brown amorphous substance (136 mg, 61 %) (cis:trans = 3:1).

¹H-NMR(400MHz, CDCl₃) δ:
1.17 (2.25H, d, J = 6.3 Hz), 1.22 (0.75H, d, J = 6.6 Hz), 1.24-1.34 (0.75H, m), 1.84-1.94 (0.25H, m), 2.21 (0.75H, s), 2.21 (2.25H, s), 2.42 (0.25H, ddd, J = 6.7, 6.7, 13,7 Hz), 2.68 (0.75H, ddd, J = 4.0, 8.3, 12.6 Hz), 3.87 (1H, d, J = 7.1 Hz), 4.07 (2H, brs), 4.12-4.21 (0.75H, m), 4.58-4.66 (0.25H, m), 4.79-4.93 (1H, m), 6.53-6.59 (2H, m), 7.10-7.17 (2H, m), 7.23-7.39 (1H, m), 7.61-7.66 (1H, m), 7.72 (0.75H, dd, J = 2.0, 8.1 Hz), 7.84 (0.25H, d, J = 2.0 Hz).

### Example 94

Preparation of 1-acetyl-4-[(4-chlorophenyl)amino]-2-methyl-6-[1,3,4-oxadiazol-2(3H)-on-5-yl]-1,2,3,4-tetrahydroquinoline (Compound 116)
To the THF (10 mL) solution of Compound 115 (100 mg, 267 µmol), triethylamine (744 µL, 534 µmol) was added at room temperature. 1,1'-Carbodiimidazole (64.9 mg, 400 µmol) was further added thereto and the mixture was stirred for 3 days at the same temperature. The reaction solution was added with water and extracted with chloroform. The organic layer was washed with saturated brine, dried over anhydrous sodium sulfate, and then concentrated under reduced pressure. The resulting residue was purified by silica gel chromatography (hexane:ethyl acetate = 1:1→1:2) to obtain the title compound as a pale yellow amorphous substance (94.8 mg, 89%) (cis:trans = 3:1).

¹H-NMR(400MHz, CDCl₃) δ:
1.19 (2.25H, d, J = 6.4 Hz), 1.25 (0.75H, d, J = 6.6 Hz), 1.27-1.37 (0.75H, m), 1.86-1.94 (0.25H, m), 2.24 (3H, s), 2.40-2.50 (0.25H, m), 2.69 (0.75H, ddd, J = 3.9, 8.4, 12.3 Hz), 3.86 (0.75H, d, J = 7.1 Hz), 3.98-4.10 (0.25H, m), 4.14-4.23 (0.75H, m), 4.57-4.65 (0.25H, m), 4.78-4.92 (1H, m), 6.54-6.61 (2H, m), 7.10-7.18 (2H, m).

### Example 95

Preparation of 1-acetyl-4-[(4-chlorophenyl)amino]-6-cyano-2-methyl-1,2,3,4-tetrahydroquinoline (Compound 117)
Compound 43 was reacted and processed according to a method well known in the art, and then recrystallized from ethyl acetate and hexane to obtain the title compound as a white solid (40 mg, 66%).
Melting point 211.5-212.8°C

¹H-NMR(400MHz, CDCl₃) δ:
1.20 (3H, d, J = 6.4 Hz), 1.30-1.39 (1H, m), 2.05 (3H, s), 2.69 (1H, ddd, J = 4.1, 9.9, 13.1 Hz), 3.84 (1H, d, J = 6.4 Hz), 4.08-4.22 (1H, m), 4.78-4.85 (1H, m), 6.54 (2H, d, J = 8.0 Hz), 7.14 (2H, d, J = 8.0 Hz), 7.28 (2H, d, J = 12.2 Hz), 7.57 (1H, s), 7.61 (1H, d, J = 12.2 Hz).

### Example 96

Preparation of 1-acetyl-4-[(4-chlorophenyl)amino]-N-methoxy-2-methyl-1,2,3,4-tetrahydroquinoline-6-carboxamide · hydrochloride (Compound 118)
Compound 42 was reacted and processed according to a method well known in the art, and then the hydrochloride was washed with diethyl ether and ethyl acetate to obtain the title compound as a pale yellow solid (73 mg, 44%) (cis:trans = 3:1).
Melting point 120.8-123.0°C

¹H-NMR(400MHz, CD₃OD) δ:
1.10-1.40 (3.75H, m), 2.00-2.06 (0.25H, m), 2.20 (2.25H, s), 2.27 (0.75H, s), 2.54-2.70 (1H, m), 3.80 (2.25H, s), 3.87 (0.75H, s), 4.39 (0.75H, dd, J = 4.1, 12.1 Hz), 4.74-4.89 (1H, m), 5.04-5.09 (0.25H, m), 6.91-6.96 (2H, m), 7.23-8.11 (5H, m).

### Example 97

Preparation of 1-acetyl-4-[(4-chlorophenyl)amino]-2-methyl-6-(1H-tetrazol-5-yl)-1,2,3,4-tetrahydroquinoline (Compound 119)
Compound 117 was reacted and processed according to a method well known in the art to obtain the title compound as a yellow solid (39 mg, 70%) (cis:trans = 10:1).
Melting point 190.5-191.1°C

¹H-NMR(400MHz, CDCl₃) δ:
1.08-1.37 (3.91H, m), 1.27-1.50 (0.09H, m), 2.20-2.42 (3H, brs), 2.62-2.78 (1H, m), 4.16-4.32 (0.91H, brs), 4.50-4.54 (0.09H, m), 4.60-4.92 (1H, m), 6.56 (2H, d, J = 8.8 Hz), 7.04 (2H, d, J = 8.8 Hz), 7.32-7.38 (1H, brs), 8.00-8.24 (2H, m).

### Example 98

Preparation of 1-acetyl-4-[(4-chlorophenyl)amino]-2-methyl-6-[1,2,4-oxadiazol-5(2H)-on-3-yl]-1,2,3,4-tetrahydroquinoline (Compound 120)
Compound 117 was reacted and processed according to a method well known in the art to obtain the title compound as a white solid (40 mg, 54%) (cis:trans = 7:1).
Melting point 163.2-164.0°C

¹H-NMR(400MHz, CDCl₃) δ:
1.15-1.36 (3.87H, m), 1.55-1.62 (0.13H, m), 2.04 (0.39H, s), 2.16 (2.61H, s), 2.61-2.75 (1H, m), 4.14-4.22 (0.87H, brs), 4.36-4.44 (0.13H, m), 4.67-4.91 (1H, m), 6.56-6.61 (2H, m), 7.13-7.17 (2H, m), 7.30-7.32 (1H, brs), 7.67 (1H,s), 7.75 (1H, d, J = 8.5 Hz).

### Example 99

Preparation of cis-1-acetyl-4-[(4-chlorophenyl)amino]-6-hydroxymethyl-2-methyl-1,2,3,4-tetrahydroquinoline (Compound 121)
Compound 41 was reduced and processed according to a method well known in the art to obtain the title compound as a clear and colorless oil (11 mg, 32%).

¹H-NMR(400MHz, CDCl₃) δ:
1.15 (3H, d, J = 6.4 Hz), 1.19-1.30 (1H, m), 1.82 (1H, brs), 2.64 (1H, ddd, J = 4.0, 8.6, 12.3 Hz), 3.85 (1H, brs), 4.12-4.20 (1H, m), 4.65 (2H, s), 4.90 (1H, brs), 6.56 (2H, d, J = 9.0 Hz), 7.12-7.17 (3H, m), 7.24-7.34(2H, m).

### Example 100

Preparation of 1-acetyl-4-[(4-ethoxycarbonylmethylphenyl)amino]-6-fluoro-2-methyl-1,2,3,4-tetrahydroquinoline (Compound 122)
The reaction and the processing were carried out in the same manner as Example 48 to obtain the title compound as a clear and colorless oil (453 mg, 39%) (cis:trans = 3:1).

¹H-NMR(400MHz, CDCl₃) δ:
1.14 (2.25H, d, J = 6.3 Hz), 1.19 (0.75H, d, J = 6.6 Hz), 1.25 (0.75H, t, J = 7.3 Hz), 1.25 (2.25H, t, J = 7.1 Hz), 1.22-1.29 (0.75H, m), 1.72-1.87 (0.25H, m), 2.15 (0.75H, s), 2.17 (2.25H, s), 2.40-2.51 (0.25H, m), 2.65 (0.75H, ddd, J = 4.2, 8.6, 12.5 Hz), 3.49 (0.5H, s), 3.51 (1.5H, s), 3.72-3.88 (1H, m), 4.10-4.18 (2.75H, m), 4.52-4.59 (0.25H, m), 4.80-4.98 (1H, m), 6.58 (1.5H, d, J = 8.6 Hz), 6.60 (0.5H, d, J = 8.1 Hz), 6.93-7.15 (5H, m).

### Example 101

Preparation of 1-acetyl-4-[(4-carboxymethylphenyl)amino]-6-fluoro-2-methyl-1,2,3,4-tetrahydroquinoline (Compound 123)
Compound 122 was reacted and processed in the same manner as Example 49 to obtain the title compound as a pale green amorphous substance (342 mg, 100%). (cis:trans = 3:1).

¹H-NMR(400MHz, CDCl₃) δ:
1.14 (2.25H, d, J = 6.4 Hz), 1.18 (0.75H, d, J = 6.6 Hz), 1.20-1.30 (0.75H, m), 1.68-1.84 (0.25H, m), 2.14 (0.75H, s), 2.17 (2.25H, s), 2.42-2.50 (0.25H, m), 2.64 (0.75H, ddd, J = 4.0, 8.4, 12.3 Hz), 3.53 (0.5H, s), 3.56 (1.5H, s), 4.14 (0.75H, dd, J = 4.0, 12.1 Hz), 4.53-4.57 (0.25H, m), 4.80-5.00 (1H, m), 6.60 (1.5H, d, J = 8.6 Hz), 6.60 (0.5H, d, J = 8.8 Hz), 6.94-7.30 (5H, m).

### Example 102

Preparation of 1-acetyl-4-[(4-carbamoylmethylphenyl)amino]-6-fluoro-2-methyl-1,2,3,4-tetrahydroquinoline (Compound 124)
Compound 123 was reacted and processed according to a method well known in the art to obtain the title compound as a pale yellow amorphous substance (64 mg, 80%) (cis:trans = 3:1).

¹H-NMR(400MHz, CDCl₃) δ:
1.15 (2.25H, d, J = 6.4 Hz), 1.19 (0.75H, d, J = 6.6 Hz), 1.21-1.32 (0.75H, m), 1.80-1.94 (0.25H, m), 2.15 (0.75H, s), 2.17 (2.25H, s), 2.41-2.53 (0.25H, m), 2.66 (0.75H, ddd, J = 4.1, 8.5, 12.4 Hz), 3.46 (0.5H, s), 3.49 (1.5H, s), 3.82-3.96 (1H, m), 4.10-4.21 (0.75H, m), 4.43-4.62 (0.25H, m), 4.82-5.02 (1H, m), 5.45(2H, brs), 6.59-6.66 (2H, m), 6.96-7.17 (5H, m).

### Example 103

Preparation of 1-acetyl-4-[4-(N,N-dimethylaminocarbonylmethyl)phenylamino]-6-fluoro-2-methyl-1,2,3,4-tetrahydroquinoline (Compound 125)
Compound 123 was reacted and processed in the same manner as Example 102 to obtain the title compound as a pale yellow amorphous substance (45 mg, 53%) (cis:trans = 5:1).

¹H-NMR(400MHz, CDCl₃) δ:
1.14 (2.52H, d, J = 6.4 Hz), 1.18 (0.48H, d, J = 6.4 Hz), 1.20-1.30 (0.84H, m), 1.70-1.84 (0.16H, m), 2.15 (0.48H, s), 2.17 (2.52H, s), 2.40-2.50 (0.16H, m), 2.64 (0.84H, ddd, J = 4.0, 8.4, 12.3 Hz), 2.95 (0.48H, s), 2.96 (2.52H, s), 3.00 (0.48H, s), 3.01 (2.52H, s), 3.59 (0.32H, s), 3.62 (1.68H, s), 3.70-3.86 (1H, m), 4.09-4.18 (0.84H, m), 4.52-4.58 (0.16H, m), 4.80-4.95 (1H, m), 6.56-6.61 (2H, m), 6.94-7.02 (1H, m), 7.03-7.15 (4H, m).

### [Experimental example]

Compounds 1, 2 and 3 used in the Test example were obtained from ChemBridge and Compound 47 was obtained from Princeton Biomolecular Research. Inc. Further, Compounds 13 and 75 were prepared according to the method described in the document (pamphlet of WO2002/79165) and then used, and Compounds 5, 8, 9, 10, 11, 12, 15, 16, 17, 21, 46, 48, 49, 50, 51 and 52 were prepared according to the method described in the document (i.e., JP-A No. 2002-053557) and then used. Others were prepared according to the method described in the Examples, and then used.

### Experimental example 1

### <Materials and Methods>

HepG2 cells originating from human liver cancer were seeded at a density of 1×10⁴ cells per well on a 24-well plate by using minimum essential medium (MEM, manufactured by Sigma) comprising 10% bovine fetal serum. Next day, the medium was exchanged with Dulbecco's modified eagle medium (DMEM, manufactured by Sigma) comprising 10% bovine fetal serum lacking phenol red. Then, the test compound dissolved in DMSO was added thereto to obtain the final concentration of 3 µM or 10 µM while having 400 µl of the medium per well. The cells were incubated for 48 hrs in a CO₂ incubator which had been adjusted to have oxygen concentration of 4%, followed by collecting the culture supernatant. The EPO concentration in the culture supernatant was immediately measured by using EPO ELISA kit (manufactured by Roche Diagnostics K.K.). Specific procedures were carried out according to the manufacturer's protocol included in the kit.
When EPO production amount in non-stimulated state, i.e., without addition of any compound, was 100%, the EPO production amount induced by each compound was calculated (% of control). The results are summarized in Table 17.

**[Table 17]**

| Compound No. | % of control (3 µM) | | Compound No. | % of control (3 µM) |
|---|---|---|---|---|
| 1 | 235 | | 49 | 271 |
| 2 | 167 | | 50 | 289 |
| 3 | 187 | | 51 | 239 |
| 4 | 163 | | 52 | 314 |
| 5 | 205 | | 53 | 134 |
| 6 | 225 | | 54 | 148 |
| 7 | 261 | | 55 | 134 |
| 8 | 248 | | 56 | 213 |
| 9 | 405 | | 57 | 251 |
| 10 | 172 | | 62 | 193 |
| 11 | 163 | | 65 | 414 |
| 12 | 197 | | 69 | 150 |
| 13 | 229 | | 70 | 165 |
| 14 | 257 | | 71 | 149 |
| 15 | 348 | | 72 | 243 |
| 16 | 288 | | 74 | 398 |
| 17 | 172 | | 75 | 346 |
| 18 | 178 | | 76 | 227 |
| 19 | 191 | | 77 | 221 |
| 20 | 174 | | 78 | 217 |
| 21 | 231 | | 79 | 476 |
| 22 | 150 | | 80 | 219 |
| 23 | 135 | | 81 | 234 |
| 24 | 117 | | 85 | 147 |
| 25 | 242 | | 92 | 192 |
| 26 | 214 | | 95 | 213 |
| 27 | 292 | | 97 | 264 |
| 28 | 167 | | 103 | 108 |
| 29 | 305 | | 104 | 204 |
| 30 | 339 | | 105 | 245 |
| 31 | 302 | | 107 | 130 |
| 34 | 288 | | 110 | 207 |
| 36 | 114 | | 111 | 126 |
| 38 | 201 | | 113 | 112 |
| 39 | 131 | | 114 | 198 |
| 40 | 144 | | 117 | 204 |
| 41 | 119 | | 118 | 138 |
| 42 | 102 | | 119 | 125 |
| 43 | 452 | | 121 | 222 |
| 44 | 301 | | 122 | 139 |
| 45 | 286 | | 123 | 142 |
| 46 | 391 | | 124 | 147 |
| 47 | 292 | | 125 | 148 |

### <Results>

When the test compound was added with the final concentration of 3 µM, EPO production was accelerated as much as 476% (Compound 79) (see, Table 17). Furthermore, Compound 42 which did not have any accelerated EPO production at 3 µM showed 138% EPO production acceleration when it was added with the final concentration of 10 µM. Thus, it is clear that these compounds have an activity of accelerating EPO production, and therefore are useful as a therapeutic agent for anemia.

### Experimental example 2

### <Materials and Methods>

K562 cells (obtained from ATCC), which are human proerythroblast cells, were seeded at a density of 1×10⁵ cells/1 mL on a 24-well plate by using complete medium (RPMI-1640 medium comprising 10% bovine fetal serum). After adding the test compound having 1000x concentration (1 µL), the cells were incubated for three days in a CO₂ incubator (37°C, 5% CO₂). After exchanging the medium, the cells were further incubated for three days. Then, the cells were collected and counted. After adjusting them to have 3×10⁵ cells, the amount of hemoglobin produced in the cells was determined by measuring fluorescence of the porphyrin ring. Specifically, the cells which had been collected by centrifugation were suspended in 2 M oxalic acid solution (500 µL) and boiled for 30 minutes under heating. After cooling the cells, the fluorescence intensity was measured by Fluorescence micro plate reader (Spectra MAX GeminiEM, manufactured by Molecular Devices Corporation) (Em: 400 nm, Ex: 603 nm). When hemoglobin amount in non-stimulated state, i.e., without addition of any compound, was 100%, the production amount of hemoglobin induced by each compound was calculated (% of control). The results are summarized in Table 18 and Table 19.

**[Table 18]**

| Compound No. | % of control (6 µM) | | Compound No. | % of control (6 µM) |
|---|---|---|---|---|
| 1 | 575 | | 45 | 387 |
| 3 | 912 | | 46 | 326 |
| 4 | 92 | | 47 | 701 |
| 5 | 245 | | 48 | 139 |
| 6 | 326 | | 53 | 307 |
| 7 | 260 | | 54 | 186 |
| 8 | 412 | | 56 | 119 |
| 9 | 406 | | 57 | 221 |
| 10 | 409 | | 58 | 107 |
| 11 | 361 | | 59 | 108 |
| 12 | 398 | | 60 | 113 |
| 13 | 365 | | 61 | 132 |
| 14 | 440 | | 62 | 168 |
| 15 | 409 | | 63 | 98 |
| 16 | 416 | | 64 | 113 |
| 17 | 517 | | 65 | 461 |
| 18 | 374 | | 66 | 151 |
| 19 | 284 | | 67 | 119 |
| 20 | 117 | | 68 | 154 |
| 21 | 301 | | 69 | 407 |
| 22 | 383 | | 71 | 150 |
| 23 | 544 | | 72 | 577 |
| 26 | 188 | | 74 | 375 |
| 27 | 281 | | 75 | 282 |
| 28 | 216 | | 76 | 185 |
| 29 | 201 | | 77 | 120 |
| 30 | 136 | | 78 | 210 |
| 31 | 156 | | 79 | 186 |
| 32 | 145 | | 82 | 325 |
| 33 | 115 | | 83 | 282 |
| 34 | 257 | | 84 | 286 |
| 35 | 287 | | 85 | 189 |
| 36 | 83 | | 86 | 237 |
| 37 | 494 | | 87 | 262 |
| 38 | 190 | | 88 | 112 |
| 39 | 327 | | 89 | 154 |
| 40 | 256 | | 90 | 241 |
| 41 | 529 | | 91 | 130 |
| 42 | 303 | | 92 | 128 |
| 44 | 162 | | 93 | 245 |

**[Table 19]**

| Compound No. | % of control (6 µM) |
|---|---|
| 94 | 199 |
| 95 | 158 |
| 96 | 530 |
| 97 | 553 |
| 98 | 56 |
| 99 | 69 |
| 100 | 105 |
| 101 | 508 |
| 102 | 91 |
| 103 | 485 |
| 104 | 536 |
| 105 | 358 |
| 106 | 227 |
| 107 | 139 |
| 108 | 399 |
| 109 | 229 |
| 110 | 211 |
| 111 | 46 |
| 112 | 139 |
| 113 | 163 |
| 114 | 152 |
| 115 | 163 |
| 119 | 124 |
| 120 | 188 |
| 121 | 149 |
| 122 | 213 |
| 124 | 119 |
| 125 | 118 |

### <Results>

When the test compound was added with the final concentration of 6 µM, hemoglobin production was increased as much as 912% (Compound 3) (see, Table 18 and Table 19). Furthermore, Compound 4, 36, 63, 98, 99, 102 and 111 also showed increased hemoglobin production of 162%, 143%, 260%, 142%, 306%, 115% and 171%, respectively, when they were added with the chemical concentration of 20 µM. Thus, it was found that these compounds can accelerate maturation of proerythroblast cells into red blood cells, and therefore have an activity of potentiating hemoglobin expression.

Based on the results above, it was demonstrated that the 1-acyl-4-(phenoxy, benzyloxy, or phenylamino)-1,2,3,4-tetrahydroquinoline derivatives, more specifically the tetrahydroquinoline compound represented by the Formula (1), salts of the tetrahydroquinoline derivatives, or solvates of the tetrahydroquinoline derivatives and the salts have an activity of accelerating EPO production and an activity of potentiating hemoglobin expression, and therefore are useful as an agent for treating anemia.

### Industrial Applicability

According to the present invention, it was first found that the 1-acyl-4-(phenoxy, benzyloxy, or phenylamino)-1,2,3,4-tetrahydroquinoline derivatives, more specifically the tetrahydroquinoline compound represented by the Formula (1), salts of the tetrahydroquinoline derivatives, or solvates of the tetrahydroquinoline derivatives and the salts have an excellent activity of accelerating EPO production and/or an excellent activity of potentiating hemoglobin expression. Thus, a low molecular weight agent for the prophylaxis and/or treatment of anemia, which has both an excellent activity of accelerating EPO production and/or and can be orally administered, is provided. The present invention has an industrial applicability in that it can provide a novel low molecular weight agent for the prophylaxis and/or treatment of anemia and it is useful for a pharmaceutical industry.

## Claims

1. An EPO production accelerating agent comprising as an effective component 1-acyl-4-(phenoxy, benzyloxy, or phenylamino)-1,2,3,4-tetrahydroquinoline derivatives, salts of the tetrahydroquinoline derivatives or solvates of the tetrahydroquinoline derivatives and the salts.

2. A hemoglobin expression potentiating agent comprising as an effective component 1-acyl-4-(phenoxy, benzyloxy, or phenylamino)-1,2,3,4-tetrahydroquinoline derivatives, salts of the tetrahydroquinoline derivatives or solvates of the tetrahydroquinoline derivatives and the salts.

3. An agent for the prophylaxis and/or treatment of anemia comprising as an effective component 1-acyl-4-(phenoxy, benzyloxy, or phenylamino)-1,2,3,4-tetrahydroquinoline derivatives, salts of the tetrahydroquinoline derivatives or solvates of the tetrahydroquinoline derivatives and the salts.

4. The agent according to any one of Claims 1 to 3, wherein the acyl group at 1-position of the 1-acyl-4-(phenoxy, benzyloxy, or phenylamino)-1,2,3,4-tetrahydroquinoline derivatives consists of alkylcarbonyl, cycloalkylcarbonyl or aminocarbonyl.

5. The agent according to any one of Claims 1 to 4, wherein the 1-acyl-4-(phenoxy, benzyloxy, or phenylamino)-1,2,3,4-tetrahydroquinoline derivatives are tetrahydroquinoline compounds represented by the following Formula (1); 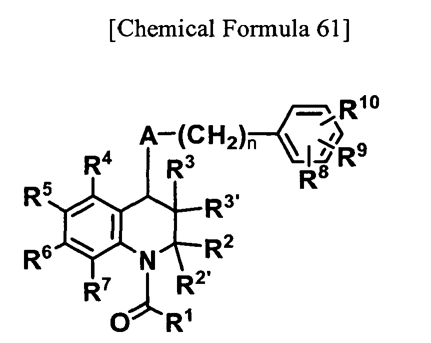 [wherein, R¹, R², R^{2'}, R³ and R^{3'} independently represent a hydrogen atom, a C₁₋₆ alkyl group, a C₂₋₆ alkenyl group, a C₂₋₆ alkynyl group, a C₁₋₆ alkylamino group, a di C₁₋₆ alkylamino group, or a C₃₋₆ cycloalkyl group,
R⁴, R⁵, R⁶, R⁷, R⁸, R⁹ and R¹⁰ independently represent a hydrogen atom, a halogen atom, a C₁₋₆ alkyl group which may be substituted, a C₂₋₆ alkenyl group which may be substituted, a C₂₋₆ alkynyl group which may be substituted, a C₃₋₆ cycloalkyl group which may be substituted, a C₆₋₁₄ aryl group which may be substituted, a 5- to 10-membered heterocyclic group which may be substituted, a C₁₋₆ alkoxy group which may be substituted, a C₆₋₁₄ aryloxy group which may be substituted, an amino group, a C₁₋₆ alkylcarbonylamino group, a C₁₋₆ alkoxycarbonylamino group, a C₁₋₆ alkylamino group which may be substituted, a di C₁₋₆ alkylamino group which may be substituted, a C₆₋₁₄ arylamino group which may be substituted, a C₁₋₆ alkylthio group which may be substituted, a C₁₋₆ alkylsulfonyl group which may be substituted, a C₁₋₆ alkylsulfonamide group which may be substituted, a C₁₋₆ alkylsulfinyl group which may be substituted, a C₁₋₆ alkoxycarbonyl group which may be substituted, a C₁₋₆ alkanoyl group which may be substituted, a 5- to 7-membered saturated heterocyclic carbonyl group which may be substituted, a hydroxy group, a nitro group, a carboxyl group, a carbamoyl group which may be substituted or a cyano group, wherein R⁹ and R¹⁰ together may form a carbon ring or a heterocycle,
A represents N-R¹¹ or an oxygen atom, wherein R¹¹ represents a hydrogen atom or a C₁₋₆ alkyl group which may be substituted, and
n represents an integer of 0 or 1.].

6. Use of the 1-acyl-4-(phenoxy, benzyloxy, or phenylamino)-1,2,3,4-tetrahydroquinoline derivatives, salts of the tetrahydroquinoline derivatives or solvates of the tetrahydroquinoline derivatives and the salts for producing an EPO production accelerating agent.

7. Use of the 1-acyl-4-(phenoxy, benzyloxy, or phenylamino)-1,2,3,4-tetrahydroquinoline derivatives, salts of the tetrahydroquinoline derivatives or solvates of the tetrahydroquinoline derivatives and the salts for producing a hemoglobin expression potentiating agent.

8. Use of the 1-acyl-4-(phenoxy, benzyloxy, or phenylamino)-1,2,3,4-tetrahydroquinoline derivatives, salts of the tetrahydroquinoline derivatives or solvates of the tetrahydroquinoline derivatives and the salts for producing an agent for the prophylaxis and/or treatment of anemia.

9. The use according to any one of Claims 6 to 8, wherein the acyl group at 1-position of the 1-acyl-4-(phenoxy, benzyloxy, or phenylamino)-1,2,3,4-tetrahydroquinoline derivatives consists of alkylcarbonyl, cycloalkylcarbonyl or aminocarbonyl.

10. The use according to any one of Claims 6 to 9, wherein the 1-acyl-4-(phenoxy, benzyloxy, or phenylamino)-1,2,3,4-tetrahydroquinoline derivatives are represented by the Formula (1) described above.

11. A method of accelerating EPO production, **characterized in that** an effective amount of the 1-acyl-4-(phenoxy, benzyloxy, or phenylamino)-1,2,3,4-tetrahydroquinoline derivatives, salts of the tetrahydroquinoline derivatives or solvates of the tetrahydroquinoline derivatives and the salts is administered to a patient who is in need of potentiated EPO production.

12. A method of potentiating hemoglobin expression, **characterized in that** an effective amount of the 1-acyl-4-(phenoxy, benzyloxy, or phenylamino)-1,2,3,4-tetrahydroquinoline derivatives, salts of the tetrahydroquinoline derivatives or solvates of the tetrahydroquinoline derivatives and the salts is administered to a patient who is in need of potentiated hemoglobin expression.

13. A method of preventing and/or treating anemia, **characterized in that** an effective amount of the 1-acyl-4-(phenoxy, benzyloxy, or phenylamino)-1,2,3,4-tetrahydroquinoline derivatives, salts of the tetrahydroquinoline derivatives or solvates of the tetrahydroquinoline derivatives and the salts is administered to a patient who is suffering from anemia.

14. The method according to any one of Claims 11 to 13, wherein the acyl group at 1-position of the 1-acyl-4-(phenoxy, benzyloxy, or phenylamino)-1,2,3,4-tetrahydroquinoline derivatives consists of alkylcarbonyl, cycloalkylcarbonyl or aminocarbonyl.

15. The method according to any one of Claims 11 to 14, wherein the 1-acyl-4-(phenoxy, benzyloxy, or phenylamino)-1,2,3,4-tetrahydroquinoline derivatives are represented by the Formula (1) described above.

16. A tetrahydroquinoline compound selected from the group of compounds consisting of the following compounds, salts of the tetrahydroquinoline compound, or solvates of the tetrahydroquinoline compound and the salts:
1-acetyl-8-fluoro-4-[(2-fluorophenyl)amino]-2-methyl-1,2,3,4-tetrahydroquinoline [Compound (4)];
1-cyclohexanecarbonyl-6-fluoro-4-[(4-fluorophenyl)amino]-2-methyl-1,2,3,4-tetrahydroquinoline [Compound (6)];
1-acetyl-7-cyano-2-methyl-4-phenylamino-1,2,3,4-tetrahydroquinoline [Compound (7)];
1-acetyl-6-cyano-2-methyl-4-phenylamino-1,2,3,4-tetrahydroquinoline [Compound (14)];
1-acetyl-4-[(4-isopropoxyphenyl)amino]-2-methyl-1,2,3,4-tetrahydroquinoline [Compound (18)];
1-acetyl-2-methyl-4-[(4-morpholinophenyl)amino]-1,2,3,4-tetrahydroquinoline [Compound (19)];
1-acetyl-4-[(4-N,N-dimethylaminophenyl)amino]-2-methyl-1,2,3,4-tetrahydroquinoline [Compound (20)];
1-acetyl-7-bromo-2-methyl-4-phenylamino-1,2,3,4-tetrahydroquinoline [Compound (23)];
1-acetyl-4-[(4-hydroxyphenyl)amino]-2-methyl-1,2,3,4-tetrahydroquinoline [Compound (24)];
1-acetyl-2-methyl-4-[(1,1'-biphenyl-4-yl)amino]-1,2,3,4-tetrahydroquinoline [Compound (25)];
1-acetyl-2-methyl-4-phenoxy-1,2,3,4-tetrahydroquinoline [Compound (26)];
1-acetyl-4-(4-fluorophenoxy)-2-methyl-1,2,3,4-tetrahydroquinoline [Compound (27)];
1-acetyl-4-(3-fluorophenoxy)-2-methyl-1,2,3,4-tetrahydroquinoline [Compound (28)];
1-acetyl-4-(2-fluorophenoxy)-2-methyl-1,2,3,4-tetrahydroquinoline [Compound (29)];
1-acetyl-4-(2,4-difluorophenoxy)-2-methyl-1,2,3,4-tetrahydroquinoline [Compound (30)];
1-acetyl-4-(3,4-difluorophenoxy)-2-methyl-1,2,3,4-tetrahydroquinoline [Compound (31)];
1-acetyl-7-fluoro-2-methyl-4-phenoxy-1,2,3,4-tetrahydroquinoline [Compound (32)];
1-acetyl-8-fluoro-2-methyl-4-phenoxy-1,2,3,4-tetrahydroquinoline [Compound (33)];
1-acetyl-4-(4-fluorophenoxy)-6-fluoro-2-methyl-1,2,3,4-tetrahydroquinoline [Compound (34)];
1-acetyl-6-fluoro-2-methyl-4-phenoxy-1,2,3,4-tetrahydroquinoline [Compound (35)];
1-acetyl-2-methyl-4-benzyloxy-1,2,3,4-tetrahydroquinoline [Compound (36)];
1-acetyl-4-[(4-fluorophenyl)amino]-2-methyl-6-methoxy-1,2,3,4-tetrahydroquinoline [Compound (37)];
1-acetyl-4-[(4-hydroxymethylphenyl)amino]-2-methyl-1,2,3,4-tetrahydroquinoline [Compound (38)];
1-acetyl-4-[(4-methanesulfonylamidephenyl)amino]-2-methyl-1,2,3,4-tetrahydroquinoline [Compound (39)];
1-acetyl-4-[(4-chlorophenyl)amino]-2-methyl-6-morpholino-1,2,3,4-tetrahydroquinoline [Compound (40)];
ethyl 1-acetyl-4-[(4-chlorophenyl)amino]-2-methyl-1,2,3,4-tetrahydroquinoline-6-carboxylate [Compound (41)];
1-acetyl-4-[(4-chlorophenyl)amino]-2-methyl-1,2,3,4-tetrahydroquinoline-6-carboxylic acid [Compound (42)];
1-acetyl-4-[(4-chlorophenyl)amino]-2-methyl-1,2,3,4-tetrahydroquinoline-6-carboxamide [Compound (43)];
1-acetyl-4-[(4-chlorophenyl)amino]-2-methyl-7-morpholino-1,2,3,4-tetrahydroquinoline [Compound (44)];
1-acetyl-4-[(4-chlorophenyl)amino]-2-methyl-6-methanesulfonylamino-1,2,3,4-tetrahydroquinoline [Compound (45)];
ethyl 1-acetyl-4-(4-fluorophenoxy)-2-methyl-1,2,3,4-tetrahydroquinoline-6-carboxylate [Compound (53)];
1-acetyl-4-(4-fluorophenoxy)-2-methyl-1,2,3,4-tetrahydroquinoline-6-carboxamide [Compound (54)];
1-acetyl-4-(4-morpholinophenoxy)-2-methyl-1,2,3,4-tetrahydroquinoline [Compound (55)];
1-acetyl-7-fluoro-4-(4-fluorophenoxy)-2-methyl-1,2,3,4-tetrahydroquinoline [Compound (56)];
1-acetyl-4-(4-hydroxyphenoxy)-2-methyl-1,2,3,4-tetrahydroquinoline [Compound (57)];
1-acetyl-7-fluoro-4-[(3-fluorophenyl)amino]-2-methyl-1,2,3,4-tetrahydroquinoline [Compound (58)];
1-acetyl-2-ethyl-4-phenylamino-1,2,3,4-tetrahydroquinoline [Compound (59)];
1-acetyl-3,3-dimethyl-4-phenylamino-1,2,3,4-tetrahydroquinoline [Compound (60)];
1-acetyl-3,3-dimethyl-4-phenylamino-1,2,3,4-tetrahydroquinoline [Compound (61)];
1-acetyl-4-(3,5-difluorophenoxy)-2-methyl-1,2,3,4-tetrahydroquinoline [Compound (62)];
1-acetyl-8-bromo-4-phenylamino-2-methyl-1,2,3,4-tetrahydroquinoline [Compound (63)];
1-acetyl-4-(4-benzyloxyphenoxy)-2-methyl-1,2,3,4-tetrahydroquinoline [Compound (64)];
6-fluoro-4-[(4-fluorophenyl)amino]-2-methyl-1-N-methylcarbamoyl-1,2,3,4-tetrahydroquinoline [Compound (65)];
1-cyclopentanecarbonyl-6-fluoro-2-methyl-4-[(4-fluorophenyl)amino]-1,2,3,4-tetrahydroquinoline [Compound (66)];
1-acetyl-2-methyl-4-(4-nitrophenoxy)-1,2,3,4-tetrahydroquinoline [Compound (67)];
1-acetyl-4-(4-aminophenoxy)-2-methyl-1,2,3,4-tetrahydroquinoline [Compound (68)];
1-acetyl-4-[(4-methoxymethylphenyl)amino]-2-methyl-1,2,3,4-tetrahydroquinoline [Compound (69)];
1-acetyl-4-[(4-ethoxycarbonylmethylphenyl)amino]-2-methyl-1,2,3,4-tetrahydroquinoline [Compound (70)];
1-acetyl-4-[(4-carboxymethylphenyl)amino]-2-methyl-1,2,3,4-tetrahydroquinoline [Compound (71)];
1-acetyl-2-methyl-4-[(2-morpholinophenyl)amino]-1,2,3,4-tetrahydroquinoline [Compound (72)];
1-acetyl-4-[(4-fluoro-3-morpholinophenyl)amino]-2-methyl-1,2,3,4-tetrahydroquinoline [Compound (73)];
1-acetyl-6-bromo-4-[(4-chlorophenyl)amino]-2-methyl-1,2,3,4-tetrahydroquinoline [Compound (74)];
1-acetyl-2-methyl-4-[(4-piperazinylphenyl)amino]-1,2,3,4-tetrahydroquinoline [Compound (76)];
cis-1-acetyl-4-{[4-(4-acetylpiperazinyl)phenyl]amino}-2-methyl-1,2,3,4-tetrahydroquinoline [Compound (77)];
cis-1-acetyl-4-{[4-(4-methanesulfonylpiperazinyl)phenyl]amino}-2-methyl-1,2,3,4-tetrahydroquinoline [Compound (78)];
cis-1-acetyl-6-[(4-acetyl)piperazino]-4-[(4-chlorophenyl)amino]-2-methyl-1,2,3,4-tetrahydroquinoline [Compound (79)];
1-acetyl-6-[(4-methanesulfonyl)piperazino]-4-[(4-chlorophenyl)amino]-2-methyl-1,2,3,4-tetrahydroquinoline [Compound (80)];
1-acetyl-4-[(4-chlorophenyl)amino]-6-[(4-isopropyl)piperazino]-2-methyl-1,2,3,4-tetrahydroquinoline [Compound (81)];
1-acetyl-4-[(4-chlorophenyl)amino]-6-[(2-hydroxy)ethylamino]-2-methyl-1,2,3,4-tetrahydroquinoline [Compound (82)];
1-acetyl-4-[(4-chlorophenyl)amino]-6-[(cis-3,5-dimethyl)morpholino]-2-methyl-1,2,3,4-tetrahydroquinoline [Compound (83)];
1-acetyl-4-[(4-chlorophenyl)amino]-6-[(4-isopropylcarbonyl)piperazino]-2-methyl-1,2,3,4-tetrahydroquinoline [Compound (84)];
1-acetyl-4-[(4-chlorophenyl)amino]-6-[(4-cyclohexylcarbonyl)piperazino]-2-methyl-1,2,3,4-tetrahydroquinoline [Compound (85)];
1-acetyl-6-[(4-benzoyl)piperazino]-2-methyl-4-[(4-chlorophenyl)amino]-1,2,3,4-tetrahydroquinoline [Compound (86)];
1-acetyl-4-[(4-chlorophenyl)amino]-6-[4-(N,N-diethylaminocarbonyl)piperazino]-2-methyl-1,2,3,4-tetrahydroquinoline [Compound (87)];
1-acetyl-4-[(4-chlorophenyl)amino]-6-[4-(isopropylaminocarbonyl)piperazino]-2-methyl-1,2,3,4-tetrahydroquinoline [Compound (88)];
1-acetyl-4-[(4-carboxymethylphenyl)amino]-6-morpholino-2-methyl-1,2,3,4-tetrahydroquinoline [Compound (89)];
1-acetyl-4-[(4-carbamoylmethylphenyl)amino]-2-methyl-6-morpholino-1,2, 3,4-tetrahydroquinoline [Compound (90)];
1-acetyl-6-(4-acetylpiperazinyl)-4-[(4-carboxymethylphenyl)amino]-2-methyl-1,2,3,4-tetrahydroquinoline [Compound (91)];
1-acetyl-2-methyl-4-[(4-morpholinophenyl)amino]-1,2,3,4-tetrahydroquinoline-6-carboxamide [Compound (92)];
1-acetyl-4-[(4-chlorophenyl)amino]-6-[(1-morpholino)carbonyl]-2-methyl-1,2,3,4-tetrahydroquinoline [Compound (93)];
cis-1-acetyl-6-[(4-acetyl)piperazino]-2-methyl-4-[(4-morpholinophenyl)amino]-1,2,3,4-tetrahydroquinoline [Compound (94)];
1-acetyl-6-amino-4-[(4-chlorophenyl)amino]-2-methyl-1,2,3,4-tetrahydroquinoline [Compound (95)];
1-acetyl-6-acetylamino-4-[(4-chlorophenyl)amino]-2-methyl-1,2,3,4-tetrahydroquinoline [Compound (96)];
1-acetyl-4-[(4-chlorophenyl)amino]-2-methyl-1,2,3,4-tetrahydroquinoline-6-ethylcarbamate [Compound (97)];
1-acetyl-6-methanesulfonylamino-2-methyl-4-[(4-morpholinophenyl)amino]-1,2,3,4-tetrahydroquinoline [Compound (98)];
1-acetyl-6-methanesulfonylamino-2-methyl-4-[(4-ethoxycarbonylmethylphenyl)amino]-1,2,3,4-tetrahydroquinoline [Compound (99)];
1-acetyl-4-[(4-fluorophenyl)amino]-2-methyl-1,2,3,4-tetrahydroquinoline-6-carboxylic acid [Compound (100)];
ethyl 1-acetyl-4-[(4-methanesulfonylaminophenyl)amino]-2-methyl-1,2,3,4-tetrahydroquinoline-6-carboxylate [Compound (101)];
cis-1-acetyl-4-[(4-methanesulfonylaminophenyl)amino]-2-methyl-1,2,3,4-tetrahydroquinoline-6-carboxamide [Compound (102)];
ethyl 1-acetyl-4-[(4-cyanomethylphenyl)amino]-2-methyl-1,2,3,4-tetrahydroquinoline-6-carboxylate [Compound (103)];
1-acetyl-4-[(4-cyanomethylphenyl)amino]-2-methyl-1,2,3,4-tetrahydroquinoline-6-carboxamide [Compound (104)];
cis-1-acetyl-4-[(4-carboxymethylphenyl)amino]-2-methyl-1,2,3,4-tetrahydroquinoline-6-carboxamide [Compound (105)];
cis-1-acetyl-4-[(4-carbamoylmethylphenyl)amino]-2-methyl-1,2,3,4-tetrahydroquinoline-6-carboxamide [Compound (106)];
1-acetyl-4-[(4-chlorophenyl)amino]-7-methanesulfonylamino-2-methyl-1,2,3,4-tetrahydroquinoline [Compound (107)];
1-acetyl-4-[(4-hydroxy-3-methoxycarbonylphenyl)amino]-2-methyl-1,2,3,4-tetrahydroquinoline [Compound (108)];
cis-1-acetyl-4-[(2-carboxyphenyl)amino]-2-methyl-1,2,3,4-tetrahydroquinoline [Compound (109)];
1-acetyl-6-[cis-2,6-dimethylmorpholin-4-yl]-4-[(4-methanesulfonylaminophenyl)amino]-2-methyl-1,2,3,4-tetrahydroquinoline [Compound (110)];
1-acetyl-6-[(4-isopropylcarbonyl)piperazino]-4-[(4-methanesulfonylaminophenyl)amino]-2-methyl-1,2,3,4-tetrahydroquinoline [Compound (111)];
cis-1-acetyl-4-[(4-benzylphenyl)amino]-2-methyl-1,2,3,4-tetrahydroquinoline [Compound (112)];
1-acetyl-4-[(4-chlorophenyl)amino]-N,N,2-trimethyl-1,2,3,4-tetrahydroquinoline-6-carboxamide [Compound (113)];
1-acetyl-4-[(4-chlorophenyl)amino]-N,2-dimethyl-1,2,3,4-tetrahydroquinoline-6-carboxamide [Compound (114)];
1 -acetyl-4-[(4-chlorophenyl)amino]-2-methyl-1,2,3,4-tetrahydroquinoline-6-carbohydrazide [Compound (115)];
1-acetyl-4-[(4-chlorophenyl)amino]-6-cyano-2-methyl-1,2,3,4-tetrahydroquinoline [Compound (117)];
1-acetyl-4-[(4-chlorophenyl)amino]-N-methoxy-2-methyl-1,2,3,4-tetrahydroquinoline-6-carboxamide [Compound (118)];
1-acetyl-4-[(4-chlorophenyl)amino]-2-methyl-6-(1H-tetrazol-5-yl)-1,2,3,4-tetrahydroquinoline [Compound (119)];
1-acetyl-4-[(4-chlorophenyl)amino]-2-methyl-6-[1,2,4-oxadiazol-5(2H)-on-3-yl]-1,2,3,4-tetrahydroquinoline [Compound (120)];
cis-1-acetyl-4-[(4-chlorophenyl)amino]-6-hydroxymethyl-2-methyl-1,2,3,4-tetrahydroquinoline [Compound (121)];
1-acetyl-4-[(4-ethoxycarbonylmethylphenyl)amino]-6-fluoro-2-methyl-1,2,3,4-tetrahydroquinoline [Compound (122)];
1-acetyl-4-[(4-carboxymethylphenyl)amino]-6-fluoro-2-methyl-1,2,3,4-tetrahydroquinoline [Compound (123)];
1-acetyl-4-[(4-carbamoylmethylphenyl)amino]-6-fluoro-2-methyl-1,2,3,4-tetrahydroquinoline [Compound (124)]; and,
1-acetyl-4-[4-(N,N-dimethylaminocarbonylmethyl)phenylamino]-6-fluoro-2-methyl-1,2,3,4-tetrahydroquinoline [Compound (125)].

17. A pharmaceutical composition comprising one, two or more of a tetrahydroquinoline compound selected from the group of compounds consisting of the following compounds, salts of tetrahydroquinoline compound, or solvates of the tetrahydroquinoline compound and the salts, and a pharmaceutically acceptable carrier:
1-acetyl-8-fluoro-4-[(2-fluorophenyl)amino]-2-methyl-1,2,3,4-tetrahydroquinoline [Compound (4)];
1-cyclohexanecarbonyl-6-fluoro-4-[(4-fluorophenyl)amino]-2-methyl-1,2,3,4-tetrahydroquinoline [Compound (6)];
1-acetyl-7-cyano-2-methyl-4-phenylamino-1,2,3,4-tetrahydroquinoline [Compound (7)];
1-acetyl-6-cyano-2-methyl-4-phenylamino-1,2,3,4-tetrahydroquinoline [Compound (14)];
1-acetyl-4-[(4-isopropoxyphenyl)amino]-2-methyl-1,2,3,4-tetrahydroquinoline [Compound (18)];
1-acetyl-2-methyl-4-[(4-morpholinophenyl)amino]-1,2,3,4-tetrahydroquinoline [Compound (19)];
1-acetyl-4-[(4-N,N-dimethylaminophenyl)amino]-2-methyl-1,2,3,4-tetrahydroquinoline [Compound (20)];
1-acetyl-7-bromo-2-methyl-4-phenylamino-1,2,3,4-tetrahydroquinoline [Compound (23)];
1-acetyl-4-[(4-hydroxyphenyl)amino]-2-methyl-1,2,3,4-tetrahydroquinoline [Compound (24)];
1-acetyl-2-methyl-4-[(1,1'-biphenyl-4-yl)amino]-1,2,3,4-tetrahydroquinoline [Compound (25)];
1-acetyl-2-methyl-4-phenoxy-1,2,3,4-tetrahydroquinoline [Compound (26)];
1-acetyl-4-(4-fluorophenoxy)-2-methyl-1,2,3,4-tetrahydroquinoline [Compound (27)];
1-acetyl-4-(3-fluorophenoxy)-2-methyl-1,2,3,4-tetrahydroquinoline [Compound (28)];
1-acetyl-4-(2-fluorophenoxy)-2-methyl-1,2,3,4-tetrahydroquinoline [Compound (29)];
1-acetyl-4-(2,4-difluorophenoxy)-2-methyl-1,2,3,4-tetrahydroquinoline [Compound (30)];
1-acetyl-4-(3,4-difluorophenoxy)-2-methyl-1,2,3,4-tetrahydroquinoline [Compound (31)];
1-acetyl-7-fluoro-2-methyl-4-phenoxy-1,2,3,4-tetrahydroquinoline [Compound (32)];
1-acetyl-8-fluoro-2-methyl-4-phenoxy-1,2,3,4-tetrahydroquinoline [Compound (33)];
1-acetyl-4-(4-fluorophenoxy)-6-fluoro-2-methyl-1,2,3,4-tetrahydroquinoline [Compound (34)];
1-acetyl-6-fluoro-2-methyl-4-phenoxy-1,2,3,4-tetrahydroquinoline [Compound (35)];
1-acetyl-2-methyl-4-benzyloxy-1,2,3,4-tetrahydroquinoline [Compound (36)];
1-acetyl-4-[(4-fluorophenyl)amino]-2-methyl-6-methoxy-1,2,3,4-tetrahydroquinoline [Compound (37)];
1-acetyl-4-[(4-hydroxymethylphenyl)amino]-2-methyl-1,2,3,4-tetrahydroquinoline [Compound (38)];
1-acetyl-4-[(4-methanesulfonylamidephenyl)amino]-2-methyl-1,2,3,4-tetrahydroquinoline [Compound (39)];
1-acetyl-4-[(4-chlorophenyl)amino]-2-methyl-6-morpholino-1,2,3,4-tetrahydroquinoline [Compound (40)];
ethyl 1-acetyl-4-[(4-chlorophenyl)amino]-2-methyl-1,2,3,4-tetrahydroquinoline-6-carboxylate [Compound (41)];
1-acetyl-4-[(4-chlorophenyl)amino]-2-methyl-1,2,3,4-tetrahydroquinoline-6-carboxylic acid [Compound (42)];
1-acetyl-4-[(4-chlorophenyl)amino]-2-methyl-1,2,3,4-tetrahydroquinoline-6-carboxamide [Compound (43)];
1-acetyl-4-[(4-chlorophenyl)amino]-2-methyl-7-morpholino-1,2,3,4-tetrahydroquinoline [Compound (44)];
1-acetyl-4-[(4-chlorophenyl)amino]-2-methyl-6-methanesulfonylamino-1,2,3,4-tetrahydroquinoline [Compound (45)];
ethyl 1-acetyl-4-(4-fluorophenoxy)-2-methyl-1,2,3,4-tetrahydroquinoline-6-carboxylate [Compound (53)];
1-acetyl-4-(4-fluorophenoxy)-2-methyl-1,2,3,4-tetrahydroquinoline-6-carboxamide [Compound (54)];
1-acetyl-4-(4-morpholinophenoxy)-2-methyl-1,2,3,4-tetrahydroquinoline [Compound (55)];
1-acetyl-7-fluoro-4-(4-fluorophenoxy)-2-methyl-1,2,3,4-tetrahydroquinoline [Compound (56)];
1-acetyl-4-(4-hydroxyphenoxy)-2-methyl-1,2,3,4-tetrahydroquinoline [Compound (57)];
1-acetyl-7-fluoro-4-[(3-fluorophenyl)amino]-2-methyl-1,2,3,4-tetrahydroquinoline [Compound (58)];
1-acetyl-2-ethyl-4-phenylamino-1,2,3,4-tetrahydroquinoline [Compound (59)];
1-acetyl-3,3-dimethyl-4-phenylamino-1,2,3,4-tetrahydroquinoline [Compound (60)];
1-acetyl-3,3-dimethyl-4-phenylamino-1,2,3,4-tetrahydroquinoline [Compound (61)];
1-acetyl-4-(3,5-difluorophenoxy)-2-methyl-1,2,3,4-tetrahydroquinoline [Compound (62)];
1-acetyl-8-bromo-4-phenylamino-2-methyl-1,2,3,4-tetrahydroquinoline [Compound (63)];
1-acetyl-4-(4-benzyloxyphenoxy)-2-methyl-1,2,3,4-tetrahydroquinoline [Compound (64)];
6-fluoro-4-[(4-fluorophenyl)amino]-2-methyl-1-N-methylcarbamoyl-1,2,3,4-tetrahydroquinoline [Compound (65)];
1-cyclopentanecarbonyl-6-fluoro-2-methyl-4-[(4-fluorophenyl)amino]-1,2,3,4-tetrahydroquinoline [Compound (66)];
1-acetyl-2-methyl-4-(4-nitrophenoxy)-1,2,3,4-tetrahydroquinoline [Compound (67)];
1-acetyl-4-(4-aminophenoxy)-2-methyl-1,2,3,4-tetrahydroquinoline [Compound (68)];
1-acetyl-4-[(4-methoxymethylphenyl)amino]-2-methyl-1,2,3,4-tetrahydroquinoline [Compound (69)];
1-acetyl-4-[(4-ethoxycarbonylmethylphenyl)amino]-2-methyl-1,2,3,4-tetrahydroquinoline [Compound (70)];
1-acetyl-4-[(4-carboxymethylphenyl)amino]-2-methyl-1,2,3,4-tetrahydroquinoline [Compound (71)];
1-acetyl-2-methyl-4-[(2-morpholinophenyl)amino]-1,2,3,4-tetrahydroquinoline [Compound (72)];
1-acetyl-4-[(4-fluoro-3-morpholinophenyl)amino]-2-methyl-1,2,3,4-tetrahydroquinoline [Compound (73)];
1-acetyl-6-bromo-4-[(4-chlorophenyl)amino]-2-methyl-1,2,3,4-tetrahydroquinoline [Compound (74)];
1-acetyl-2-methyl-4-[(4-piperazinylphenyl)amino]-1,2,3,4-tetrahydroquinoline [Compound (76)];
cis-1-acetyl-4-([4-(4-acetylpiperazinyl)phenyl]amino)}-2-methyl-1,2,3,4-tetrahydroquinoline [Compound (77)];
cis-1-acetyl-4-{[4-(4-methanesulfonylpiperazinyl)phenyl]amino}-2-methyl-1,2,3,4-tetrahydroquinoline [Compound (78)];
cis-1-acetyl-6-[(4-acetyl)piperazino]-4-[(4-chlorophenyl)amino]-2-methyl-1,2,3,4-tetrahydroquinoline [Compound (79)];
1-acetyl-6-[(4-methanesulfonyl)piperazino]-4-[(4-chlorophenyl)amino]-2-methyl-1,2,3,4-tetrahydroquinoline [Compound (80)];
1-acetyl-4-[(4-chlorophenyl)amino]-6-[(4-isopropyl)piperazino]-2-methyl-1,2,3,4-tetrahydroquinoline [Compound (81)];
1-acetyl-4-[(4-chlorophenyl)amino]-6-[(2-hydroxy)ethylamino]-2-methyl-1,2,3,4-tetrahydroquinoline [Compound (82)];
1-acetyl-4-[(4-chlorophenyl)amino]-6-[(cis-3,5-dimethyl)morpholino]-2-methyl-1,2,3,4-tetrahydroquinoline [Compound (83)];
1-acetyl-4-[(4-chlorophenyl)amino]-6-[(4-isopropylcarbonyl)piperazino]-2-methyl-1,2,3,4-tetrahydroquinoline [Compound (84)];
1-acetyl-4-[(4-chlorophenyl)amino]-6-[(4-cyclohexylcarbonyl)piperazino]-2-methyl-1,2,3,4-tetrahydroquinoline [Compound (85)];
1-acetyl-6-[(4-benzoyl)piperazino]-2-methyl-4-[(4-chlorophenyl)amino]-1,2,3,4-tetrahydroquinoline [Compound (86)];
1-acetyl-4-[(4-chlorophenyl)amino]-6-[4-(N,N-diethylaminocarbonyl)piperazino]-2-methyl-1,2,3,4-tetrahydroquinoline [Compound (87)];
1-acetyl-4-[(4-chlorophenyl)amino]-6-[4-(isopropylaminocarbonyl)piperazino]-2-methyl-1,2,3,4-tetrahydroquinoline [Compound (88)];
1-acetyl-4-[(4-carboxymethylphenyl)amino]-6-morpholino-2-methyl-1,2,3,4-tetrahydroquinoline [Compound (89)];
1-acetyl-4-[(4-carbamoylmethylphenyl)amino]-2-methyl-6-morpholino-1,2,3,4-tetrahydroquinoline [Compound (90)];
1-acetyl-6-(4-acetylpiperazinyl)-4-[(4-carboxymethylphenyl)amino]-2-methyl-1,2,3,4-tetrahydroquinoline [Compound (91)];
1-acetyl-2-methyl-4-[(4-morpholinophenyl)amino]-1,2,3,4-tetrahydroquinoline-6-carboxamide [Compound (92)];
1-acetyl-4-[(4-chlorophenyl)amino]-6-[(1-morpholino)carbonyl]-2-methyl-1,2,3,4-tetrahydroquinoline [Compound (93)];
cis-1-acetyl-6-[(4-acetyl)piperazino]-2-methyl-4-[(4-morpholinophenyl)amino]-1,2,3,4-tetrahydroquinoline [Compound (94)];
1-acetyl-6-amino-4-[(4-chlorophenyl)amino]-2-methyl-1,2,3,4-tetrahydroquinoline [Compound (95)];
1-acetyl-6-acetylamino-4-[(4-chlorophenyl)amino]-2-methyl-1,2,3,4-tetrahydroquinoline [Compound (96)];
1-acetyl-4-[(4-chlorophenyl)aminol-2-methyl-1,2,3,4-tetrahydroquinoline-6-ethylcarbamate [Compound (97)];
1-acetyl-6-methanesulfonylamino-2-methyl-4-[(4-morpholinophenyl)amino]-1,2,3,4-tetrahydroquinoline [Compound (98)];
1-acetyl-6-methanesulfonylamino-2-methyl-4-[(4-ethoxycarbonylmethylphenyl)amino]-1,2,3,4-tetrahydroquinoline [Compound (99)];
1-acetyl-4-[(4-fluorophenyl)amino]-2-methyl-1,2,3,4-tetrahydroquinoline-6-carboxylic acid [Compound (100)];
ethyl 1-acetyl-4-[(4-methanesulfonylaminophenyl)amino]-2-methyl-1,2,3,4-tetrahydroquinoline-6-carboxylate [Compound (101)];
cis-1-acetyl-4-[(4-methanesulfonylaminophenyl)amino]-2-methyl-1,2,3,4-tetrahydroquinoline-6-carboxamide [Compound (102)];
ethyl 1-acetyl-4-[(4-cyanomethylphenyl)amino]-2-methyl-1,2,3,4-tetrahydroquinoline-6-carboxylate [Compound (103)];
1-acetyl-4-[(4-cyanomethylphenyl)amino]-2-methyl-1,2,3,4-tetrahydroquinoline-6-carboxamide [Compound (104)];
cis-1-acetyl-4-[(4-carboxymethylphenyl)amino]-2-methyl-1,2,3,4-tetrahydroquinoline-6-carboxamide [Compound (105)];
cis-1-acetyl-4-[(4-carbamoylmethylphenyl)amino]-2-methyl-1,2,3,4-tetrahydroquinoline-6-carboxamide [Compound (106)];
1-acetyl-4-[(4-chlorophenyl)amino]-7-methanesulfonylamino-2-methyl-1,2,3,4-tetrahydroquinoline [Compound (107)];
1-acetyl-4-[(4-hydroxy-3-methoxycarbonylphenyl)amino]-2-methyl-1,2,3,4-tetrahydroquinoline [Compound (108)];
cis-1-acetyl-4-[(2-carboxyphenyl)amino]-2-methyl-1,2,3,4-tetrahydroquinoline [Compound (109)];
1-acetyl-6-[cis-2,6-dimethylmorpholin-4-yl]-4-[(4-methanesulfonylaminophenyl)amino]-2-methyl-1,2,3,4-tetrahydroquinoline [Compound (110)];
1-acetyl-6-[(4-isopropylcarbonyl)piperazino]-4-[(4-methanesulfonylaminophenyl)amino]-2-methyl-1,2,3,4-tetrahydroquinoline [Compound (111)];
cis-1-acetyl-4-[(4-benzylphenyl)amino]-2-methyl-1,2,3,4-tetrahydroquinoline [Compound (112)];
1-acetyl-4-[(4-chlorophenyl)amino]-N,N,2-trimethyl-1,2,3,4-tetrahydroquinoline-6-carboxamide [Compound (113)];
1-acetyl-4-[(4-chlorophenyl)amino]-N,2-dimethyl-1,2,3,4-tetrahydroquinoline-6-carboxamide [Compound (114)];
1-acetyl-4-[(4-chlorophenyl)amino]-2-methyl-1,2,3,4-tetrahydroquinoline-6-carbohydrazide [Compound (115)];
1-acetyl-4-[(4-chlorophenyl)amino]-6-cyano-2-methyl-1,2,3,4-tetrahydroquinoline [Compound (117)];
1-acetyl-4-[(4-chlorophenyl)amino]-N-methoxy-2-methyl-1,2,3,4-tetrahydroquinoline-6-carboxamide [Compound (118)];
1-acetyl-4-[(4-chlorophenyl)amino]-2-methyl-6-(1H-tetrazol-5-yl)-1,2,3,4-tetrahydroquinoline [Compound (119)];
1-acetyl-4-[(4-chlorophenyl)amino]-2-methyl-6-[1,2,4-oxadiazol-5(2H)-on-3-yl]-1,2,3,4-tetrahydroquinoline [Compound (120)];
cis-1-acetyl-4-[(4-chlorophenyl)amino]-6-hydroxymethyl-2-methyl-1,2,3,4-tetrahydroquinoline [Compound (121)];
1-acetyl-4-[(4-ethoxycarbonylmethylphenyl)amino]-6-fluoro-2-methyl-1,2,3,4-tetrahydroquinoline [Compound (122)];
1-acetyl-4-[(4-carboxymethylphenyl)amino]-6-fluoro-2-methyl-1,2,3,4-tetrahydroquinoline [Compound (123)];
1-acetyl-4-[(4-carbamoylmethylphenyl)amino]-6-fluoro-2-methyl-1,2,3,4-tetrahydroquinoline [Compound (124)]; and
1-acetyl-4-[4-(N,N-dimethylaminocarbonylmethyl)phenylamino]-6-fluoro-2-methyl-1,2,3,4-tetrahydroquinoline [Compound (125)].

18. The pharmaceutical composition according to Claim 17, wherein the pharmaceutical composition is used for accelerating EPO production.

19. The pharmaceutical composition according to Claim 17, wherein the pharmaceutical composition is used for potentiating hemoglobin expression.

20. The pharmaceutical composition according to any one of Claims 17 to 19, wherein the pharmaceutical composition is used for the prophylaxis and/or treatment of anemia.
